(19) Europäisches Patentamt / European Patent Office / Office européen des brevets

(11) **EP 4 613 752 A1**

(12) **EUROPEAN PATENT APPLICATION**
published in accordance with Art. 153(4) EPC


(43) Date of publication:
**10.09.2025 Bulletin 2025/37**

(21) Application number: **23884886.5**

(22) Date of filing: **31.10.2023**

(51) International Patent Classification (IPC):
***C07D 519/00*** *(2006.01)* ***C07D 471/04*** *(2006.01)*
***C07D 401/14*** *(2006.01)* ***A61K 31/444*** *(2006.01)*
***A61K 31/4545*** *(2006.01)* ***A61P 35/00*** *(2006.01)*

(52) Cooperative Patent Classification (CPC):
**A61K 31/444; A61K 31/4545; A61P 35/00; C07D 401/14; C07D 471/04; C07D 519/00**

(86) International application number:
**PCT/CN2023/128071**

(87) International publication number:
**WO 2024/093956 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.11.2022 CN 202211363520**
**25.02.2023 CN 202310164531**
**22.03.2023 CN 202310283136**
**27.06.2023 CN 202310763102**



(71) Applicant: **Xuanzhu Biopharmaceutical Co., Ltd.**
**Shijiazhuang, Hebei 050035 (CN)**

(72) Inventors:
- **LIU, Bin**
**Jinan, Shandong 250101 (CN)**
- **CHEN, Bo**
**Jinan, Shandong 250101 (CN)**

(74) Representative: **Murgitroyd & Company**
**165-169 Scotland Street**
**Glasgow G5 8PL (GB)**


(54) **POLYCYCLIC POLY(ADP-RIBOSE) POLYMERASE SELECTIVE INHIBITOR**


(57) The present application relates to the technical field of medicines, in particular to a polycyclic poly(ADP-ribose) polymerase (PARP) selective inhibitor compound as shown in general formula (I), a pharmaceutically acceptable salt thereof or a stereoisomer thereof, a pharmaceutical composition including the compound, the pharmaceutically acceptable salt thereof or the stereoisomer thereof, a method for preparing the compound, the pharmaceutically acceptable salt thereof or the stereoisomer thereof, and a use of the compound, the pharmaceutically acceptable salt thereof or the stereoisomer thereof.

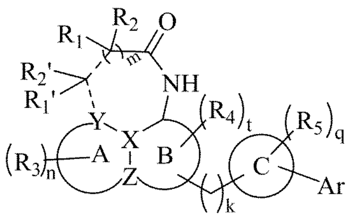

(I)


EP 4 613 752 A1

## Description

### TECHNICAL FIELD

The present application belongs to the technical field of medicines, in particular to a polycyclic poly(ADP-ribose) polymerase selective inhibitor compound, a pharmaceutically acceptable salt thereof or a stereoisomer thereof, a pharmaceutical composition and formulation including the compound, the pharmaceutically acceptable salt thereof or the stereoisomer thereof, a method for preparing the compound, the pharmaceutically acceptable salt thereof or the stereoisomer thereof, and a use of the compound, the pharmaceutically acceptable salt thereof or the stereoisomer thereof.

### BACKGROUND

Targeted therapy to inhibit PARP1 (poly(ADP-ribose) polymerase 1) is one of the research hotspots at home and abroad at the present stage. The PARP1 is the most typical member of PARP family, which plays more than 90% functions in the PARP family. The PARP1 is a ribozyme, which regulates a variety of cellular processes through PARylation (that is, Poly(ADP-ribosyl)ation), including DNA damage signal, chromatin remodeling, transcription, stabilization of replication fork, sensing of unligated Okazaki fragment during replication, inflammation and metabolism, etc. The PARP1 consists of 1014 amino acid residues, including 3 structural domains, namely, an N-terminal DNA binding domain (DBD), an intermediate auto-regulatory domain (AD) and a C-terminal catalytic domain (CAT). The N-terminal DNA binding domain includes 3 zinc finger motifs (Zn I, Zn II and Zn III) and a DNA strand break-sensing element (Nuclear Localization Signal, NLS). Zn I and Zn II recognize damaged DNA, and Zn III participates in the connection between domains and activates proteins. The intermediate auto-regulatory domain includes a carboxyl terminal (DNA repair and cell signal transduction) of one BRCA1 (Breast Cancer 1 gene) and has a Capase-3 digestion function. The C-terminal catalytic domain includes a tryptophan-glycine-arginine-rich domain (WGR), an $\alpha$-helix domain (HD) and an ADP ribosyltransferase domain (ART). The PARP1 is very important for timely and accurate repair of DNA single-strand damage, and the PARP1 is quickly recruited to single-strand breaks (SSBs) when DNA is damaged, and polymerizes itself with other proteins by combining with single-strand DNA (ssDNA), so as to complete the recruitment of downstream DNA repair factor.

Homologous recombination repair (HRR) of DNA is one of the core repair methods of DNA double-strand damage. After the BRCA1 and BRCA2 (Breast Cancer 2 gene) are recruited, the homologous recombination repair is regulated, the BRCA1 initiates homologous recombination (HR) by promoting the terminal excision of double-strand breaks (DSBs), and then acts together with the BRCA2 and PALB2 (Partner and Localizer of BRCA2) downstream to stimulate RAD51 to gather at excised single-strand DNA, and then a sister chromatid is used as a template to accurately repair the DNA damage. In addition to the actions in HR, the BRCA1 and the BRCA2 are also important in an S phase, which can protect stopped replication fork from being degraded by nuclease. In view of the above functions of the BRCA1 and the BRCA2, their mutations will increase the occurrence of breast cancer, ovarian cancer, prostate cancer and pancreatic cancer because of the loss of remaining wild alleles and the high-level genome instability caused by HR deficiency. BRCA1/2 mutant tumors of HR deficiency rely on compensatory DNA repair pathways, drug inhibition of key components (such as PARP1) of these pathways may cause DNA damage, leading to key genome instability, mitosis disaster and cell death in the absence of BRCA1/2, and finally, the BRCA1/2 and the PARP synergistically cause death.

A molecular action mechanism of a PARP inhibitor includes two aspects. On one hand, the PARP1 inhibitor competitively binds to the CAT (C-terminal catalytic domain) of the PARP-1 to inhibit its catalytic activity, which prevents the timely repair of SSB and leads to the formation of DSB. On the other hand, the PARP-1 inhibitor inhibits the PARylation of the PARP1 and binds to the CAT, leading to conformational changes of the PARP1. This enhances the binding affinity between the PARP1 and the damaged DNA, resulting in the PARP1 being "captured" on the damaged DNA. The captured PARP1 prevents other PARP1 in the nucleus from binding to the damaged DNA, thereby blocking possible repair pathways of DSB and promoting cell apoptosis.

Since Olaparib has been approved for BRCA mutant ovarian cancer in 2014, many PARP inhibitors have been developed and marketed, and achieved widespread success. However, the adverse reactions of the drugs limit their ability to be combined with chemotherapy drugs. Most of the first-generation PARP inhibitors were developed and optimized before the concept of PARP1-DNA capture was discovered, which is the mechanism by which the PARP inhibitors exert a synthetic lethal effect on BRCAm cells. In addition, the first-generation PARP inhibitors are not selectively optimized in the PARP family, which may lead to adverse side effects, including intestinal toxicity caused by tankyrase inhibition or blood toxicity caused by PARP2 inhibition. Therefore, the development of inhibitors with capture ability and high selectivity for PARP1 to reduce the toxicity of existing PARP inhibitors while ensuring the drug efficacy has become a new research direction of PARP inhibitors.

## SUMMARY

A technical problem to be solved by the present application is to provide a polycyclic compound with a novel structure and a good selective inhibition effect on PARP1. Further, this compound can be used for preventing and/or treating a disease related to PARP.

Technical solutions of the present application are as follows:

In one aspect, the present application provides a compound as shown in formula (I), a pharmaceutically acceptable salt thereof or a stereoisomer thereof,

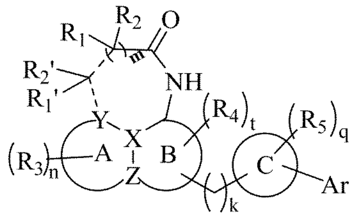

(I)

where,

X, Y and Z are each independently selected from N, C and CH;

ring A and ring B are each independently selected from 5-7 membered cycloalkyl, 5-7 membered heterocyclyl, phenyl and 5-7 membered heteroaryl;

ring C is selected from 3-11 membered cycloalkyl, 3-11 membered heterocyclyl, 6-11 membered aryl and 5-11 membered heteroaryl;

Ar is selected from 3-11 membered cycloalkyl, 3-11 membered heterocyclyl, 6-11 membered aryl and 5-11 membered heteroaryl, each of which is optionally substituted by 1-3 Q substituents; each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p$-3-10 membered cycloalkyl, $-(CH_2)_p$-3-10 membered heterocycloalkyl, $-(CH_2)_p-N(R^a)(R^b)$, $-(CH_2)_p-O-R^a$, $-(CHz)_p-P(O)(R^a)(R^b)$, $-(CH_2)_p-S(O)(R^a)$, $-(CH_2)_p-S(O)_2(R^a)$, $-(CH_2)_p-C(O)(R^a)$, $-(CH_2)_p-C(O)O(R^a)$, $-(CH_2)_p-O-C(O)(R^a)$, $-(CH_2)_p-C(O)N(R^a)(R^b)$ and $-(CH_2)_p-N(R^b)-C(O)(R^a)$;

each $R_1$ and each $R_2$ are independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl; or $R_1$, $R_2$ and a carbon atom linked thereto form 3-7 membered cycloalkyl or 3-7 membered heterocyclyl;

$R_1'$ and $R_2'$ are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

each $R_3$, each $R_4$ and each $R_5$ are independently selected from H, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy-$C_{1-6}$ alkyl, 3-10 membered cycloalkyl and 3-10 membered heterocyclyl;

m is 0, 1 or 2, and when m is 2, two adjacent ring carbon atoms are linked by a single bond or a double bond;

n and t are each independently selected from 0, 1, 2 and 3;

p and k are each independently selected from 0, 1 and 2;

q is 0, 1, 2, 3 or 4; and

- - - represents a single bond or a double bond.

In some embodiments, X, Y and Z are each independently selected from N and C.

In some embodiments, X, Y and Z are each independently selected from N and C; and at most one of X, Y and Z is N.

In some embodiments, X is N, and Y and Z are C.

In some embodiments, Y is N, and X and Z are C.

In some embodiments, Z is N, and X and Y are C.

In some embodiments, X, Y and Z are all C.

In some embodiments, ring A and ring B are each independently selected from 5-6 membered cycloalkyl, 5-6 membered heterocyclyl, phenyl and 5-6 membered heteroaryl.

In some embodiments, ring A and ring B are each independently selected from 5-6 membered cycloalkyl, 5-6 membered heterocyclyl including 1-2 heteroatoms, phenyl and 5-6 membered heteroaryl including 1-2 heteroatoms; and the heteroatom is a nitrogen atom, an oxygen atom or a sulfur atom.

In some embodiments, ring A is selected from 5-6 membered cycloalkyl, 5-6 membered heterocyclyl, phenyl and 5-6 membered heteroaryl; and ring B is phenyl or 5-6 membered heteroaryl.

In some embodiments, ring A is selected from 5-6 membered cycloalkyl, 5-6 membered heterocyclyl including 1-2 heteroatoms, phenyl and 5-6 membered heteroaryl including 1-2 heteroatoms; ring B is phenyl or 5-6 membered heteroaryl including 1-2 heteroatoms; and the heteroatom is selected from a nitrogen atom, an oxygen atom and a sulfur atom.

In some embodiments, ring B is selected from phenyl and 6 membered heteroaryl including 1-2 heteroatoms.

In some embodiments, ring A and ring B are each independently selected from cyclopentyl, cyclohexyl, cyclopentenyl, cyclohexenyl, phenyl, pyrrolyl, pyrazolyl, imidazolyl, pyridyl, pyrimidinyl, pyrazinyl, pyridazinyl, dihydropyrrolyl, pyrrolidinyl, dihydropyrazolyl, pyrazolidinyl, dihydroimidazolyl, imidazolidinyl, dihydropyridyl, tetrahydropyridyl, piperidinyl, dihydropyrimidinyl, tetrahydropyrimidinyl, hexahydropyrimidinyl, dihydropyrazinyl, tetrahydropyrazinyl, piperazinyl, dihydropyridazinyl, tetrahydropyridazinyl, hexahydropyridazinyl, furyl, dihydrofuryl, tetrahydrofuryl, pyranyl, dihydropyranyl, tetrahydropyranyl, thiazolyl, oxazolyl, triazolyl, dihydrothiazolyl, tetrahydrothiazolyl, dihydrooxazolyl and tetrahydrooxazolyl.

In some embodiments, ring A is selected from cyclopentyl, cyclohexyl, cyclopentenyl, cyclohexenyl, phenyl, pyrrolyl, pyrazolyl, imidazolyl, pyridyl, pyrimidinyl, pyrazinyl, pyridazinyl, dihydropyrrolyl, pyrrolidinyl, dihydropyrazolyl, pyrazolidinyl, dihydroimidazolyl, imidazolidinyl, dihydropyridyl, tetrahydropyridyl, piperidinyl, dihydropyrimidinyl, tetrahydropyrimidinyl, hexahydropyrimidinyl, dihydropyrazinyl, tetrahydropyrazinyl, piperazinyl, dihydropyridazinyl, tetrahydropyridazinyl, hexahydropyridazinyl, furyl, dihydrofuryl, tetrahydrofuryl, pyranyl, dihydropyranyl, tetrahydropyranyl, thiazolyl, oxazolyl, triazolyl, dihydrothiazolyl, tetrahydrothiazolyl, dihydrooxazolyl and tetrahydrooxazolyl; and
ring B is selected from phenyl, pyrrolyl, dihydropyrrolyl, pyrazolyl, dihydropyrazolyl, imidazolyl, dihydroimidazolyl, pyridyl, dihydropyridyl, pyrimidinyl, dihydropyrimidinyl, pyrazinyl, dihydropyrazinyl, pyridazinyl, dihydropyridazinyl, piperidinyl, dihydropiperidinyl, piperazinyl, dihydropiperazinyl, furyl, pyranyl, dihydropyranyl, thiazolyl, oxazolyl and triazolyl.

In some embodiments, ring A, ring B, and X, Y and Z together form the following group:

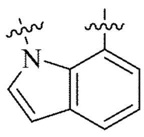, 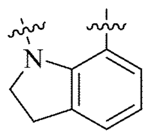, 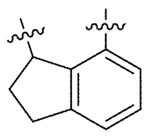, 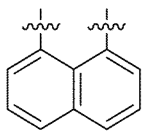, 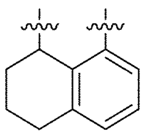, 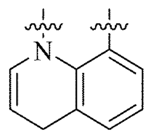, 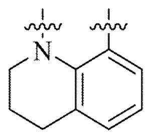,

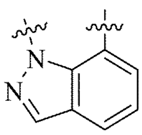, 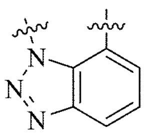, 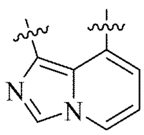, 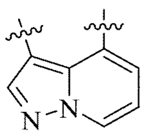, 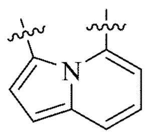, 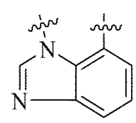 or 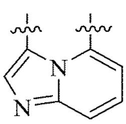.

In some embodiments, ring A, ring B, and X, Y and Z together form the following group:

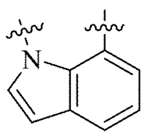, 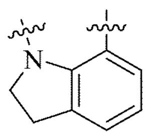, 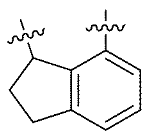, 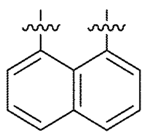, 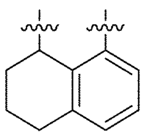, 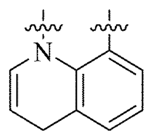, 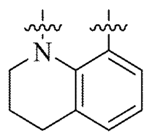,

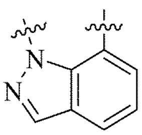, 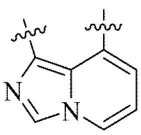, 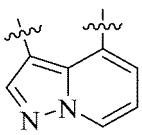, 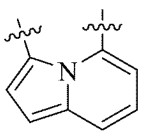, 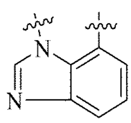 or 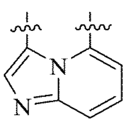.

In some embodiments, ring A, ring B, and X, Y and Z together form the following group:

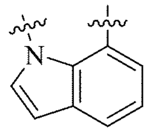, 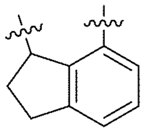, 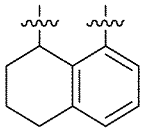, 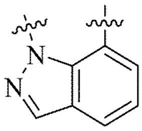, 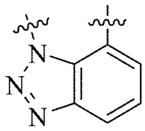, 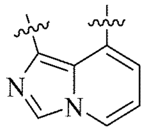, 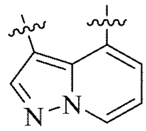 or

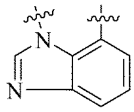.

In some embodiments, ring C is selected from 5-6 membered cycloalkyl, 5-6 membered heterocyclyl, phenyl, 5-6 membered heteroaryl, 8-11 membered fused cycloalkyl, 8-11 membered spiro cycloalkyl, 7-9 membered bridged cycloalkyl, 8-11 membered fused heterocyclyl, 8-11 membered spiro heterocyclyl and 7-9 membered bridged heterocyclyl.

In some embodiments, ring C is selected from 5-6 membered cycloalkyl, 5-6 membered heterocyclyl, phenyl, 5-6 membered heteroaryl, 8-11 membered fused cycloalkyl, 8-11 membered spiro cycloalkyl, 7-9 membered bridged cycloalkyl, 8-11 membered fused heterocyclyl, 8-11 membered spiro heterocyclyl and 7-9 membered bridged heterocyclyl;

In some embodiments, ring C is selected from 5-6 membered cycloalkyl, 5-6 membered heterocyclyl, 7-9 membered bridged cycloalkyl and 7-9 membered bridged heterocyclyl.

In some embodiments, ring C is selected from 5-6 membered cycloalkyl, 5-6 membered heterocyclyl, 7-8 membered bridged cycloalkyl and 7-8 membered bridged heterocyclyl.

In some embodiments, ring C is selected from the following group:

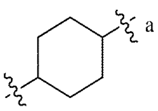, 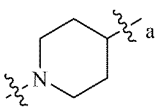, 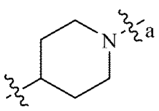, 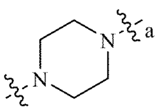, 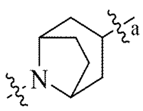, 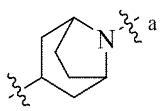, 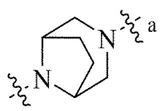,

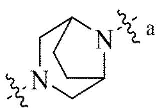, 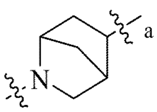, 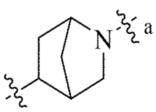, 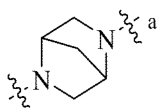 or 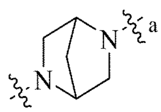,

and a-terminal is linked to Ar.

In some embodiments, ring C is selected from 5-6 membered cycloalkyl and 5-6 membered heterocyclyl.

In some embodiments, ring C is selected from the following group:

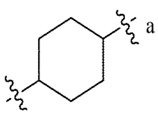, 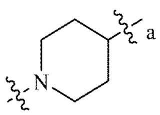, 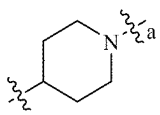 or 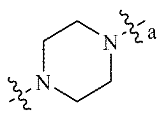,

and a-terminal is linked to Ar.

In some embodiments, Ar is selected from 5-6 membered cycloalkyl, 5-6 membered heterocyclyl, phenyl and 5-6 membered heteroaryl optionally substituted by 1-2 Q groups; each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p-N(R^a)(R^b)$, $-(CHz)_p-O-R^a$, $-(CH_2)_p-P(O)(R^a)(R^b)$, $-(CH_2)_p-S(O)(R^a)$, $-(CH_2)_p-S(O)_2(R^a)$, $-(CH_2)_p-C(O)(R^a)$, $-(CH_2)_p-C(O)O(R^a)$, $-(CH_2)_p-O-C(O)(R^a)$, $-(CH_2)_p-C(O)N(R^a)(R^b)$ and $-(CH_2)_p-N(R^b)-C(O)(R^a)$, and

$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy-$C_{1-6}$ alkyl, cyclopropyl and cyclobutyl.

In some embodiments, Ar is phenyl or 5-6 membered heteroaryl optionally substituted by 1-2 Q groups.

In some embodiments, Ar is phenyl or 5-6 membered nitrogen-containing heteroaryl optionally substituted by 1-2 Q groups.

In some embodiments, Ar is selected from phenyl, pyrrolyl, pyrazolyl, imidazolyl, thiazolyl, oxazolyl, pyridyl, pyrimidinyl, pyrazinyl and pyridazinyl optionally substituted by 1-2 Q groups.

In some embodiments, Ar is phenyl or 6 membered heteroaryl optionally substituted by 1-2 Q groups.

In some embodiments, Ar is phenyl or 6 membered nitrogen-containing heteroaryl optionally substituted by 1-2 Q groups.

In some embodiments, Ar is selected from phenyl, pyridyl, pyrimidinyl, pyrazinyl and pyridazinyl optionally substituted by 1-2 Q groups.

In some embodiments, X, Y and Z are each independently selected from N and C;
ring A, ring B, and X, Y and Z together form the following group:

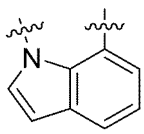, 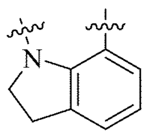, 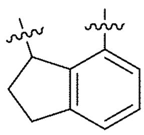, 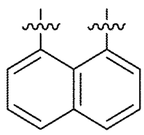, 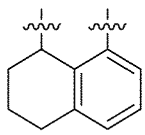, 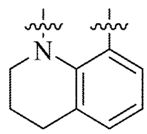, 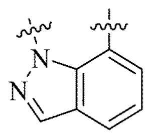,

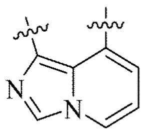, 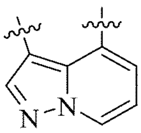, 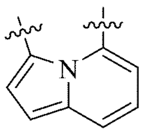, 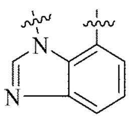 or 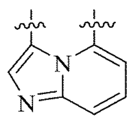;

ring C is selected from 5-6 membered cycloalkyl and 5-6 membered heterocyclyl;

Ar is pyridyl optionally substituted by 1-2 Q groups; each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p-C(O)N(R^a)(R^b)$ and $-(CH_2)_p-N(R^b)-C(O)(R^a)$;

each $R_1$ and each $R_2$ are independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl; or $R_1$, $R_2$ and a carbon atom linked thereto form 3-4 membered cycloalkyl or 3-4 membered heterocyclyl;

$R_1'$ and $R_2'$ are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

each $R_3$, each $R_4$ and each $R_5$ are each independently selected from H, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl and cyclopropyl;

m is 0 or 1;

n and t are each independently selected from 0, 1, 2 and 3;

p and k are each independently selected from 0, 1 and 2;

q is 0, 1, 2, 3 or 4; and

- - - represents the single bond or the double bond .

In some embodiments, X, Y and Z are each independently selected from N and C;
ring A, ring B, and X, Y and Z together form the following group:

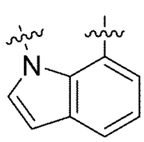, 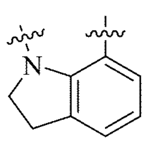, 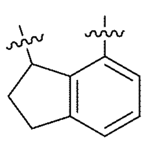, 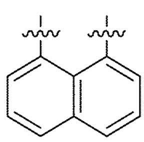, 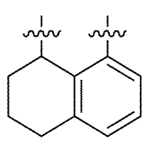, 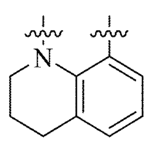, 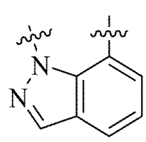,

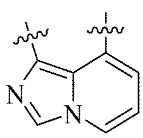, 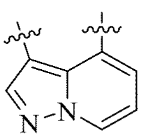, 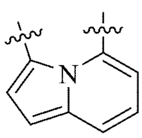, 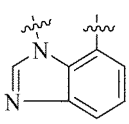 or 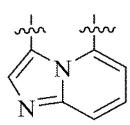;

ring C is selected from the following group:

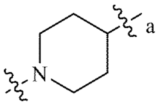, 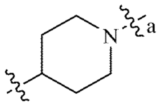 or 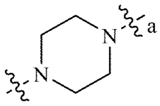,

and a-terminal is linked to Ar;

Ar is pyridyl optionally substituted by 1-2 Q groups; each Q is each independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p-C(O)N(R^a)(R^b)$ and $-(CH_2)_p-N(R^b)-C(O)(R^a)$;

each $R_1$ and each $R_2$ are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio, $C_{1-6}$ alkoxy-$C_{1-6}$ alkyl; or $R_1$, $R_2$ and a carbon atom linked thereto form 3-4 membered cycloalkyl and 3-4 membered heterocyclyl;

$R_1'$ and $R_2'$ are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

each $R_3$, each $R_4$ and each $R_5$ are each independently selected from H, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;

$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl and cyclopropyl;

m is 0 or 1;

n and t are each independently selected from 0, 1, 2 and 3;

p and k are each independently selected from 0, 1 and 2;

q is 0, 1, 2, 3 or 4; and

- - - represents a single bond or a double bond.

In some embodiments, ring C is

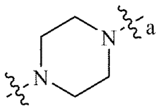,

and a-terminal is linked to Ar and the other terminal is linked to a para-position of X in ring B through alkylene.

In some embodiments, X, Y and Z are each independently selected from N and C;

ring A, ring B, and X, Y and Z together form the following group:

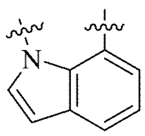, 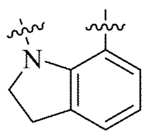, 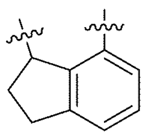, 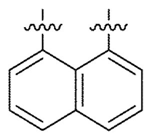, 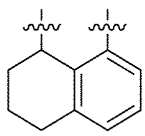, 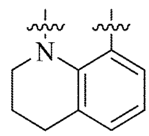, 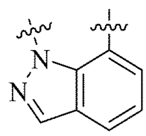,

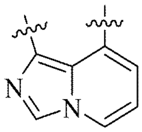, 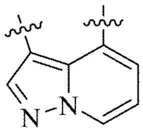, 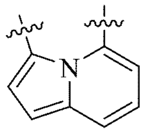, 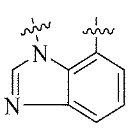 or 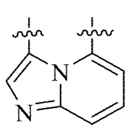;

ring C is

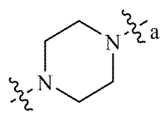,

and a-terminal is linked to Ar and the other terminal is linked to a para-position of X in ring B through alkylene;
Ar is pyridyl optionally substituted by 1-2 Q groups; each Q is each independently selected from H, fluorine, chlorine, hydroxyl, amino, $C_{1-4}$ alkyl, fluoro $C_{1-4}$ alkyl, hydroxyl $C_{1-4}$ alkyl, amino $C_{1-4}$ alkyl, $C_{1-4}$ alkoxy-$C_{1-4}$ alkyl, $C_{1-4}$ alkoxy, fluoro $C_{1-4}$ alkoxy, hydroxyl $C_{1-4}$ alkoxy, amino $C_{1-4}$ alkoxy and -C(O)N($R^a$)($R^b$),
each $R_1$ and each $R_2$ are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl; or $R_1$, $R_2$ and a carbon atom linked thereto form cyclopropyl or cyclobutyl;
$R_1$' and $R_2$' are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl;
each $R_3$, each $R_4$ and each $R_5$ are independently selected from H, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl;
$R^a$ and $R^b$ are each independently selected from hydrogen, methyl, ethyl, isopropyl and cyclopropyl;
m is 0 or 1;
n and t are each independently selected from 0, 1 or 2;
k is 1; and
q is 0 or 1; and
- - - represents a single bond or a double bond.

In some embodiments, ring A, ring B, and X, Y and Z together form the following group:

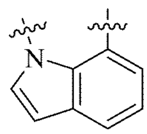, 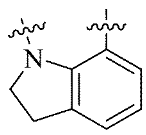, 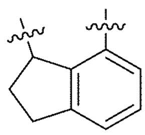, 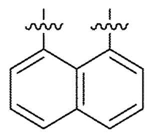, 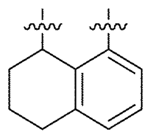, 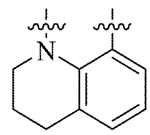, 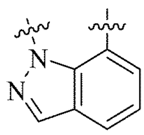,

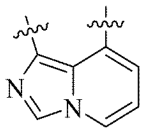, 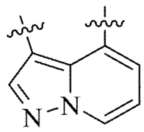, 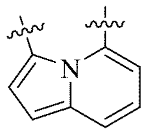, 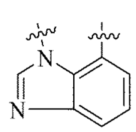 or 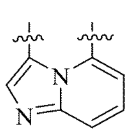;

ring C is

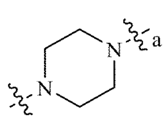

and a-terminal is linked to Ar and the other terminal is linked to a para-position of X in ring B through alkylene;
Ar is selected from pyridyl optionally substituted by 1-2 Q groups; each Q is independently selected from H, fluorine, chlorine, hydroxyl, amino, $C_{1-4}$ alkyl, fluoro $C_{1-4}$ alkyl, hydroxyl $C_{1-4}$ alkyl, amino $C_{1-4}$ alkyl, $C_{1-4}$ alkoxy-$C_{1-4}$ alkyl, $C_{1-4}$ alkoxy, fluoro $C_{1-4}$ alkoxy, hydroxyl $C_{1-4}$ alkoxy, amino $C_{1-4}$ alkoxy and -C(O)N($R^a$)($R^b$),
each $R_1$ and each $R_2$ are independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl and $C_{1-4}$ alkoxy-$C_{1-4}$ alkyl;
or $R_1$, $R_2$ and a carbon atom linked thereto form cyclopropyl or cyclobutyl;
$R_1$' and $R_2$' are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl and $C_{1-4}$ alkoxy-$C_{1-4}$ alkyl;

$R_3$ and $R_4$ are each independently selected from H, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio, $C_{1-4}$ alkoxy-$C_{1-4}$ alkyl;

$R^a$ and $R^b$ are each independently selected from hydrogen, methyl, ethyl, isopropyl or cyclopropyl;

m is selected from 0 or 1;

n and t are each independently selected from 0 or 1;

k is 1;

q is 0; and

- - - represents a single bond or the a bond.

In some embodiments, ring A, ring B, and X, Y and Z together form the following group:

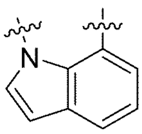, 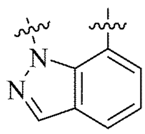, 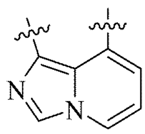, 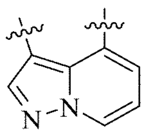, 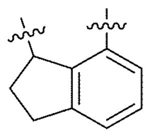 or 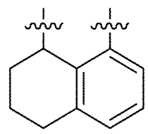;

ring C is

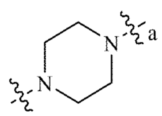

and a-terminal is linked to Ar and the other terminal is linked to a para-position of X in ring B through alkylene;

Ar is pyridyl optionally substituted by 1-2 Q groups; each Q is independently selected from H, fluorine, chlorine, hydroxyl, amino, $C_{1-4}$ alkyl, fluoro $C_{1-4}$ alkyl, hydroxyl $C_{1-4}$ alkyl, amino $C_{1-4}$ alkyl, $C_{1-4}$ alkoxy-$C_{1-4}$ alkyl, $C_{1-4}$ alkoxy, fluoro $C_{1-4}$ alkoxy, hydroxyl $C_{1-4}$ alkoxy, amino $C_{1-4}$ alkoxy and -C(O)N($R^a$)($R^b$);

each $R_1$ and each $R_2$ are independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl and $C_{1-4}$ alkoxy-$C_{1-4}$ alkyl;

or $R_1$, $R_2$ and a carbon atom linked thereto form cyclopropyl or cyclobutyl;

$R_1$' and $R_2$' are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl and $C_{1-4}$ alkoxy-$C_{1-4}$ alkyl;

$R_3$ and $R_4$ are each independently selected from H, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl;

$R^a$ and $R^b$ are each independently selected from hydrogen, methyl, ethyl, isopropyl and cyclopropyl;

m is 0 or 1;

n and t are each independently selected from 0 and 1;

k is 1;

q is 0; and

- - - represents a single bond or a double bond.

In some embodiments, ring A, ring B, and X, Y and Z together form the following group:

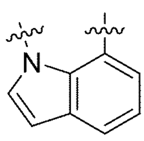, 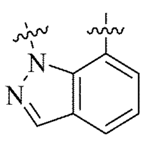, 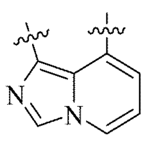 or 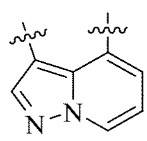;

ring C is

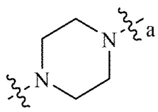

and a-terminal is linked to Ar and the other terminal is linked to a para-position of X in ring B through alkylene;
Ar is pyridyl optionally substituted by 1-2 Q groups; each Q is independently selected from H, fluorine, chlorine, hydroxyl, amino, $C_{1-4}$ alkyl, fluoro $C_{1-4}$ alkyl, hydroxyl $C_{1-4}$ alkyl, amino $C_{1-4}$ alkyl, $C_{1-4}$ alkoxy-$C_{1-4}$ alkyl, $C_{1-4}$ alkoxy, fluoro $C_{1-4}$ alkoxy, hydroxyl $C_{1-4}$ alkoxy, amino $C_{1-4}$ alkoxy and -C(O)N($R^a$)($R^b$);
each $R_1$ and each $R_2$ are independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl and $C_{1-4}$ alkoxy-$C_{1-4}$ alkyl;
or $R_1$, $R_2$ and a carbon atom linked thereto form cyclopropyl or cyclobutyl;
$R_1$' and $R_2$' are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl or $C_{1-4}$ alkoxy-$C_{1-4}$ alkyl;
$R_3$ and $R_4$ are each independently selected from H, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio, $C_{1-4}$ alkoxy-$C_{1-4}$ alkyl;
$R^a$ and $R^b$ are each independently selected from hydrogen, methyl, ethyl, isopropyl or cyclopropyl;
m is selected from 0 or 1;
n and t are each independently selected from 0 or 1;
k is 1;
q is 0; and
- - - represents a single bond or a double bond.

In one aspect, the present application provides a compound as shown in formula (II), a pharmaceutically acceptable salt thereof or a stereoisomer thereof,

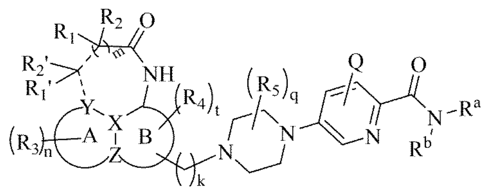

(II),

where, definitions of $R_1$, $R_2$, $R_1$', $R_2$', $R_3$, $R_4$, $R_5$, $R^a$, $R^b$, Q, X, Y, Z, ring A, ring B, m, n, t, k, q and - - - are as described in any of the above embodiments.

In one aspect, the present application provides a compound as shown in formula (II-1), a pharmaceutically acceptable salt thereof or a stereoisomer thereof,

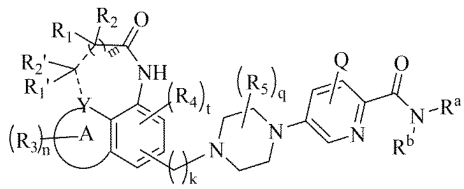

(II-1),

where, definitions of $R_1$, $R_2$, $R_1$', $R_2$', $R_3$, $R_4$, $R_5$, $R^a$, $R^b$, Q, Y, ring A, m, n, t, k, q and --- are as described in any of the above embodiments.

In one aspect, the present application provides a compound as shown in formula (II-2), a pharmaceutically acceptable salt thereof or a stereoisomer thereof,

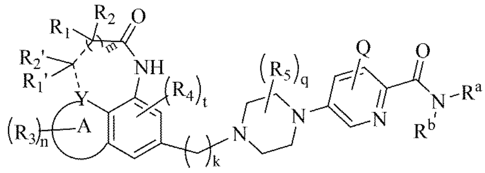

(II-2),

where, definitions of $R_1$, $R_2$, $R_1$', $R_2$', $R_3$, $R_4$, $R_5$, $R^a$, $R^b$, Q, Y, ring A, m, n, t, k, q and --- are as described in any of the above embodiments.

In one aspect, the present application provides a compound as shown in formula (II-3), a pharmaceutically acceptable salt thereof or a stereoisomer thereof,

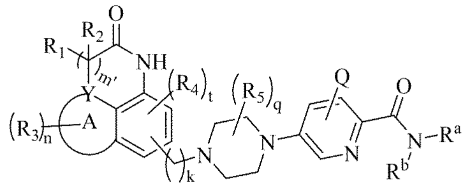

(II-3),

where, m' is 1 or 2, and when m' is 2, two adjacent ring carbon atoms are linked by a single bond or a double bond; definitions of $R_1$, $R_2$, $R_3$, $R_4$, $R_5$, $R^a$, $R^b$, Q, Y, ring A, ring B, n, t, k and q are as described in any of the above embodiments.

In one aspect, the present application provides a compound as shown in formula (II-4), a pharmaceutically acceptable salt thereof or a stereoisomer thereof,

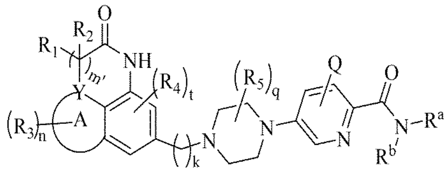

(II-4),

where, m' is 1 or 2, and when m' is 2, two adjacent ring carbon atoms are linked by a single bond or a double bond; definitions of $R_1$, $R_2$, $R_3$, $R_4$, $R_5$, $R^a$, $R^b$, Q, Y, ring A, ring B, n, t, k and q are as described in any of the above embodiments.

In one aspect, the present application provides a compound as shown in formula (II-5), a pharmaceutically acceptable salt thereof or a stereoisomer thereof,

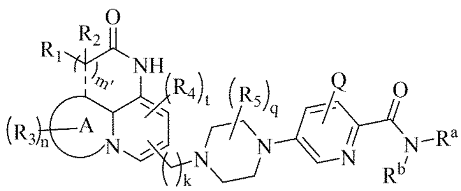

(II-5),

where, m' is 1 or 2, and when m' is 2, two adjacent ring carbon atoms are linked by a single bond or a double bond; - - - represents the single bond or the double bond; and definitions of $R_1$, $R_2$, $R_3$, $R_4$, $R_5$, $R^a$, $R^b$, Q, ring A, ring B, n, t, k and q are as described in any of the above embodiments.

In one aspect, the present application provides a compound as shown in formula (II-6), a pharmaceutically acceptable salt thereof or a stereoisomer thereof,

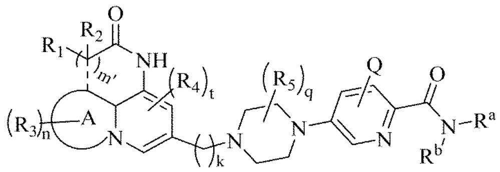

(II-6),

where, m' is 1 or 2, and when m' is 2, two adjacent ring carbon atoms are linked by a single bond or a double bond; - - - represents the single bond or the double bond; and definitions of $R_1$, $R_2$, $R_3$, $R_4$, $R_5$, $R^a$, $R^b$, Q, ring A, ring B, n, t, k and q are as described in any of the above embodiments.

In one aspect, the present application provides a compound as shown in formula (II-7), a pharmaceutically acceptable salt thereof or a stereoisomer thereof,

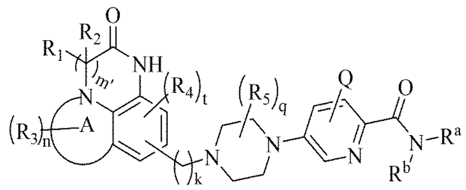

(II-7),

where, m' is 1 or 2, and when m' is 2, two adjacent ring carbon atoms are linked by a single bond or a double bond; definitions of $R_1$, $R_2$, $R_3$, $R_4$, $R_5$, $R^a$, $R^b$, Q, ring A, ring B, n, t, k and q are as described in any of the above embodiments.

In one aspect, the present application provides a compound as shown in formula (II-8), a pharmaceutically acceptable salt thereof or a stereoisomer thereof,

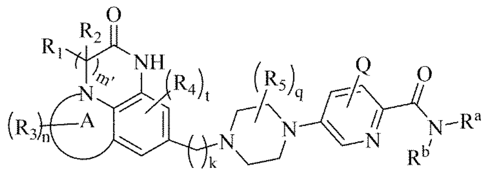

(II-8),

where, m' is 1 or 2, and when m' is 2, two adjacent ring carbon atoms are linked by a single bond or a double bond; definitions of $R_1$, $R_2$, $R_3$, $R_4$, $R_5$, $R^a$, $R^b$, Q, ring A, ring B, n, t, k and q are as described in any of the above embodiments.

In one aspect, the present application provides a compound as shown in formula (III), a pharmaceutically acceptable salt thereof or a stereoisomer thereof,

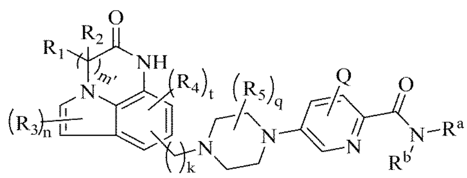

(III),

where, m' is 1 or 2, and when m' is 2, two adjacent ring carbon atoms are linked by a single bond or a double bond; and definitions of $R_1$, $R_2$, $R_3$, $R_4$, $R_5$, $R^a$, $R^b$, Q, n, t, k and q are as described in any of the above embodiments.

In one aspect, the present application provides a compound as shown in formula (III-1), a pharmaceutically acceptable salt thereof or a stereoisomer thereof,

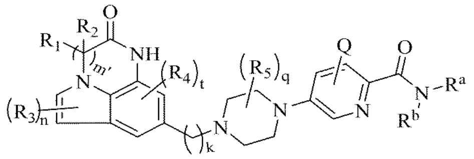

(III-1),

where, m' is 1 or 2, and when m' is 2, two adjacent ring carbon atoms are linked by a single bond or a double bond; and definitions of $R_1$, $R_2$, $R_3$, $R_4$, $R_5$, $R^a$, $R^b$, Q, n, t, k and q are as described in any of the above embodiments.

In one aspect, the present application provides a compound as shown in formula (IV), a pharmaceutically acceptable salt thereof or a stereoisomer thereof,

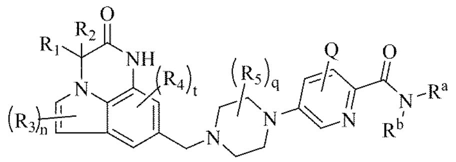

(IV),

where, definitions of $R_1$, $R_2$, $R_3$, $R_4$, $R_5$, $R^a$, $R^b$, Q, n, t and q are as described in any of the above embodiments.

In one aspect, the present application provides a compound as shown in formula (IV-1), a pharmaceutically acceptable salt thereof or a stereoisomer thereof,

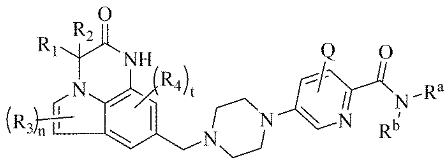

(IV-1),

where, definitions of $R_1$, $R_2$, $R_3$, $R_4$, $R^a$, $R^b$, Q, n and t are as described in any of the above embodiments.

In one aspect, the present application provides a compound as shown in formula (IV-2), a pharmaceutically acceptable salt thereof or a stereoisomer thereof,

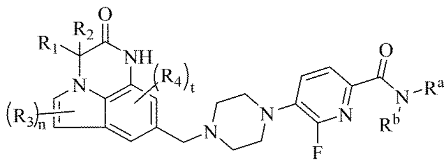

(IV-2),

where, definitions of $R_1$, $R_2$, $R_3$, $R_4$, $R^a$, $R^b$, n and t are as described in any of the above embodiments.

In one aspect, the present application provides a compound as shown in formula (IV-3), a pharmaceutically acceptable salt thereof or a stereoisomer thereof,

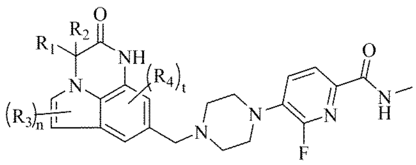

(IV-3),

where, definitions of $R_1$, $R_2$, $R_3$, $R_4$, n and t are as described in any of the above embodiments.

In one aspect, the present application provides a compound as shown in formula (V), a pharmaceutically acceptable salt thereof or a stereoisomer thereof,

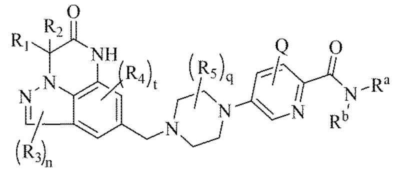

(V),

where, n is 0 or 1; and definitions of $R_1$, $R_2$, $R_3$, $R_4$, $R_5$, $R^a$, $R^b$, Q, t and q are as described in any of the above embodiments.

In one aspect, the present application provides a compound as shown in formula (V-1), a pharmaceutically acceptable salt thereof or a stereoisomer thereof,

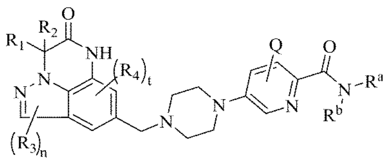

(V-1),

where, n is 0 or 1; and definitions of $R_1$, $R_2$, $R_3$, $R_4$, $R^a$, $R^b$, Q and t are as described in any of the above embodiments.

In one aspect, the present application provides a compound as shown in formula (V-2), a pharmaceutically acceptable salt thereof or a stereoisomer thereof,

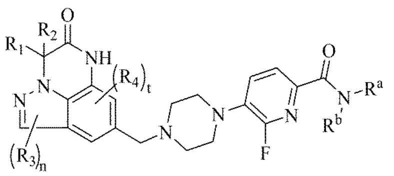

(V-2),

where, n is 0 or 1; and definitions of $R_1$, $R_2$, $R_3$, $R_4$, $R^a$, $R^b$ and t are as described in any of the above embodiments.

In one aspect, the present application provides a compound as shown in formula (V-3), a pharmaceutically acceptable salt thereof or a stereoisomer thereof,

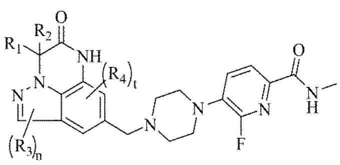

(V-3),

where, n is 0 or 1; and definitions of $R_1$, $R_2$, $R_3$, $R_4$ and t are as described in any of the above embodiments.

In one aspect, the present application provides a compound as shown in formula (VI) or (VII), a pharmaceutically acceptable salt thereof or a stereoisomer thereof,

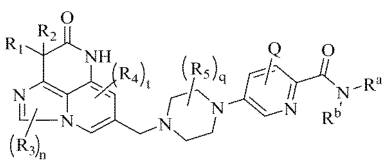

(VI),

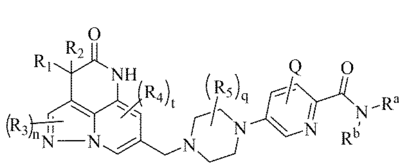

(VII),

where, n is 0 or 1; and definitions of $R_1$, $R_2$, $R_3$, $R_4$, $R_5$, $R^a$, $R^b$, Q, t and q are as described in any of the above embodiments.

In one aspect, the present application provides a compound as shown in formula (VI-1) or (VII-1), a pharmaceutically acceptable salt thereof or a stereoisomer thereof,

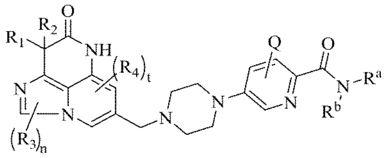 (VI-1), 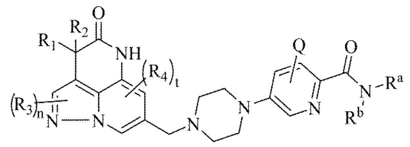 (VII-1),

where, n is 0 or 1; and definitions of $R_1$, $R_2$, $R_3$, $R_4$, $R^a$, $R^b$, Q and t are as described in any of the above embodiments.

In one aspect, the present application provides a compound as shown in formula (VI-2) or (VII-2), a pharmaceutically acceptable salt thereof or a stereoisomer thereof,

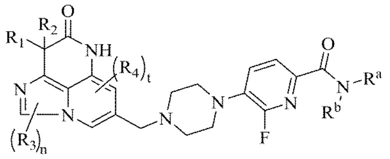 (VI-2), 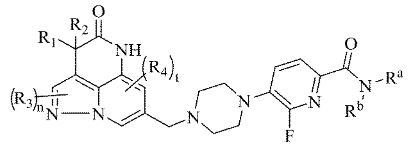 (VII-2),

where, n is 0 or 1; and definitions of $R_1$, $R_2$, $R_3$, $R_4$, $R^a$, $R^b$ and t are as described in any of the above embodiments.

In one aspect, the present application provides a compound as shown in formula (VI-3) or (VII-3), a pharmaceutically acceptable salt thereof or a stereoisomer thereof,

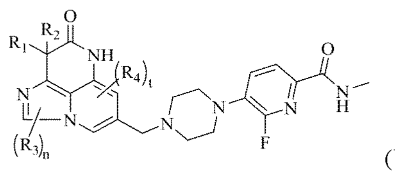 (VI-3), 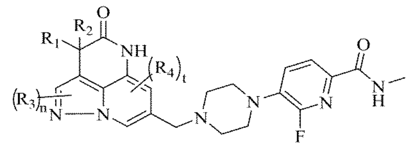 (VII-3),

where, n is 0 or 1; and definitions of $R_1$, $R_2$, $R_3$, $R_4$ and t are as described in any of the above embodiments.

In one aspect, the present application provides a compound as shown below, a pharmaceutically acceptable salt thereof or a stereoisomer thereof:

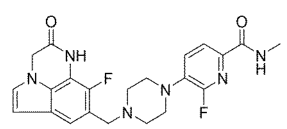
1

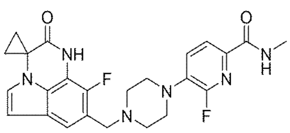
2

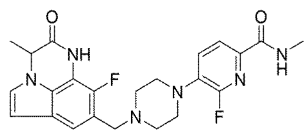
3

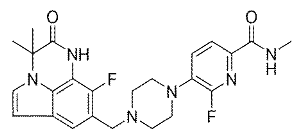
4

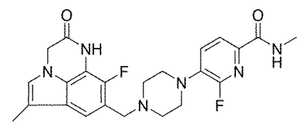
5

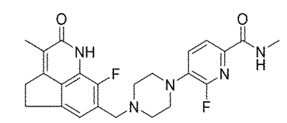
6

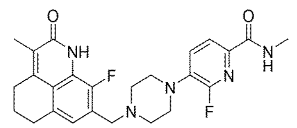
7

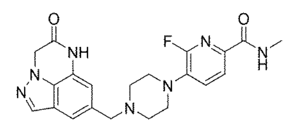
8

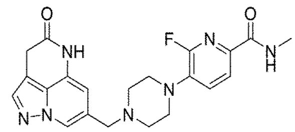
9

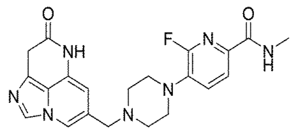
10

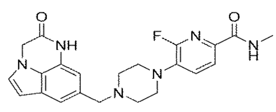
11

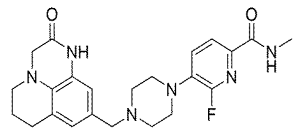
12

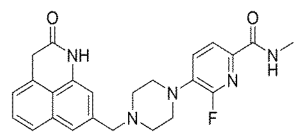
13

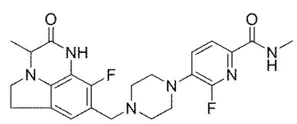
14

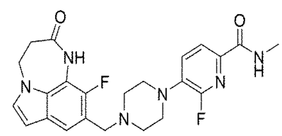
15

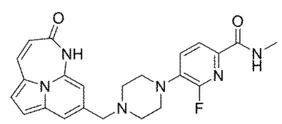
16

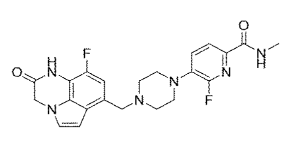
17

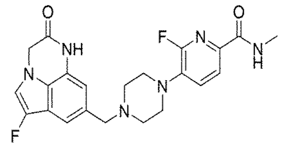
18

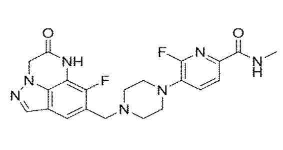
19

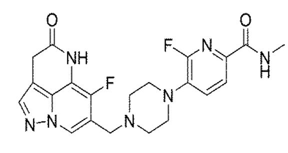
20

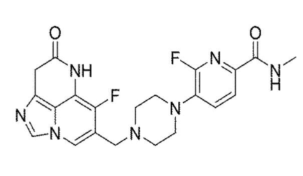
21

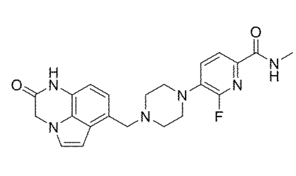
22

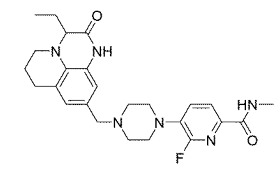
23

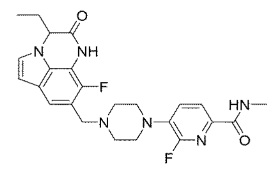
24

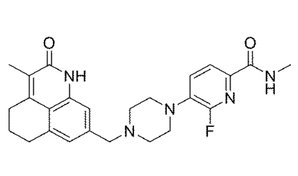
25

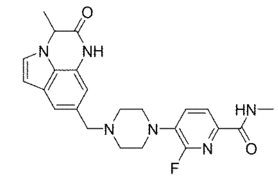
26

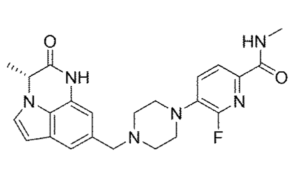
27

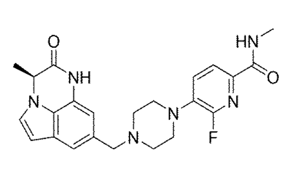
28

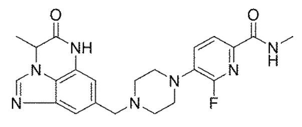
29

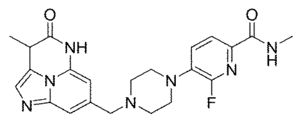
30

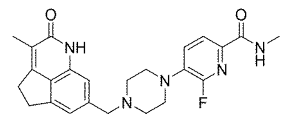
31

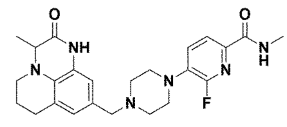
32

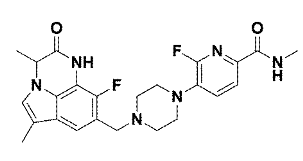
33

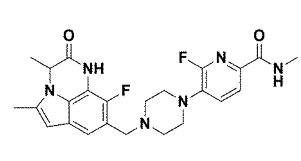
34

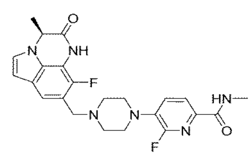
35

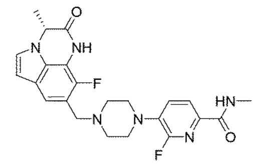
36

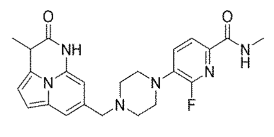
37

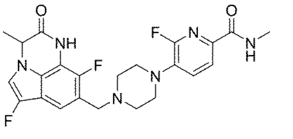
38

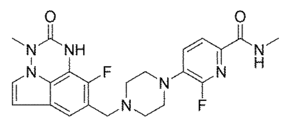
39

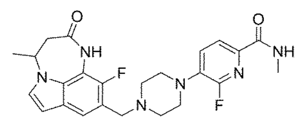
40

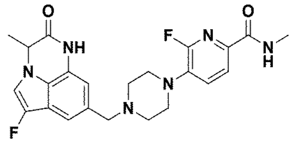
41

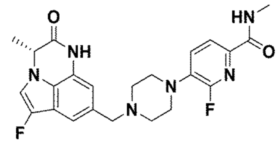
42

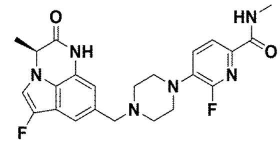
43

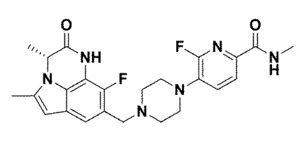
44

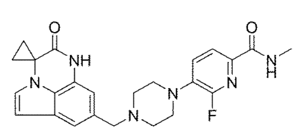
45

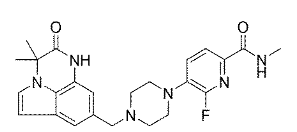
46

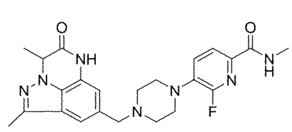
47

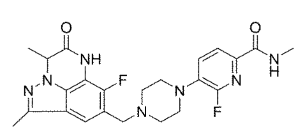
48

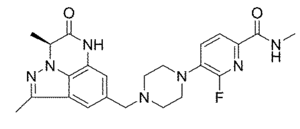 47-1 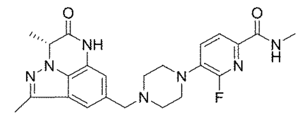 47-2 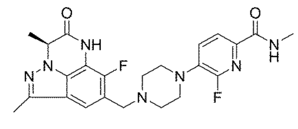 48-1 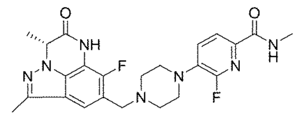 48-2

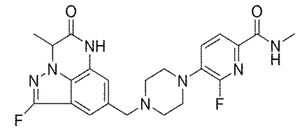 49 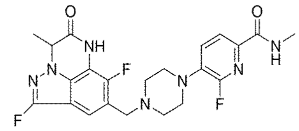 50 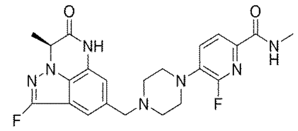 49-1 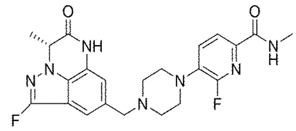 49-2

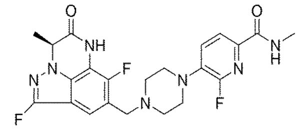 50-1 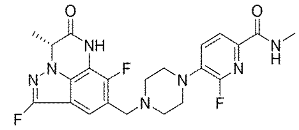 50-2 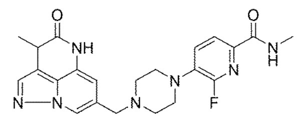 51 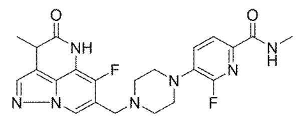 52

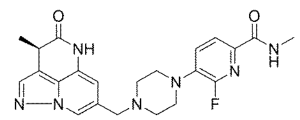 51-1 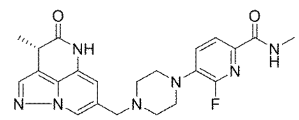 51-2 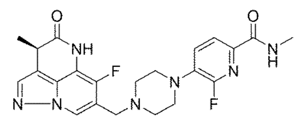 52-1 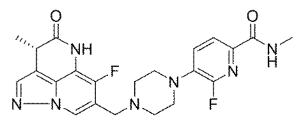 52-2

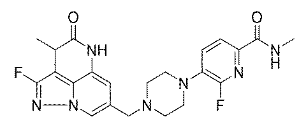 53 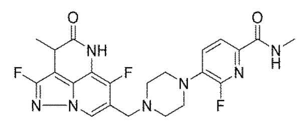 54 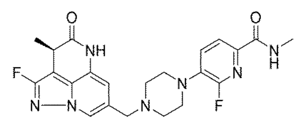 53-1 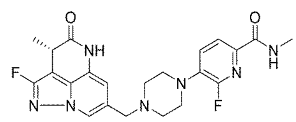 53-2

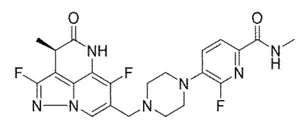 54-1 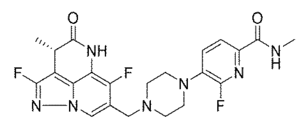 54-2 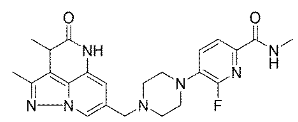 55 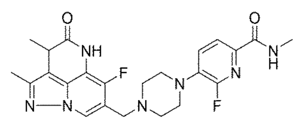 56

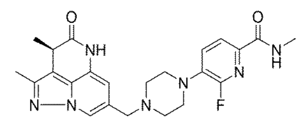 55-1 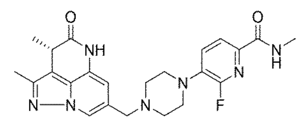 55-2 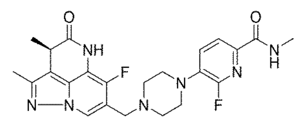 56-1 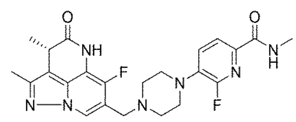 56-2

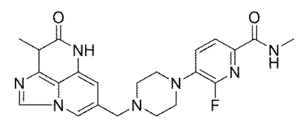 57 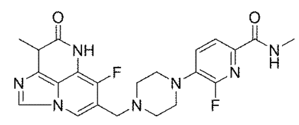 58 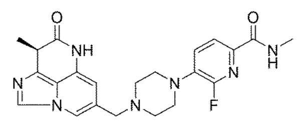 57-1 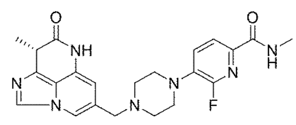 57-2

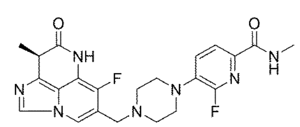 58-1 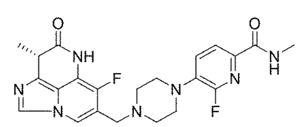 58-2 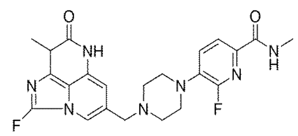 59 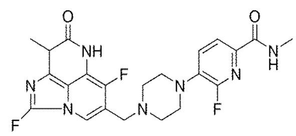 60

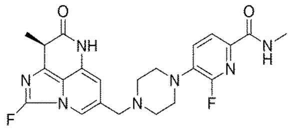
59-1

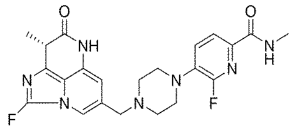
59-2

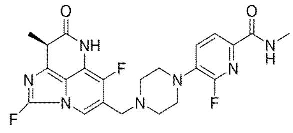
60-1

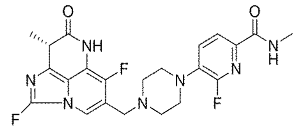
60-2

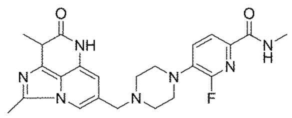
61

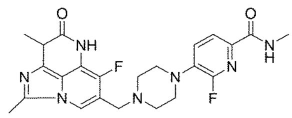
62

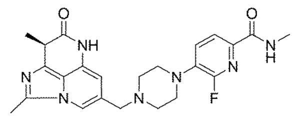
61-1

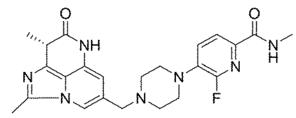
61-2

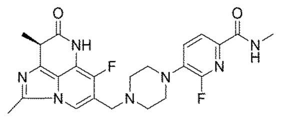
62-1

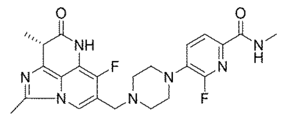
62-2

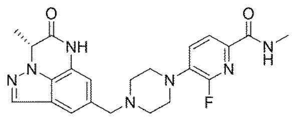
63-1

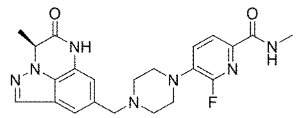
63-2

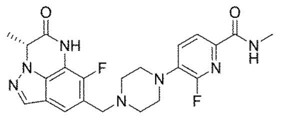
64-1

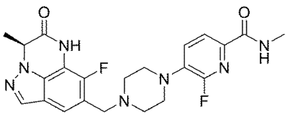
64-2

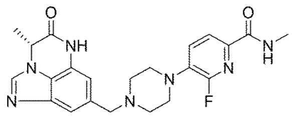
29-1

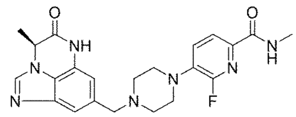
29-2

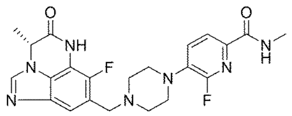
65-1

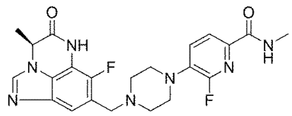
65-2

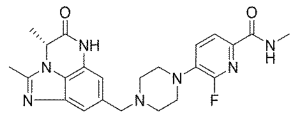
66-1

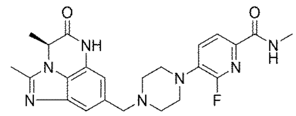
66-2

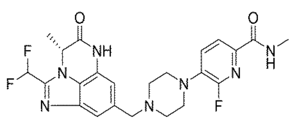
67-1

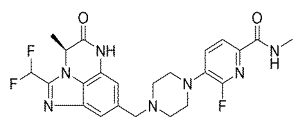
67-2

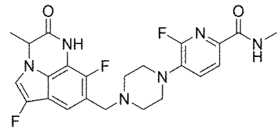
38-1

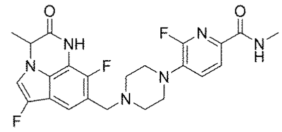
38-2

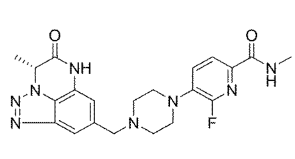
68-1

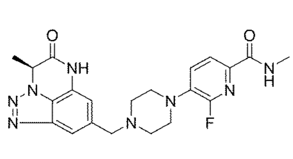
68-2

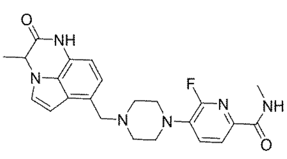
69

In another aspect, the present application further provides a pharmaceutical composition, which includes the compound, the pharmaceutically acceptable salt thereof or the stereoisomer thereof above, and one or more pharmaceutically acceptable excipients, and the pharmaceutical composition may be any one pharmaceutically acceptable dosage form. The pharmaceutically acceptable excipient is a substance that is non-toxic, compatible with active ingredients, and otherwise biologically suitable for living organisms. The selection of specific excipients will depend on the administration mode used to treat specific patients or disease type and status.

In some embodiments, the above pharmaceutical composition may be administered orally, parenterally, rectally or pulmonically to a patient or a subject in need of such treatment. When administered orally, the pharmaceutical composition may be made into an oral formulation, for example, may be made into a conventional oral solid preparation, such as a tablet, a capsule, a pill a granule and the like; and the pharmaceutical composition may also be made into an oral liquid preparation, such as an oral solution, an oral suspension, a syrup, and the like. When administered parenterally, the above pharmaceutical preparation may also be made into an injection, including an injection solution, a sterile powder for

injection and a concentrated solution for injection. When administered rectally, the pharmaceutical composition may be made into a suppository and the like. When administered pulmonically, the pharmaceutical composition may be made into an inhalation preparation, an aerosol, a powder aerosol or a spray, and the like.

In another aspect, the pharmaceutical composition of the present application includes the compound, the pharmaceutically acceptable salt thereof or the stereoisomer thereof above, and may further include one or more second therapeutically active agents.

In another aspect, the present application further relates to a use of the compound, the pharmaceutically acceptable salt thereof or the stereoisomer thereof above in preparing a drug for preventing and/or treating a disease related to PARP overexpression, where the disease is selected from neuropathic pain, epilepsy, stroke, Alzheimer's disease, Parkinson's disease, amyotrophic lateral sclerosis, Huntington's disease, schizophrenia, chronic and acute pain, anemia, neuron damage after hypoxia, neurodegenerative diseases, atherosclerosis, hyperlipidemia, heart tissue damage, coronary artery disease, myocardial infarction, cardiogenic shock, diabetic neuropathy, osteoarthritis and osteoporosis.

In another aspect, the present application further relates to a use of the compound, the pharmaceutically acceptable salt thereof or the stereoisomer thereof above in preparing a drug for preventing and/or treating a cancer.

Further, the present application further relates to a use of the pharmaceutical composition of the compound, the pharmaceutically acceptable salt thereof or the stereoisomer thereof above in preparing a drug for preventing and/or treating a cancer related to PARP overexpression.

In some embodiments, the cancer lacks an HR-dependent DNA DSB repair pathway.

In some embodiments, the cancer includes one or more cancer cells, the cancer cells have a reduced or eliminated ability to repair DNA DSB by HR relative to normal cells.

In some embodiments, the cancer includes one or more cancer cells, and the cancer cells lack BRCA1 and/or BRCA2.

In some embodiments, the cancer includes one or more cancer cells, and the cancer cells have a BRCA1 and/or BRCA2 deficient phenotype.

In another aspect, the present application further provides a method for treating a disease related to PARP, which includes administering an effective amount of the compound, the pharmaceutically acceptable salt thereof or the stereoisomer thereof above, or the pharmaceutical composition above to a patient in need.

Further, the present application further provides a method for treating a cancer, which includes administering an effective amount of the compound, the pharmaceutically acceptable salt thereof or the stereoisomer thereof above, or the pharmaceutical composition above to a patient in need.

In some embodiments, the cancer lacks an HR-dependent DNA DSB repair pathway.

In some embodiments, the cancer includes one or more cancer cells, the cancer cells have a reduced or eliminated ability to repair DNA DSB by HR relative to normal cells.

In some embodiments, the cancer includes one or more cancer cells, and the cancer cells lack BRCA1 and/or BRCA2.

In some embodiments, the cancer includes one or more cancer cells, and the cancer cells have a BRCA1 and/or BRCA2 deficient phenotype.

In another aspect, the present application further provides a kit, which includes an effective amount of one or more of the compound, the pharmaceutically acceptable salt thereof or the stereoisomer thereof above.

In another aspect, the present application further provides a kit, which includes:

(a) an effective amount of one or more of the compound, the pharmaceutically acceptable salt thereof or the stereoisomer thereof above,
and (b) an effective amount of one or more of anticancer agents.

The "effective amount" in the present application refers to a drug dosage sufficient to prevent, alleviate, delay, inhibit or cure the disease of the subject. The drug dosage is related to the administration mode, the pharmacokinetics of drug, the severity of disease, the physical signs (gender, weight, height and age) of subject, and the like.

In the present application, unless otherwise specified, scientific and technical terms used herein have the meanings commonly understood by those skilled in the art. However, in order to better understand the present application, the definitions of some terms are provided below. When the definitions and explanations of terms provided by the present application are inconsistent with those commonly understood by those skilled in the art, the definitions and explanations of terms provided by the present application shall prevail.

The "halogen" in the present application refers to a fluorine atom, a chlorine atom, a bromine atom, or an iodine atom.

The "$C_{1-6}$ alkyl" in the present application refers to a straight chain or branched chain alkyl containing 1-6 carbon atoms, including, for example, "$C_{1-4}$ alkyl", "$C_{1-3}$ alkyl", "$C_{1-2}$ alkyl", "$C_{2-6}$ alkyl", "$C_{2-5}$ alkyl", "$C_{2-4}$ alkyl". "$C_{2-3}$ alkyl", and

the like. Specific examples include, but are not limited to: methyl, ethyl, n-propyl, isopropyl, n-butyl, isobutyl, sec-butyl, tert-butyl, n-pentyl, isopentyl, 2-rnethylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, and the like. The "$C_{1-4}$ alkyl" in the present application refers to a specific example of the $C_{1-6}$ alkyl containing 1-4 carbon atoms.

The "$C_{1-6}$ alkoxy" in the present application refers to "$C_{1-6}$ alkyl-O-", and the "$C_{1-6}$ alkyl" is defined as previously described. The "$C_{1-4}$ alkoxy" in the present application refers to "$C_{1-4}$ alkyl-O-", and the "$C_{1-4}$ alkyl" is defined as previously described.

The "$C_{1-6}$ alkylthio" in the present application refers to "$C_{1-6}$ alkyl-S-", and the "$C_{1-6}$ alkyl" is defined as previously described. The "$C_{1-4}$ alkylthio" in the present application refers to "$C_{1-4}$ alkyl-S-", and the "$C_{1-4}$ alkyl" is defined as previously described.

The "hydroxy $C_{1-6}$ alkyl, amino $C_{1-6}$ alkyl, halo $C_{1-6}$ alkyl, cyano $C_{1-6}$ alkyl and $C_{1-6}$ alkoxy-$C_{1-6}$ alkyl" in the present application refer to one or more hydrogen atoms in the $C_{1-6}$ alkyl being respectively substituted by one or more hydroxyls, aminos, halogens, cyanos or $C_{1-6}$ alkoxys, and the $C_{1-6}$ alkyl and the $C_{1-6}$ alkoxy are defined as previously described.

The "hydroxy $C_{1-6}$ alkoxy, amino $C_{1-6}$ alkoxy, halo $C_{1-6}$ alkoxy and cyano $C_{1-6}$ alkoxy" in the present application refer to one or more hydrogen atoms in the "$C_{1-6}$ alkoxy" being respectively substituted by one or more hydroxyls, aminos, halogens or cyanos.

The "hydroxy $C_{1-6}$ alkylthio, amino $C_{1-6}$ alkylthio and halo $C_{1-6}$ alkylthio" in the present application refer to one or more hydrogen atoms in the "$C_{1-6}$ alkylthio" being respectively substituted by one or more hydroxyls, aminos or halogens.

The "fluoro $C_{1-6}$ alkyl" and "fluoro $C_{1-6}$ alkoxy" in the present application respectively refer to one or more hydrogen atoms in the "$C_{1-6}$ alkyl" and "$C_{1-6}$ alkoxy" being substituted by one or more fluorine atoms.

The "3-11 membered heterocyclyl" in the present application refers to a saturated or partially saturated monocyclic or polycyclic cyclic group without aromaticity containing at least one heteroatom or group (such as 1, 2, 3, 4 or 5 heteroatoms or groups) and having 3-11 ring atoms, the heteroatom or group is selected from a nitrogen atom, an oxygen atom and a sulfur atom, and optionally, the ring atom (such as a carbon atom, a nitrogen atom or a sulfur atom) in the ring structure may be oxygenated. The "3-11 membered heterocyclyl" in the present application includes, but is not limited to "3-10 membered heterocyclyl", "3-8 membered monoheterocyclyl", "8-11 membered fused heterocyclyl", "8-11 membered spiro heterocyclyl" and "7-9 membered bridged heterocyclyl".

The "3-8 membered monoheterocyclyl" in the present application refers to a saturated or partially saturated monocyclic cyclic group without aromaticity that containing at least one heteroatom (such as 1, 2, 3, 4 or 5 heteroatoms) and having 3-8 ring atoms, the heteroatom is a nitrogen atom, an oxygen atom and/or a sulfur atom, and optionally, the ring atom (such as a carbon atom, a nitrogen atom or a sulfur atom) in the ring structure may be oxygenated. The "3-8 membered monoheterocyclyl" in the present application includes "3-8 membered saturated monoheterocyclyl" and "3-8 membered partially saturated monoheterocyclyl". Preferably, the "3-8 membered monoheterocyclyl" in the present application contains 1-3 heteroatoms; preferably, the "3-8 membered monoheterocyclyl" in the present application contains 1-2 heteroatoms, and the heteroatom is selected from a nitrogen atom and/or an oxygen atom; and preferably, the "3-8 membered monoheterocyclyl" in the present application contains 1 heteroatom, and the heteroatom is a nitrogen atom, an oxygen atom and/or a sulfur atom. The "3-8 membered monoheterocyclyl" is preferably "3-7 membered monoheterocyclyl", "3-6 membered monoheterocyclyl", "4-7 membered monoheterocyclyl", "4-6 membered monoheterocyclyl", "6-8 membered monoheterocyclyl", "5-7 membered monoheterocyclyl" and "5-7 membered saturated monoheterocyclyl", "5-7 membered partially saturated monoheterocyclyl", "5-6 membered monoheterocyclyl", "5-6 membered saturated monoheterocyclyl", "5-6 membered partially saturated monoheterocyclyl", "3-6 membered saturated monoheterocyclyl", "5-6 membered saturated monoheterocyclyl", "3-6 membered nitrogen-containing monoheterocyclyl", "3-6 membered saturated nitrogen-containing monoheterocyclyl", "5-6 membered nitrogen-containing monoheterocyclyl", "5-6 membered saturated nitrogen-containing monoheterocyclyl, "5-6 membered partially saturated nitrogen-containing monoheterocyclyl", "6 membered saturated monoheterocyclyl", "6 membered saturated nitrogen-containing monoheterocyclyl, and the like. Specific examples of the "3-8 membered monoheterocyclyl" include, but are not limited to: azacyclopropyl, 2H-azacyclopropyl, diazacyclopropyl, 3H-diazacyclopropenyl, azacyclobutyl, oxacyclobutyl, 1,4-dioxacyclohexyl, 1,3-dioxacyclohexyl, 1,3-dioxacyclopentyl, 1,4-dioxacyclohexdienyl, tetrahydrofuranyl, dihydropyrrolyl, pyrrolidinyl, imidazolidinyl, 4,5-dihydroimidazolyl, pyrazolidinyl, 4,5-dihydropyrazolyl, 2,5-dihydrothienyl, tetrahydrothienyl, 4,5-dihydrothiazolyl, thiazolidinyl, piperidyl, tetrahydropyridyl, piperidinonyl, tetrahydropyridonyl, dihydropiperidinonyl, piperazinyl, morpholinyl, 4,5-dihydrooxazolyl, 4,5-dihydroisoxazolyl, 2,3-dihydroisoxazolyl, oxazolidinyl, 2H-1,2-oxazinyl, 4H-1,2-oxazinyl, and the like.

The "8-11 membered fused heterocyclyl" in the present application refers to a saturated or partially saturated cyclic group without aromaticity that is formed by two or more cyclic structures sharing two adjacent atoms with each other and contains 8-11 ring atoms, where at least one ring atom is a heteroatom, one of the fused rings may be an aromatic ring, but the fused ring has no aromaticity as a whole, and the heteroatom is a nitrogen atom, an oxygen atom and/or a sulfur

atom. Optionally, the ring atom (such as a carbon atom, a nitrogen atom or a sulfur atom) in the ring structure may be oxygenated. Specific examples include, but are not limited to dihydrofuropyridine, 3,4-dihydro-2H-pyranopyridine, 3,4-dihydro-2H-oxazopyridine, dihydrooxazopyrimidine, benzodihydrofuryl,

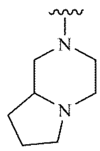 , 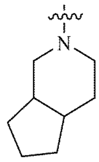 ,

and the like.

The "8-11 membered spiro heterocyclyl" in the present application refers to a saturated or partially saturated cyclic structure that is formed by two or more cyclic structures sharing one ring atom with each other and contains 8-11 ring atoms, where at least one ring atom is a heteroatom or group, such as N, NH, O, S, CO, SO and $SO_2$, etc, and the number of heteroatoms or groups is preferably 1, 2, 3, 4 or 5, and further preferably 1 or 2. For example, the "8-11 membered spiro heterocyclyl" includes "9-11 membered spiro heterocyclyl", "9-11 membered saturated spiro heterocyclyl", "9-11 membered partially saturated spiro heterocyclyl", and the like. Specific examples include, but are not limited to:

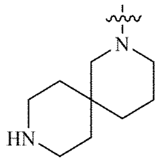 ,

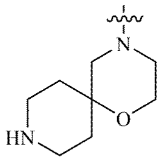 , 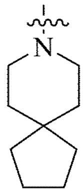 , 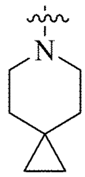 and 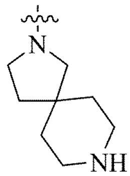 .

The "7-9 membered bridged heterocyclyl" in the present application refers to a saturated or partially saturated cyclic structure that is formed by two or more cyclic structures sharing two non-adjacent ring atoms with each other and contains 7-9 ring atoms, where at least one ring atom is a heteroatom or group, such as N, NH, O, S, CO, SO and $SO_2$, etc, and the number of heteroatoms or groups is preferably 1, 2, 3, 4 or 5, and further preferably 1 or 2. For example, the "7-9 membered bridged heterocyclyl" includes "7-8 membered bridged heterocyclyl", "7-8 membered saturated bridged heterocyclyl", "8 membered bridged heterocyclyl", "8 membered saturated bridged heterocyclyl", and the like. Specific examples include, but are not limited to:

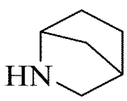 , 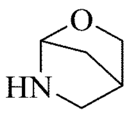 , 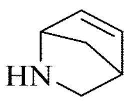 , 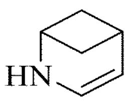 , 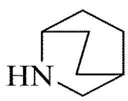 , 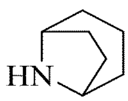 and 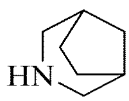 .

The "3-11 member cycloalkyl" in the present application refers to a saturated or partially saturated monocyclic or polycyclic cyclic group without aromaticity containing 3-11 ring atoms, and the "3-11 membered cycloalkyl" in the present application includes, but is not limited to "3-10 membered cycloalkyl", "3-8 membered monocyclic cycloalkyl", "5-7 membered monocyclic cycloalkyl", "5-6 membered monocyclic cycloalkyl", "8-11 membered fused cycloalkyl", "8-11 membered spiro cycloalkyl" and "7-9 membered bridged cycloalkyl". The "3-8 membered monocyclic cycloalkyl" includes, but is not limited to cyclopentyl, cyclohexyl and

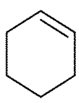 .

The "8-11 membered spiro cycloalkyl" in the present application refers to a saturated or partially saturated cyclic

structure that is formed by two or more cyclic structures sharing one ring atom with each other and contains 8-11 ring carbon atoms, such as "9-11 membered spiro cycloalkyl", "9-11 membered saturated spiro cycloalkyl", "9-11 membered partially saturated spiro cycloalkyl", and the like. Specific examples include, but are not limited to:

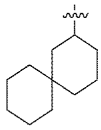 , 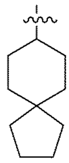 , 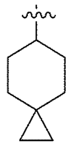

and

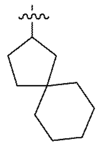 .

The "7-9 membered bridged cycloalkyl" in the present application refers to a saturated or partially saturated cyclic structure that is formed by two or more cyclic structures sharing two non-adjacent ring atoms with each other and contains 7-9 ring carbon atoms, such as "7-8 membered bridged cycloalkyl", "7-8 membered saturated bridged cycloalkyl", "8 membered bridged cycloalkyl", "8 membered saturated bridged cycloalkyl", "8 membered partially saturated bridged cycloalkyl", and the like. Specific examples include, but are not limited to:

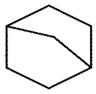 , 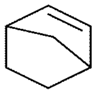 , 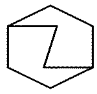 and 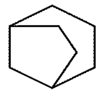 .

The "8-11 membered fused cycloalkyl" in the present application refers to a saturated or partially saturated cyclic group without aromaticity that is formed by two or more cyclic structures sharing two adjacent atoms with each other and contains 8-11 ring carbon atoms, one of the fused rings may be an aromatic ring, but the fused ring has no aromaticity as a whole. Specific examples include, but are not limited to:

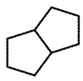 , 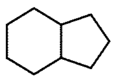 , 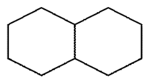 , 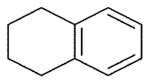 , 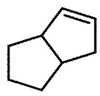

and

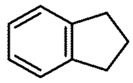 .

The definition of "8-11 membered fused cyclyl" is the same as that of "8-11 membered fused cycloalkyl".

The "6-11 membered aryl" in the present application includes "6-8 membered monocyclic aryl" and "8-11 membered fused cyclic aryl".

The "6-8 membered monocyclic aryl" in the present application refers to monocyclic aryl containing 6-8 ring carbon atoms, and examples include, but are not limited to: phenyl, cyclooctatetraenyl, and the like; and phenyl is preferred.

The "5-11 membered heteroaryl" in the present application includes "5-8 membered monocyclic heteroaryl" and "8-11 membered fused heteroaryl".

The "5-8 membered monocyclic heteroaryl" in the present application refers to a monocyclic cyclic group with aromaticity that containing 5-8 ring atoms (where at least one ring atom is a heteroatom, such as a nitrogen atom, an oxygen atom or a sulfur atom). Optionally, the ring atom (such as a carbon atom, a nitrogen atom or a sulfur atom) in the ring structure may be oxygenated. The "5-8 membered monocyclic heteroaryl" includes, for example, "5-7 membered monocyclic heteroaryl", "5-6 membered monocyclic heteroaryl", "5-6 membered nitrogen-containing monocyclic heteroaryl", "6 membered nitrogen-containing monocyclic heteroaryl", and the like. The heteroatom in the "nitrogen-containing

heteroaryl" contains at least one nitrogen atom, such as containing only 1 or 2 nitrogen atoms, or containing one nitrogen atom and other 1 or 2 heteroatoms (such as an oxygen atom and/or a sulfur atom), or containing 2 nitrogen atoms and other 1 or 2 heteroatoms (such as an oxygen atom and/or a sulfur atom). Specific examples of "5-8 membered monocyclic heteroaryl" include, but are not limited to furyl, thienyl, pyrrolyl, thiazolyl, isothiazolyl, thiadiazole, oxazolyl, isoxazole, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, 2-pyridonyl, 4-pyridonyl, pyrimidinyl, pyridazinyl, pyrazinyl, 1,2,3-triazinyl, 1,3,5-triazinyl, 1,2,4,5-tetrazinyl, aza-cycloheptatrienyl, 1,3-diaza-cycloheptatrienyl, aza-cyclooctatetraenyl, and the like. The "5-6 membered monocyclic heteroaryl" refers to a specific example in which 5-8 membered heteroaryl contains 5-6 ring atoms.

The "8-10 membered fused heteroaryl" in the present application refers to an unsaturated ring structure with aromaticity that is formed by two or more ring structures sharing two adjacent atoms with each other and contains 8-10 ring atoms (where at least one ring atom is a heteroatom, such as a nitrogen atom, an oxygen atom or a sulfur atom). Optionally, the ring atom (such as a carbon atom, a nitrogen atom or a sulfur atom) in the ring structure may be oxygenated. The "8-10 membered fused heteroaryl" includes "9-10 membered fused heteroaryl", "8-9 membered fused heteroaryl" and the like, and a fusion mode may be benzo 5-6 membered heteroaryl, 5-6 membered heteroaryl-o-5-6 membered heteroaryl, and the like. Specific examples include, but are not limited to: pyrrolopyrrole, pyrrolofuran, pyrazolopyrrole, pyrazolothiophene, furanothiophene, pyrazoloxazole, benzofuryl, benzoisofuryl, benzothiophenyl, indolyl, isoindolyl, benzoxazolyl, benzimidazolyl, indazolyl, benzotriazolyl, quinolyl, 2-quinolinonyl, 4-quinolinonyl, 1-isoquinolinonyl, isoquinolinyl, acridinyl, phenanthridinyl, benzopyridazinyl, phthalazinyl, quinazolinyl, quinoxalinyl, purinyl, naphthyridinyl, and the like.

The "oxo group" in the present application refers to a structure in which, when the substituted position is a carbon atom, a nitrogen atom or a sulfur atom, the carbon atom, the nitrogen atom or the sulfur atom may be oxygenated to form C=O, N=O, S=O or $SO_2$.

The "optionally substituted" in the present application refers to two situations in which one or more hydrogen atoms on the substituent may be "substituted" or "unsubstituted" by one or more substituents.

The full English name of "DSB" in the present application is Double-strand breaks, which refers to DNA double-strand breaks.

The "HR" in the present application refers to Homologous Recombination.

The "CH" in the present application refers to the following structure:

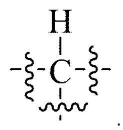

The "N" in the present application refers to the following structure:

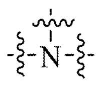

The "C" in the present application refers to the following structure:

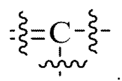

The "$-(CH_2)_p-P(O)(R^a)(R^b)$" in the present application refers to

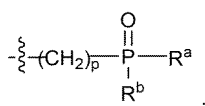

Definitions of other similar groups in the present application are the same as that of "$-(CH_2)_p-P(O)(R^a)(R^b)$".

The "each $R^1$" in the present application means that, when m is 2, 3 or 4, each $R_1$ in multiple $R_1$ is independently selected from the groups described in the above technical solution.

The "each $R^2$" in the present application means that, when n is 2, 3 or 4, each $R^2$ in multiple $R^2$ is independently selected from the groups described in the above technical solution.

The "optionally substituted" in the present application refers to two situations in which one or more hydrogen atoms on the substituted group are "substituted" or "unsubstituted" by one or more substituents.

When ring A contains NH, such as

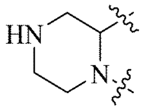

H in NH on the ring may be substituted by $R^1$.

When ring B is selected from nitrogen-containing heterocyclyl or heteroaryl, and contains NH, H in NH on the ring may be substituted by $R^2$.

The "pharmaceutically acceptable salt" in the present application refers to a salt formed by an acidic functional group (such as -COOH, -OH, and $-SO_3H$) existing in the compound with an appropriate inorganic or organic cation (base), including a salt formed with alkali metal or alkaline earth metal, an ammonium salt, and a salt formed with a nitrogen-containing organic base; and a salt formed by a basic functional group (such as $-NH_2$) existing in the compound with an appropriate inorganic or organic anion (acid), including a salt formed with an inorganic acid or an organic acid (such as a carboxylic acid, etc.).

The "stereisomer" in the present application means that the compound of the present application contains one or more asymmetric centers, so it may be used as a raceme and racemic mixture, a single enantiomer, a diastereomer mixture, and a single diastereomer. The compound of the present application may have the asymmetric centers, and such asymmetric centers each independently generate two optical stereoisomers. The scope of the present application includes all possible optical stereoisomers and their mixtures.

If the compound in the present application contains an olefin double bond, unless otherwise specified, it includes a cis-isomer and a trans-isomer. The compound of the present application may exist in the form of tautomer (one of functional group stereoisomers), and it has different hydrogen linkage points by one or more double bond shifts, for example, ketone and its enol form are keto-enol tautomers. Each tautomer and the mixture thereof are all included in the scope of the present application.

The stereisomers, cis-trans isomers, tautomers, geometric isomers, epimers and the mixtures thereof, and the like of all compounds are included in the scope of the present application.

The compound of the present application may be prepared in the form of individual enantiomer by enantiospecific synthesis or resolution from the enantiomer mixture. Conventional resolution technologies include the use of various well-known chromatographic methods to split the enantiomer mixture of a starting substance or a final product.

While the stereochemistry of the compound disclosed is named or described by the structure, the named or described stereisomer is at least 60%, 70%, 80%, 90%, 99% or 99.9% pure by weight relative to other stereisomers. While the single isomer is named or described by the structure, the described or named enantiomer is at least 60%, 70%, 80%, 90%, 99%, or 99.9% pure by weight. The weight % of optical purity is a ratio of the weight of enantiomer to the weight of enantiomer plus its optical isomers.

**Beneficial effects of invention**

1. The compound of the present application, the pharmaceutically acceptable salt thereof or the stereisomer thereof has the excellent PARP1 inhibitory effect, can effectively inhibit the growth of tumor cells, and has the good pharmacokinetic properties in the living organisms (such as a mouse, a rat and a dog), the effect is long-lasting, and the bioavailability is high.
2. The compound of the present application, the pharmaceutically acceptable salt thereof or the stereisomer thereof has the better therapeutic effect on the cancer, and the stability of liver microsomes is high.
3. The preparation process of the compound of the present application is simple, the drug purity is high, the quality is stable, and it is easy to perform large-scale industrial production.

**DETAILED DESCRIPTIONS OF THE EMBODIMENTS**

Technical solutions of the present application are described hereinafter with reference to specific embodiments, and the above contents of the present application are further described in detail, However, this should not be understood as limiting the scope of the above subject matter of the present application to the following embodiments. All technologies achieved based on the above contents of the present application belong to the scope of the present application.

Abbreviation:

NBS: N-bromosuccinimide; TBSCl: tert-butyldimethylsilyl chloride; TBAF: tetrabutyl ammonium fluoride; Prep-TLC: preparative thin layer chromatography; Xphos Pd G2: chlorine(2-dicyclohexylphosphino-2',4',6'-triisopropyl-1,1'-biphenyl)[2-(2'-amino-1,1'-biphenyl)]palladium (II); DIPEA: N,N-diisopropylethylamine; DIEA: N,N-diisopropylethylamine; DMF: N,N-dimethylformamide; DBU: 1,8-diazabicyclo[5.4.0]undec-7-ene; Xantphos: 4,5-bis diphenylphosphines-9,9-dimethylxanthene; $Tf_2O$: trifluoromethanesulfonic anhydride; select F: 1-chloromethyl-4-fluoro-1,4-diazabicyclo[2.2.2]octane bis(tetrafluoroborate); LDA: lithium diisopropylamide; $Pd_2(dba)_3$: tris(dibenzylideneacetone)dipalladium; RuPhosPdG3: methanesulfonato(2-dicyclohexylphosphino-2',6'-diisopropoxy-1,1'-biphenyl)(2-amino-1,1'-biphenyl-2-yl)palladium (II); DAST: diethylaminosulfur trifluoride; T3P: 1-propylphosphonic anhydride; DIAD: diisopropyl azodicarboxylate.

**Example 1: Preparation of 6-fluoro-5-(4-((9-fluoro-2-oxo-2,3-dihydro-1H-pyrrolo[1,2,3-de]quinoxaline-8-yl)methyl)piperazine-1-yl)-N-methylpicolin amide (Compound 1)**

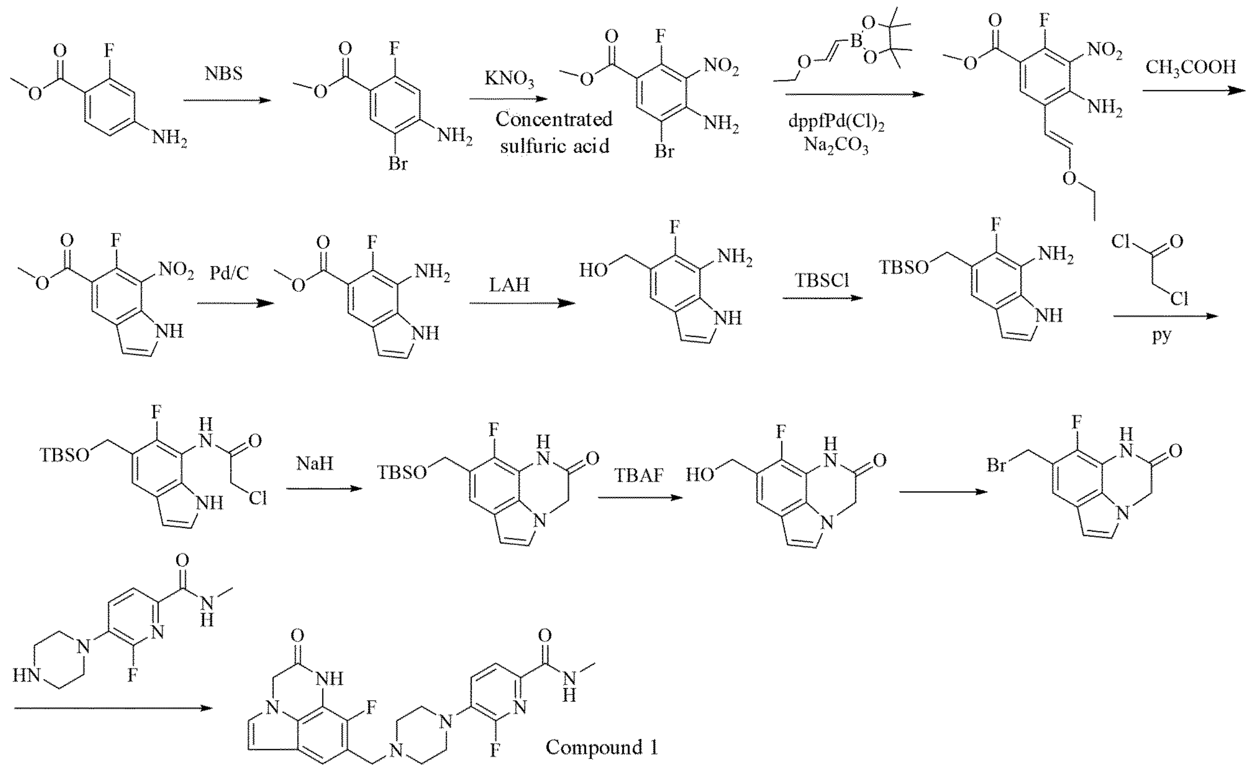


Compound 1

**1. Preparation of methyl 4-amino-5-bromo-2-fluorobenzoate**

Methyl 4-amino-2-fluorobenzoate (20.0 g, 118.3 mmol) was dissolved in acetonitrile (300 mL), and added with NBS (19.0 g, 106.5 mmol) at 0°C to react at 0°C for 1 hour. Then, the reaction solution was transferred to an environment at 25°C to react for 8 hours, and the reaction was finished. The reaction solution was purified by spin-drying column chromatography (ethyl acetate/petroleum ether=0-20%) to obtain the product (21.0 g, yield: 71.9%).

**2. Preparation of methyl 4-amino-5-bromo-2-fluoro-3-nitrobenzoate**

The methyl 4-amino-5-bromo-2-fluorobenzoate (21.0 g, 85.0 mmol) was dissolved in concentrated sulfuric acid (300 mL), and slowly added with potassium nitrate (9.5 g, 93.5 mmol) at 0°C to continuously react for 0.5 hour, and the reaction was finished. The reaction solution was added into ice water for quenching, and extracted with ethyl acetate. An organic phase was collected, concentrated, and purified by column chromatography (ethyl acetate/petroleum ether=0-20%) to obtain the target compound (24.0 g, yield: 96.4%).

**3. Preparation of (E)-4-amino-5-(2-ethoxyvinyl)-2-fluoro-3-nitrobenzoate**

The methyl 4-amino-5-bromo-2-fluoro-3-nitrobenzoate (12.0 g, 41.0 mmol) and (E)-2-(2-ethoxyvinyl)-4,4,5,5-tetramethyl-1,3,2-dioxborane (9.7 g, 49.2 mmol) were dissolved in dioxane (200 mL) and water (40 mL), and added with sodium carbonate (13.0 g, 123.0 mmol) and Pd(dppf)$Cl_2$ (3.0 g, 4.1 mmol) to react at 100°C for 6 hours under the protection of nitrogen, and the reaction was finished. The reaction solution was extracted with water (200 mL) and ethyl acetate (200 mLx2). An organic phase was collected, and the crude product was purified by silica gel column chromatography (n-heptane: ethyl acetate=3: 1) to obtain the product (6.0 g, yield 51.3%).

**4. Preparation of methyl 6-fluoro-7-nitro-1H-indole-5-carboxylate**

The (E)-4-amino-5-(2-ethoxyvinyl)-2-fluoro-3-nitrobenzoate (6.0 g, 21.0 mmol) was dissolved in glacial acetic acid (70 mL) to react at 125°C for 0.5 hour, and the reaction was finished. The reaction solution was concentrated, and crude ethyl acetate was pulped to obtain the product (3.6 g, yield 71.3%).

**5. Preparation of methyl 7-amino-6-fluoro-1H-indole-5-carboxylate**

The methyl 6-fluoro-7-nitro-1H-indole-5-carboxylate (3.6 g, 15.1 mmol) was dissolved in methanol (20 mL), and added with Pd/C (550 mg, N/A). The mixture was subjected to gas exchange with hydrogen for 3 times to react at 25°C for 3 hours under hydrogen, and the reaction was finished. The reaction solution was filtered to obtain the product (3.0 g, yield 95.1%).

**6. Preparation of (7-amino-6-fluoro-1H-indole-5-yl)methanol**

The methyl 7-amino-6-fluoro-1H-indole-5-carboxylate (700 mg, 3.3 mmol) was dissolved in tetrahydrofuran (10 mL), and added with lithium aluminum hydride (380 mg, 10.0 mmol) at 0°C to react at 70°C for 1 hour, and the reaction was finished. The reaction solution was quenched with water, and concentrated, and the crude product was purified by silica gel column chromatography (ethyl acetate/petroleum ether=0-30%) to obtain the product (400 mg, yield 67.3%).

**7. Preparation of 5-((tert-butyldimethylsilyl)oxy)methyl)-6-fluoro-1H-indole-7-amine**

The (7-amino-6-fluoro-1H-indole-5-yl)methanol (350 mg, 1.9 mmol) was dissolved in dichloromethane (20 mL), and added with imidazole (258 mg, 3.8 mmol) and TBSCl (437 mg, 2.9 mmol) to react at 25°C for 1 hour, and the reaction was finished. The reaction solution was concentrated, and subjected to column chromatography (ethyl acetate/petroleum ether=0-20%) to obtain the target compound (400 mg, yield 71.5%).

**8. Preparation of N-(5-((tert-butyldimethylsilyl)oxy)methyl)-6-fluoro-1H-indole-7-yl)-2-chloroacetamide**

The 5-((tert-butyldimethylsilyl)oxy)methyl)-6-fluoro-1H-indole-7-amine (350 mg, 1.2 mmol) was dissolved in dichloromethane (8 mL), and added with pyridine (142 mg, 1.8 mmol) and chloroacetyl chloride (203 mg, 1.8 mmol) at 0°C to react at 25°C for 1 hour, and the reaction was finished. The reaction solution was concentrated, and the crude product was purified by silica gel column chromatography (ethyl acetate/petroleum ether=0-30%) to obtain the product (300 mg, yield 67.4%).

**9. Preparation of 8-((tert-butyldimethylsilyl)oxy)methyl)-9-fluoro-1H-pyrrolo[1,2,3-de]quinoxaline-2(3H)-one**

The N-(5-((tert-butyldimethylsilyl)oxy)methyl)-6-fluoro-1H-indole-7-yl)-2-chloroacetamide (250 mg, 0.67 mmol) was dissolved in DMF (5 mL), and added with NaH (60%)(80 mg, 2.0 mmol) to react at 25°C for 0.5 hour, and the reaction was finished. The reaction solution was extracted with water and ethyl acetate, and an organic phase was collected and concentrated. The crude product was purified by silica gel column chromatography (ethyl acetate/petroleum ether=0-50%) to obtain the product (100 mg, yield 44.5%).

**10. Preparation of 9-fluoro-8-(hydroxymethyl)-1H pyrrolo[1,2,3-de]quinoxaline-2(3H)-one**

The 8-((tert-butyldimethylsilyl)oxy)methyl)-9-fluoro-1H-pyrrolo[1,2,3-de]quinoxaline-2(3H)-one (80 mg, 0.24 mmol) was dissolved in tetrahydrofuran (3 mL), and added with TBAF (0.50 mL) to react at 25°C for 1 hour, and the reaction was finished. The reaction solution was concentrated, washed with water, and filtered to obtain the product (40mg, yield 75.4%).

**11. Preparation of 8-(bromomethyl)-9-fluoro-1H-pyrrolo[1,2,3-de]quinoxaline-2(3H)-one**

The 9-fluoro-8-(hydroxymethyl)-1H pyrrolo[1,2,3-de]quinoxaline-2(3H)-one (20 mg, 0.09 mmol) was dissolved in dichloromethane (3 mL), and added with triphenylphosphine (37 mg, 0.14 mmol) and tetrabromomethane (46 mg, 0.14 mmol) at 0°C to react at 0°C for 6 hours, and the reaction was finished. The reaction solution was concentrated, and directly used in the next step.

**12. Preparation of 6-fluoro-5-(4-((9-fluoro-2-oxo-2,3-dihydro-1H-pyrrolo[1,2,3-de]quinoxaline-8-yl)methyl)piperazine-1-yl)-N-methylpicolin amide**

6-fluoro-N-methyl-5-(piperazine-1-yl)picolinamide hydrochloride (39 mg, 0.14 mmol) and the 8-(bromomethyl)-9-fluoro-1H-pyrrolo[1,2,3-de]quinoxaline-2(3H)-one (the crude product of the previous step) were dissolved in acetonitrile (4 mL), and added with N,N-diisopropylethylamine (36 mg, 0.28 mmol) to react at 25°C for 2 hours, and the reaction was finished. The reaction solution was concentrated, and purified by TLC (methanol/dichloromethane=1/10) to obtain the product (5.8 mg, two-step yield: 14.6%).

Molecular formula: $C_{22}H_{22}F_2N_6O_2$, Molecular weight: 440.2, LC-MS (M/e): 441.2 (M +H$^+$)
$^1$H-NMR(400MHz, DMSO-$d_6$) $\delta$: 11.08 (s, 1H), 8.41-8.39 (m, 1H), 7.83 (d, 1H, $J$=7.64), 7.31-7.30 (m, 1H), 7.29 (s, 1H), 7.10-7.09 (m, 1H), 6.46-6.45 (m, 1H), 4.99 (s, 2H), 3.61 (s, 2H), 3.17-3.14 (m, 4H), 2.77-2.75 (m, 3H), 2.50-2.47 (s, 4H).

**Example 2: Preparation of 6-fluoro-5-(4-(9-fluoro-3-methyl-2-oxy-2,3-dihydro-1H-pyrrole[1,2,3-de]quinoxaline-8-yl)methyl)piperazine-1-yl)-n-meth ylpicolinamide (Compound 3)**

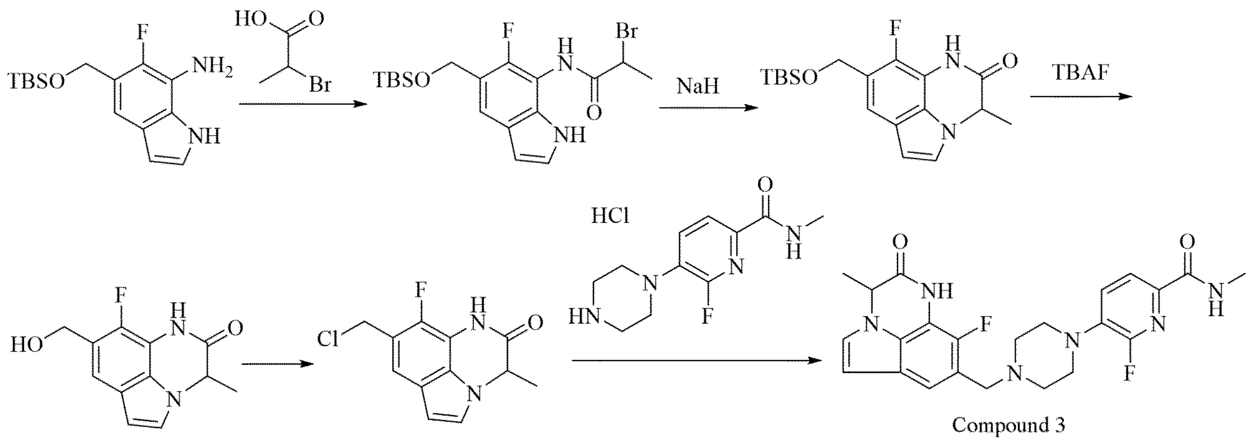


Compound 3

**1. Preparation of 2-bromo-N-(5-((tert-butyldimethylsilyl) oxy)methyl)-6-fluoro-1H-indole-7-yl)propionamide**

The 5-((tert-butyldimethylsilyl)oxy)methyl)-6-fluoro-1H-indole-7-amine (500 mg, 1.7 mmol) was dissolved in ethyl acetate (50 mL), added with pyridine (500 mg, 6.3 mmol) and 2-bromopropionic acid (550 mg, 3.6 mmol), and added with 1-propylphosphoric anhydride (w50% 3.3 g, 5.2 mmol) at -50°C to react for 2 hours, and then the reaction solution was added with water for quenching. The reaction solution was concentrated, and the crude product was purified by silica gel column chromatography (ethyl acetate/petroleum ether=0-40%) to obtain the product (300 mg, yield 41.2%).

**2. Preparation of 8-((tert-butyldimethylsilyl)oxy)methyl)-9-fluoro-3-methyl-1H-pyrrole[1,2,3-de]quinoxaline-2(3H)-1 one**

The 2-bromo-N-(5-((tert-butyldimethylsilyl) oxy)methyl)-6-fluoro-1H-indole-7-yl)propionamide (250 mg, 0.58 mmol) was dissolved in DMF(5 mL), and added with NaH (60%)(50 mg, 1.3 mmol) to react at 25 °C for 0.5 hour, and the reaction was finished. The reaction solution was extracted with water and ethyl acetate. An organic phase was collected and concentrated, and the crude product was directly used in the next step of reaction.

**3. Preparation of 9-fluoro-8-(hydroxymethyl)-3-methyl-1H-pyrrole[1,2,3-de]quinoxaline-2(3H)-one**

The crude product of the previous step was dissolved in tetrahydrofuran (10 mL), and added with TBAF (5 mL) to react at 25°C for 1 hour, and the reaction was finished. The reaction solution was concentrated, washed with water, and purified by column chromatography (EA: PE=100%) to obtain the product (100 mg, two-step yield 73.3%).

**4. Preparation of 8-(chloromethyl)-9-fluoro-1H-pyrrolo[1,2,3-de]quinoxaline-2(3H)-one**

The 9-fluoro-8-(hydroxymethyl)-3-methyl-1h-pyrrole[1,2,3-de]quinoxaline-2(3H)-one (80 mg, 0.34 mmol) was dissolved in dichloromethane (5 mL), and added with thionyl chloride (450 mg, 3.8 mmol) and DMF (0.1 mL) at 0°C to react at 30 °C for 1 hour, and the reaction was finished. The reaction solution was concentrated, and directly used in the next step.

**5. Preparation of 6-fluoro-5-(4-(9-fluoro-3-methyl-2-oxy-2,3-dihydro-1H-pyrrole[1,2,3-de]quinoxaline-8-yl) methyl)piperazine-1-yl)-n-meth ylpicolinamide**

6-fluoro-N-methyl-5-(piperazine-1-yl)picolinamide hydrochloride (200 mg, 0.73 mmol) and the 8-(chloromethyl)-9-fluoro-1H-pyrrolo[1,2,3-de]quinoxaline-2(3H)-one (the crude product of the previous step) were dissolved in acetonitrile (10 mL), and added with N,N-diisopropylethylamine (250 mg, 1.9 mmol) to react at 25°C for 2 hours, and the reaction was finished. The reaction solution was concentrated, purified by TLC (methanol/dichloromethane=10%), and then purified by reversed-phase column chromatography (MeOH: $H_2O$=70%) to obtain the product (7 mg, two-step yield 4.7%).

Molecular formula: $C_{23}H_{24}F_2N_6O_2$, Molecular weight: 454.5, LC-MS (M/e): 455.3 (M +$H^+$)
$^1$H-NMR(400MHz, DMSO-$d_6$) $\delta$: 8.09 (s, 1H), 7.99 (d, J=7.88Hz, 1H), 7.50 (d, J=4.68Hz, 1H), 7.28-7.32 (m, 1H), 7.14-7.19 (m, 2H), 6.54 (d, J=2.88Hz, 1H), 5.13-5.18 (m, 1H), 3.71-3.77 (m, 2H), 3.22-3.26 (m, 4H), 3.00-3.05(m, 3H), 2.65-2.72 (s, 4H), 1.85-1.87 (m, 3H).

**Example 3: Preparation of 6-fluoro-N-methyl-5-(4-((2-oxo-2,3-dihydro-1H-pyrazolo[1,5,4-de]quinoxaline-8-yl) methyl)piperazine-1-yl)picolinamide (Compound 8)**

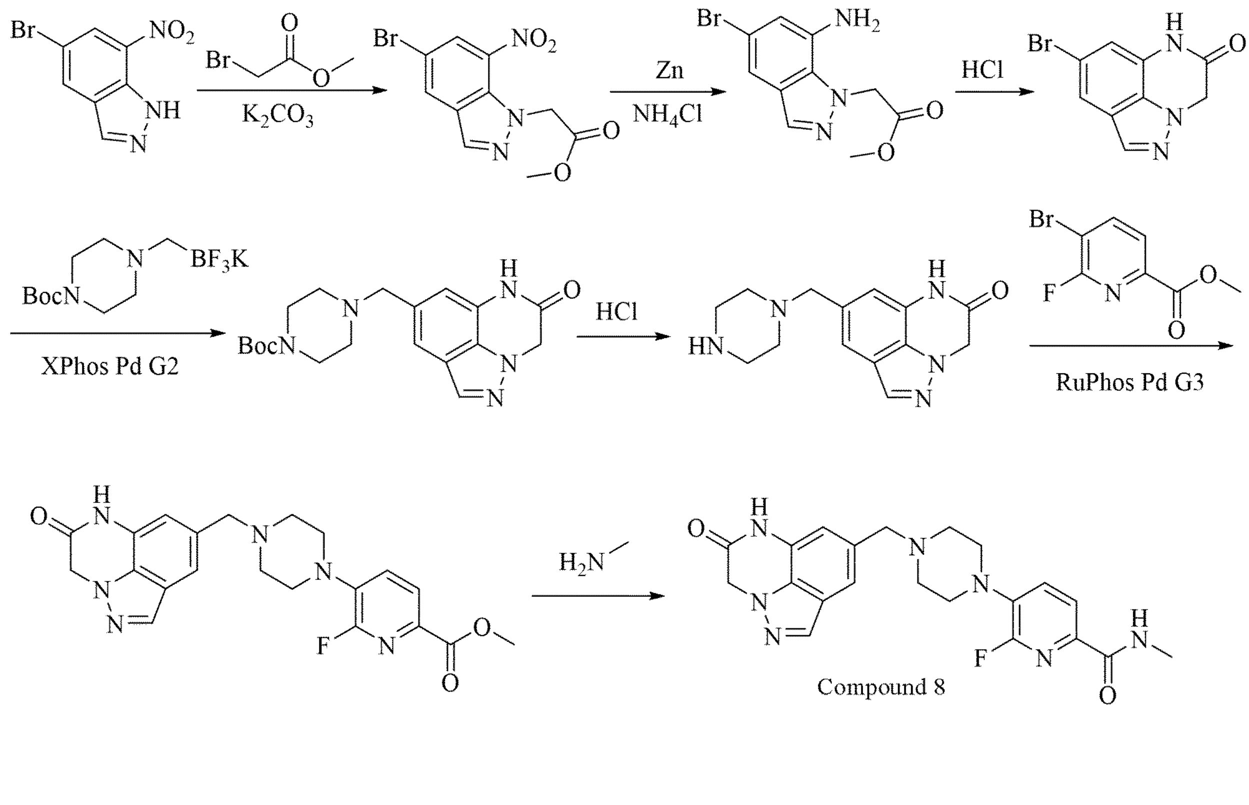


Compound 8

**1. Preparation of methyl 2-(5-bromo-7-nitro-1H-indazole-1-yl)acetate**

5-bromo-7-nitro-1H-indazole (3.0 g, 12.4 mmol) and methyl bromoacetate (2.3 g, 15.0 mmol) were dissolved in acetonitrile (100 mL), and added with potassium carbonate (5.1 g, 36.9 mmol) to react at 50 °C for 3 hours. The reaction solution was filtered to remove solids, and the crude product was purified by silica gel column chromatography (n-heptane: ethyl acetate=3: 1) to obtain the product (800 mg, yield 20.5%).

**2. Preparation of methyl 2-(7-amino-5-bromo-1H-indazole-1-yl)acetate**

The methyl 2-(5-bromo-7-nitro-1H-indazole-1-yl)acetate (800 mg, 2.5 mmol) was dissolved in methanol/water (20 mL/2 mL), and added with zinc powder (1.3 g, 19.9 mmol) and ammonium chloride (1.1 g, 20.6 mmol) to react at 50 °C for 3 hours. The reaction solution was filtered to remove solids, concentrated, extracted with water (50 mL) and ethyl acetate (50 mL), dried, and then concentrated to obtain the crude product (500 mg).

**3. Preparation of 8-bromo-1H-pyrazolo[1,5,4-de]quinoxaline-2(3H)-one**

The methyl 2-(7-amino-5-bromo-1H-indazole-1-yl)acetate (500 mg, the crude product) was dissolved in methanol (10 mL), and added with an ethyl acetate solution of hydrogen chloride (4 M)(2 mL) to react at 25°C for 1 hour. The reaction solution was concentrated, added with a saturated sodium bicarbonate solution to adjust a pH value to be 8-9, and extracted with water (20 mL) and ethyl acetate (30 mL). The crude product was purified by silica gel column chromatography (n-heptane: ethyl acetate =3: 1) to obtain the product (300 mg, two-step yield 46.7%).

**4. Preparation of tert-butyl 4-((2-oxo-2,3-dihydro-1H-pyrazolo[1,5,4-de]quinoxaline-8-yl)methyl)piperazine-1-carboxylate**

The 8-bromo-1H-pyrazolo[1,5,4-de]quinoxaline-2(3H)-one (350 mg, 1.4 mmol) and potassium(4-tert-butoxycarbonyl piperazine-1-yl)methyl trifluoroborate (560 mg, 1.8 mmol) were dissolved in 1,4-dioxane/water (15 mL/2.5 mL), added with XPhos Pd G2 (112 mg, 0.14 mmol) and cesium carbonate (910 mg, 2.8 mmol), and subjected to gas exchange with nitrogen to react at 80°C for 2 hours under nitrogen. The reaction solution was concentrated, and the crude product was purified by silica gel column chromatography (n-heptane: ethyl acetate=2: 1) to obtain the product (270 mg, yield: 52.4%).

**5. Preparation of 8-(piperazine-1-ylmethyl)-1H-pyrazolo[1,5,4-de]quinoxaline-2(3H)-one**

The tert-butyl 4-((2-oxo-2,3-dihydro-1H-pyrazolo[1,5,4-de]quinoxaline-8-yl)methyl)piperazine-1-carboxylate (270 mg, 0.73 mmol) was dissolved in dichloromethane (5 mL), and added with an ethyl acetate solution of hydrogen chloride (4 M)(1 mL) to react at 25°C for 1 hour. The reaction solution was concentrated, added with a saturated sodium bicarbonate solution to adjust a pH value to be 8-9, and extracted with water (20 mL) and ethyl acetate (30 mL). The crude product was purified by silica gel column chromatography (dichloromethane: methanol=12: 1) to obtain the product (50 mg, yield 25.4%).

**6. Preparation of methyl 6-fluoro-5-(4-((2-oxo-2,3-dihydro-1H-pyrazolo[1,5,4-de]quinoxaline-8-yl)methyl)piperazine-1-yl)picolinate**

The 8-(piperazine-1-ylmethyl)-1H-pyrazolo[1,5,4-de]quinoxaline-2(3H)-one (50 mg, 0.18 mmol) and methyl 5-bromo-6-fluoropicolinate (65 mg, 0.28 mmol) were dissolved in 1,4-dioxane (5 mL), added with RuPhos Pd G3 (10 mg, 0.026 mmol) and cesium carbonate (179 mg, 0.55 mmol), and subjected to gas exchange with nitrogen to react by microwave at 140°C for 1 hour under nitrogen. The reaction solution was concentrated, and the crude product was purified by silica gel column chromatography (dichloromethane: methanol=20: 1) to obtain the product (15 mg, yield 19.2%).

**7. Preparation of 6-fluoro-N-methyl-5-(4-((2-oxo-2,3-dihydro-1H-pyrazolo[1,5,4-de]quinoxaline-8-yl)methyl)piperazine-1-yl)picolinamide**

The methyl 6-fluoro-5-(4-((2-oxo-2,3-dihydro-1H-pyrazolo[1,5,4-de]quinoxaline-8-yl)methyl)piperazine-1-yl)picolinate (15 mg, 0.035 mmol) was dissolved in acetonitrile (3 mL), and added with an aqueous solution of methylamine (0.5 mL) to react at 25°C for 3 hours, and the reaction was finished. The reaction solution was concentrated, and purified by Prep-TLC (dichloromethane: methanol=12: 1) to obtain a crude product (5 mg), and then the crude product was purified by Prep-HPLC (water: methanol=1:8) to obtain the product (3.5 mg, yield 23.4%).

Molecular formula: $C_{21}H_{22}FN_7O_2$, Molecular weight: 423.5, LC-MS (M/e): 424.2 (M +$H^+$)
$^1$H-NMR (400MHz, DMSO-$d_6$) $\delta$: 11.07 (s, 1H), 8.40 (s, 1H), 8.01 (s, 1H), 7.83 (d, *J*=8.0 Hz, 1H), 7.56 (t, *J*=8.0 Hz, 1H),7.20 (s, 1H), 6.74 (s, 1H), 5.19 (s, 2H), 3.60-3.40 (m, 2H), 3.20-3.15 (m, 4H), 2.76 (d, J=4.8 Hz, 3H), 2.60-2.40 (m, 4H)

**Example 4: Preparation of 6-fluoro-N-methyl-5-(4-((2-oxo-2,3-dihydro-1H-pyrrolo[1,2,3-de]quinoxaline-8-yl) methyl)piperazine-1-yl)picolinamide (Compound 11)**

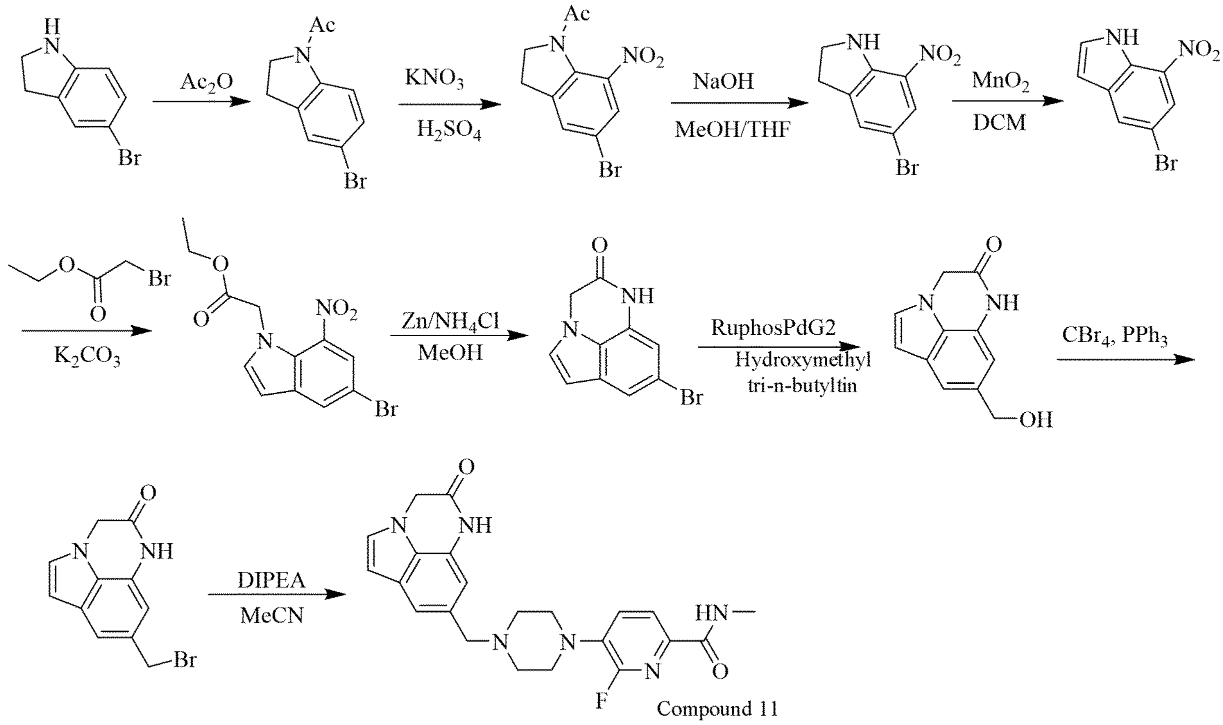


Compound 11

**1. Preparation of 1-(5-bromoindole-1-yl)ethane-1-one**

5-bromoindoline (3.9 g, 19.8 mmol) was dissolved in acetic acid (20 mL) and cooled to 0°C, and slowly added with acetic anhydride (20 mL) to react at 25°C for 1 hour, and the reaction was finished. The reaction solution was concentrated to obtain the product (4.0 g), and the product was directly used in the next step.

**2. Preparation of 1-(5-bromo-7-nitroindole-1-yl)ethyl-1-one**

The 1-(5-bromoindole-1-yl)ethane-1-one (3.7 g, the crude product) was dissolved in concentrated sulfuric acid (50 mL), and added with potassium nitrate (1.9 g, 18.5 mmol) at 0°C to react at 0°C for 1 hour. The reaction solution was poured into cold water to precipitate solids, filtered, and dried to obtain a crude product (3.6 g), and the crude product was directly used in the next step.

**3. Preparation of 5-bromo-7-nitroindoline**

The 1-(5-bromo-7-nitroindole-1-yl)ethyl-1-one (3.4 g, 11.9 mmol) and sodium hydroxide (4.8 g, 119 mmol) were dissolved in THF(40 mL), and added with MeOH (8 mL) to react at 25°C for 4 hours, and the reaction was finished. The reaction solution was concentrated, the residue was washed with water, filtered and dried to obtain the product (2.9 g), and the product was directly used in the next step.

**4. Preparation of 5-bromo-7-nitro-1H-indole**

The 5-bromo-7-nitroindoline (2.9 g, the crude product) was dissolved in DCM (50 mL), and added with

manganese dioxide (9.5 g, 106.6 mmol) to react at 25°C for 1 hour, and the reaction was finished. The reaction solution was filtered with diatomite, the filtrate was concentrated, and the residue was purified by silica gel column chromatography (n-heptane: ethyl acetate=5: 1) to obtain the product (2.4 g).

**5. Preparation of ethyl 2-(5-bromo-7-nitro-1H-indole-1-yl)acetate**

The 5-bromo-7-nitro-1H-indole (2.1 g, 8.7 mmol) was dissolved in acetonitrile (40 mL), and added with potassium carbonate (1.8 g, 13.0 mmol) and ethyl bromoacetate (2.2 g, 13.2 mmol) to react at 50°C for 2 hours, and the reaction was finished. The reaction solution was extracted with water (30 mL) and ethyl acetate (40 mLx2). Organic phases were combined and concentrated, and the crude product was purified by silica gel column chromatography (ethyl acetate: petroleum ether=0-70%) to obtain the product (2.4 g, yield 84.1%).

**6. Preparation of 8-bromo-1H-pyrrolo[1,2,3-*de*]quinoxaline-2(3H)-one**

The ethyl 2-(5-bromo-7-nitro-1H-indole-1-yl)acetate (2.2 g, 6.7 mmol) and zinc powder (4.3 g, 66.1 mmol) were added into methanol (30 mL), and added with water (3 mL) and ammonium chloride (3.7 g, 66.0 mmol) to react at 50°C for 1 hour, and the reaction was finished. The reaction solution was filtered with diatomite, the filtrate was concentrated, and the crude product was purified by silica gel column chromatography (ethyl acetate: petroleum ether=0-100%) to obtain the product (260 mg, yield 15.4%).

**7. Preparation of 8-(hydroxymethyl)-1H pyrrolo[1,2,3-*de*]quinoxaline-2(3H)-one**

The 8-bromo-1H-pyrrolo[1,2,3-de]quinoxaline-2(3H)-one (230 mg, 0.92 mmol) was dissolved in dioxane (10 mL), and added with Ruphos Pd G 2 (79 mg, 0.085 mmol) and hydroxymethyl tri-n-butyltin (518 mg, 1.6 mmol) to react at 75°C for 3 hours under the protection of nitrogen, and the reaction was finished. The reaction solution was cooled and concentrated, and the crude product was purified by silica gel column chromatography (ethyl acetate: petroleum ether=0-100%) to obtain the product (90 mg, yield 48.4%).

**8. Preparation of 8-(bromomethyl)-1H-pyrrolo[1,2,3-*de*]quinoxaline-2(3H)-one**

The 8-(hydroxymethyl)-1H pyrrolo[1,2,3-*de*]quinoxaline-2(3H)-one (70 mg, 0.35 mmol), triphenylphosphine (136 mg, 0.52 mmol) and carbon tetrabromide (172 mg, 0.52 mmol) were dissolved in DCM (10 mL) to react at 25°C for 1 hour, and the reaction was finished. The reaction solution was concentrated at 25°C, and the crude product was directly used in the next step.

**9. Preparation of 6-fluoro-N-methyl 5-(4-((2-oxo-2,3-dihydro-1H-pyrrolo[1,2,3-*de*]quinoxaline-8-yl)methyl)piperazine-1-yl)picolinamide**

The 8-(bromomethyl)-1H-pyrrolo[1,2,3-*de*]quinoxaline-2(3H)-one (the crude product of the previous step) was dissolved in acetonitrile (5 mL), and added with DIPEA (4 M)(90 mg, 0.70 mmol) and 6-fluoro-N-methyl-5-(piperazine-1-yl) picolinamide (83 mg, 0.35 mmol) to react at 25°C for 2 hours, and it was detected by LCMS until the reaction was finished. The reaction solution was concentrated. An organic phase was concentrated and purified by silica gel plate ($SiO_2$, dichloromethane: methanol=20: 1) to obtain the target compound (12 mg, yield 14.6%).

Molecular formula: $C_{22}H_{23}FN_6O_2$, Molecular weight: 422.25, LC-MS (M/e): 423.2(M +H$^+$)
$^1$H-NMR(400MHz, DMSO-$d_6$) $\delta$: 10.79 (s, 1H), 8.41 (s, 1H), 7.85 (m, 1H), 7.58-7.56 (m, 1H), 7.29 (s, 1H), 7.05 (s, 1H), 6.57 (s, 1H), 6.44-6.43 (m, 1H), 5.33 (s, 2H), 3.46 (s, 2H), 3.29-3.15 (m, 4H), 2.77 (s, 3 H), 2.57-2.47 (m, 4H)

**Example 5: Preparation of 6-fluoro-5-(4-((10-fluoro-2-oxo-1,2,3,4-tetrahydro-[1,4]diazepine[3,2,1-hilindole-9-yl) methyl)piperazine-1-yl)-N-methylpi colinamide (Compound 15)**

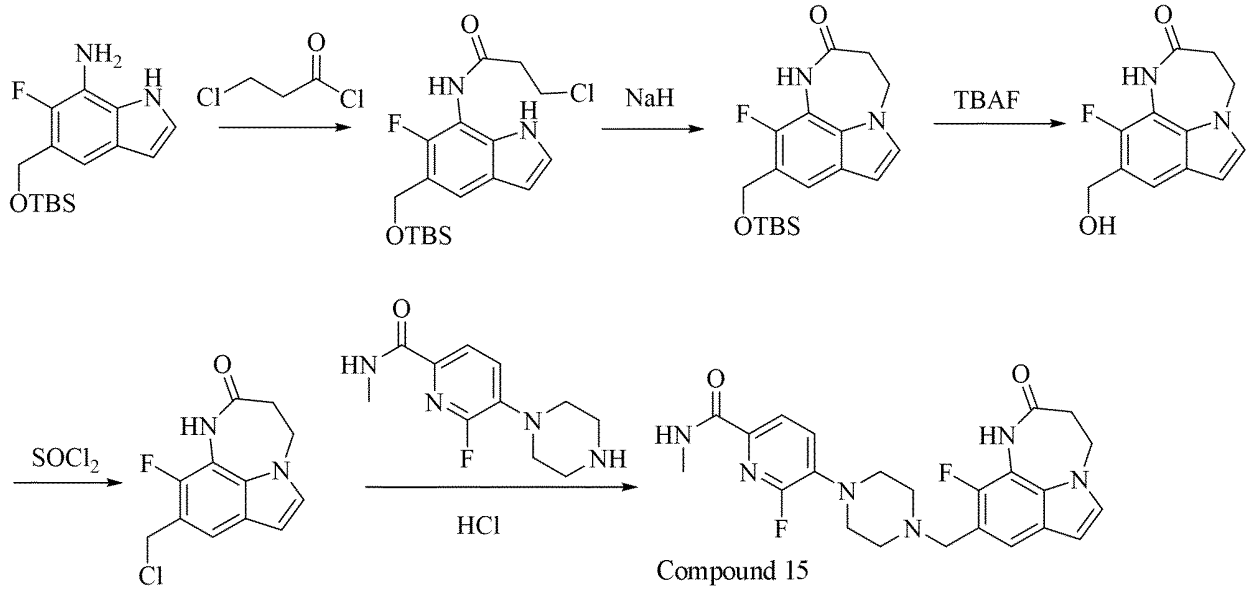

Compound 15

**1. Preparation of N-(5-((tert-butyldimethylsilyl)oxy)methyl)-6-fluoro-1H-indole-7-yl)-3-chloropropionamide**

The 5-((tert-butyldimethylsilyl)oxy)methyl)-6-fluoro-1H-indole-7-amine (350 mg, 1.2 mmol) was dissolved in dichloromethane (20 mL), cooled to 0°C, and added with 3-chloropropionyl chloride (226 mg, 1.8 mmol) to react at 0°C for 30 minutes, and it was detected by LCMS until the reaction was finished. The reaction solution was added with a saturated sodium bicarbonate solution (15 mL), extracted with dichloromethane (30 mL), dried and concentrated to obtain the product (crude product, 400 mg).

**2. Preparation of 9-((tert-butyldimethylsilyl)oxy)methyl)-10-fluoro-3,4-dihydro-[1,4]diazepine[3,2,1-hi]indole-2(1H)-one**

The N-(5-((tert-butyldimethylsilyl)oxy)methyl)-6-fluoro-1H-indole-7-yl)-3-chloropropionamide (400 mg, the crude product of the previous step) was dissolved in DMF (10 mL), cooled to 0°C, and added with sodium hydride (60%)(125 mg, 5.2 mmol) to react at 25°C for 20 minutes. The reaction solution was added with water for quenching, then added with water (30 mL) and ethyl acetate (50 mL), and subjected to liquid separation. An organic phase was washed with water (30 mL), and concentrated to obtain a crude product (400 mg), and the crude product was purified by silica gel column chromatography (n-heptane: ethyl acetate=6: 1) to obtain the product (200 mg, two-step yield 48.3%).

**3. Preparation of 10-fluoro-9-(hydroxymethyl)-3,4-dihydro-[1,4]diazepine[3,2,1-hi]indole-2(1H)-one**

The 9-((tert-butyldimethylsilyl)oxy)methyl)-10-fluoro-3,4-dihydro-[1,4]diazepine[3,2,1-hi]indole-2(1H)-one (200 mg, 0.57 mmol) was dissolved in tetrahydrofuran (20 mL), and added with TBAF (1.4 mL, 1.4 mmol) to react at 25°C for 2 hours, and the reaction was finished. The reaction solution was concentrated, and purified by silica gel column chromatography (n-heptane: ethyl acetate=1: 2) to obtain the product (120 mg, yield 89.3%).

**4. Preparation of 9-(chloromethyl)-10-fluoro-3,4-dihydro-[1,4]diazepine[3,2,1-hi]indole-2(1H)-one**

The 10-fluoro-9-(hydroxymethyl)-3,4-dihydro-[1,4]diazepine[3,2,1-hi]indole-2(1H)-one (60 mg, 0.26 mmol) and N,N-dimethylformamide (6 mg, 0.082 mmol) were dissolved in dichloromethane (7 mL), cooled to -5°C, and added with thionyl chloride (60 mg, 0.50 mmol) to react at -5°C for 20 minutes, and it was detected by LCMS until the reaction was finished. The reaction solution was concentrated, and the crude product was directly used in the next step.

**5. Preparation of 6-fluoro-5-(4-((10-fluoro-2-oxo-1,2,3,4-tetrahydro-[1,4]diazepine[3,2,1-hi]indole-9-yl)methyl) piperazine-1-yl)-N-methylpi colinamide**

The 9-(chloromethyl)-10-fluoro-3,4-dihydro-[1,4]diazepine[3,2,1-hi]indole-2(1H)-one (the crude product of the previous step) and 6-fluoro-N-methyl-5-(piperazine-1-yl)picolinamide hydrochloride (72 mg, 0.26 mmol) were dissolved in acetonitrile (10 mL), and added with DIEA (170 mg, 1.3 mmol) to react at 80°C for 2 hours after the addition, and it was

detected by LCMS until the reaction was finished. The reaction solution was concentrated, and the crude product was purified by silica gel column chromatography (dichloromethane: methanol=30: 1) to obtain a crude product (48 mg). The crude product was purified by Prep-TLC (dichloromethane: methanol=25: 1) to obtain the product (37 mg, two-step yield 31.8%).

Molecular formula: $C_{23}H_{24}F_2N_6O_2$, Molecular weight: 454.5, LC-MS (m/z): 455.2($M+H^+$)
$^1$H-NMR(400MHz, $CDCl_3$) $\delta$: 10.71 (s, 1H), 7.99 (d, *J*=8.0 Hz, 1H), 7.52 (s, 1H), 7.39-7.20 (m, 3H), 6.49 (s, 1H), 4.11-4.08 (m, 2H), 3.73 (s, 2H), 3.25-3.20 (m, 6H), 3.01 (d, *J*=5.2 Hz, 3H), 2.77-2.69 (m, 4H).

**Example 6: Preparation of 5-(4-((3-ethyl-9-fluoro-2-oxo-2,3-dihydro-1H-pyrrolo[1,2,3-de]quinoxaline-8-yl) methyl)piperazine-1-yl)-6-fluoro-N-methy lpicolinamide (Compound 24)**

Referring to the preparation of Example 2, the raw material 2-bromopropionic acid was replaced by 2-bromobutyric acid.

Molecular formula: $C_{24}H_{26}F_2N_6O_2$, Molecular weight: 468.5, LC-MS (M/e):469.2 ($M +H^+$)
$^1$H-NMR(400MHz, DMSO-$d_6$) $\delta$: 8.14 (s, 1H), 7.99-77 (m, 1H), 7.51-7.49 (m, 1H), 7.31-7.28 (m, 1H), 7.18 (s, 1H), 7.11 (s,1H), 6.55 (s, 1H), 5.22-5.20 (m, 1H), 3.75-3.71 (m, 2H), 3.25-3.22 (m, 4H), 3.00 (s, 3H), 2.73-2.71(s, 4H), 2.44-2.39 (m, 1H), 2.16-2.13 (m, 1H), 0.85 (t, J=6.8, 3H).

**Example 7: Preparation of 6-fluoro-N-methyl-5-(4-((3-methyl-2-oxo-2,4,5,6-tetrahydro-1H-benzo[de]quinoline-8-yl)methyl)piperazine-1-yl)picolina mide (Compound 25)**

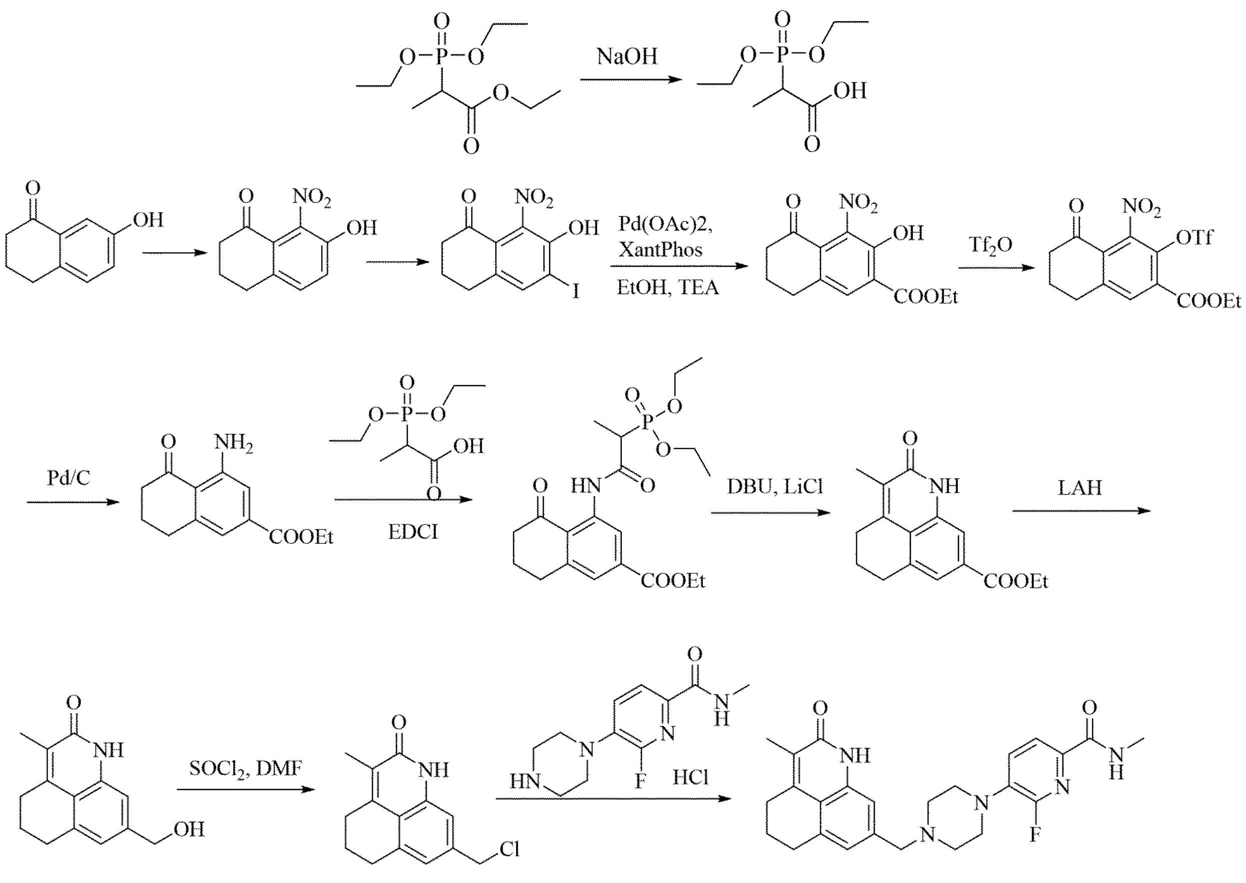


**1. Preparation of 2-(diethoxyphosphoryl)propionic acid**

Ethyl 2-(diethoxyphosphoryl)propionate (5.0 g, 21.0 mmol) was dissolved in ethanol/water (100 mL/20 mL), and added with sodium hydroxide (1.7 g, 42.5 mmol) to react at 25°C for 2 hours, and the reaction was finished. The reaction

solution was concentrated to remove ethanol, added with 2 M hydrochloric acid to adjust a pH value to be 1-2, extracted with ethyl acetate (100 mL), dried and concentrated to obtain the product (4.7 g).

**2. Preparation of 7-hydroxy-8-nitro-3,4-dihydronaphthalene-1(2H)-one**

7-hydroxy-3,4-dihydronaphthalene-1(2H)-one (50 g, 308.3 mmol) was dissolved in concentrated sulfuric acid (400 mL), and the mixture was cooled to -5°C, added with potassium nitrate (32 g, 316.5 mmol) in batches, and maintained at this temperature to react for 2 hours. The reaction solution was poured into ice water, extracted with ethyl acetate, and subjected to liquid separation to obtain an organic phase. The organic phase was dried with anhydrous sodium sulfate and then filtered, and the filtrate was concentrated. The crude product was purified by silica gel column chromatography (n-heptane and ethyl acetate were mobile phases, and a proportion of ethyl acetate was from 0% to 55%) to obtain the product (24.5 g, yield 38.4%).

**3. Preparation of 7-hydroxy-6- iodo-8- nitro-3,4-dihydronaphthalene-1(2H)-one**

The 7-hydroxy-8-nitro-3,4-dihydronaphthalene-1(2H)-one (20 g, 96.5 mmol) was dissolved in acetonitrile (300 mL), and added with N-iodosuccinimide (21.8 g, 96.9 mmol) to react at 80°C for 24 hours, and then the reaction solution was concentrated. The crude product was purified by silica gel column chromatography (n-heptane and ethyl acetate were mobile phases, and a proportion of ethyl acetate was from 0% to 45%) to obtain the product (12.4 g, yield 38.6%).

**4. Preparation of ethyl 3-hydroxy-4-nitro-5-oxo-5,6,7,8-tetrahydronaphthalene-2-carboxylate**

The 7-hydroxy-6- iodo-8- nitro-3,4-dihydronaphthalene-1(2H)-one (6.0 g, 18.0 mmol) was dissolved in ethanol (50 mL)/N,N-dimethylformamide (100 mL), added with palladium acetate (410 mg, 1.8 mmol), XantPhos (1.0 g, 1.7 mmol) and triethylamine (5.5 g, 54.3 mmol), subjected to gas exchange with CO to react at 90°C for 4 hours under CO, and the reaction was finished. The reaction solution was concentrated, and the crude product was purified by silica gel column chromatography (n-heptane: ethyl acetate=2: 1) to obtain the product (1.5 g, yield 29.8%).

**5. Preparation of ethyl 4-nitro-5-oxo-3-((trifluoromethyl)sulfonyl)oxy)-5,6,7,8-tetrahydronaphthalene-2-carboxylate**

The ethyl 3-hydroxy-4-nitro-5-oxo-5,6,7,8-tetrahydronaphthalene-2-carboxylate (500 mg, 1.8 mmol) and N,N-diisopropylethylamine (910 mg, 7.0 mmol) were dissolved in dichloromethane (20 mL), cooled to -10°C, and added with trifluoromethanesulfonic anhydride (760 mg, 2.7 mmol) to react at -10°C for 1 hour, and the reaction was finished. The reaction solution was concentrated, and the crude product was purified by silica gel column chromatography (n-heptane: ethyl acetate=1: 2) to obtain the product (500 mg, yield 67.9%).

**6. Preparation of ethyl 4-amino-5-oxo-5,6,7,8-tetrahydronaphthalene-2-carboxylate**

The ethyl 4-nitro-5-oxo-3-((trifluoromethyl)sulfonyl)oxy)-5,6,7,8-tetrahydronaphthalene-2-carboxylate (500 mg, 1.2 mmol) was dissolved in ethanol (20 mL), added with Pd/C (500 mg), and subjected to gas exchange with hydrogen to react at 25°C for 3 hours under hydrogen, the reaction was finished. The reaction solution was concentrated, and the crude product was purified by silica gel column chromatography (n-heptane: ethyl acetate=3: 1) to obtain the product (60 mg, yield 21.2%).

**7. Preparation of ethyl 4-(2-(diethoxyphosphoryl)propionamide)-5-oxo-5,6,7,8-tetrahydronaphthalene-2-carboxylate**

The ethyl 4-amino-5-oxo-5,6,7,8-tetrahydronaphthalene-2-carboxylate (60 mg, 0.26 mmol) and 2-(diethoxyphosphoryl)propionic acid (216 mg, 1.0 mmol) were dissolved in N,N-dimethylformamide (4 mL), and added with EDCI (210 mg, 1.1 mmol) to react at 25°C for 2 hours, and the reaction was finished. The reaction solution was added with water (10 mL) and ethyl acetate (15 mLx2). An organic phase was washed with water (20 mLx2), dried and concentrated to obtain a crude product (100 mg). The crude product was directly used in the next step.

**8. Preparation of ethyl 3-methyl-2-oxo-2,4,5,6-tetrahydro-1H-benzo[de]quinoline-8-carboxylate**

The ethyl 4-(2-(diethoxyphosphoryl)propionamide)-5-oxo-5,6,7,8-tetrahydronaphthalene-2-carboxylate (100 mg, the crude product) was dissolved in tetrahydrofuran (8 mL), and added with DBU (140 mg, 0.92 mmol). The mixture

was stirred at 25°C for 5 minutes, and added with lithium chloride (20 mg, 0.47 mmol) to react at 25°C for 1 hour, and the reaction was finished. The reaction solution was concentrated, and the crude product was purified by silica gel column chromatography (n-heptane: ethyl acetate=1: 1) to obtain the product (33 mg, two-step yield 47.3%).

**9. Preparation of 8-(hydroxymethyl)-3-methyl-5,6-dihydro-1H-benzo[de]quinoline-2(4H)-one**

The ethyl 3-methyl-2-oxo-2,4,5,6-tetrahydro-1H-benzo[de]quinoline-8-carboxylate (33 mg, 0.12 mmol) was dissolved in tetrahydrofuran (7 mL), cooled to 0°C, and added with lithium aluminium hydride (33 mg, 0.87 mmol) to react at 0°C for 1 hour, and the reaction was finished. The reaction solution was added with water for quenching, and added with anhydrous sodium sulfate, and the mixture was stirred for 10 minutes, filtered, and concentrated. The crude product was purified by preparative TLC (n-heptane: ethyl acetate=1: 2) to obtain the product (15 mg, yield 54.1%).

**10. Preparation of 8-(chloromethyl)-3-methyl-5,6-dihydro-1H-benzo[de]quinoline-2(4H)-one**

The 8-(hydroxymethyl)-3-methyl-5,6-dihydro-1H-benzo[de]quinoline-2(4H)-one (15 mg, 0.065 mmol) and N,N-dimethylformamide (9 mg, 0.12 mmol) were dissolved in dichloromethane (7 mL), and added with thionyl chloride (78 mg, 0.66 mmol) to react at 25°C for 16 hours. The reaction solution was concentrated, and the crude product was directly used in the next step.

**11. Preparation of 6-fluoro-N-methyl-5-(4-((3-methyl-2-oxo-2,4,5,6-tetrahydro-1H-benzo[de]quinoline-8-yl) methyl)piperazine-1-yl)picolina mide**

The 8-(chloromethyl)-3-methyl-5,6-dihydro-1H-benzo[de]quinoline-2(4H)-one (the crude product of the previous step) and 6-fluoro-N-methyl-5-(piperazine-1-yl)picolinamide hydrochloride (27 mg, 0.098 mmol) were dissolved in acetonitrile (7 mL), and added with DIEA (63 mg, 0.49 mmol) and potassium carbonate (33 mg, 0.24 mmol) to react at 85 °C for 10 hours, and it was detected by LCMS until the reaction was finished. The reaction solution was concentrated, and the crude product was purified by preparative TLC (dichloromethane: methanol=16: 1) to obtain a crude product (20 mg). The crude product was purified by preparative TLC (dichloromethane: methanol=16: 1, and separation with ethyl acetate twice) to obtain the product (17 mg, two-step yield 54.4%).

Molecular formula: $C_{25}H_{28}FN_5O_2$, Molecular weight: 449.5, LC-MS (m/z): 450.2(M+H$^+$)
$^1$H-NMR(400MHz, $CDCl_3$) $\delta$: 10.05 (s, 1H), 7.99 (d, *J*=8.0 Hz, 1H), 7.52 (s, 1H), 7.34-7.31 (m, 1H), 7.05 (s, 1H), 6.99 (s, 1H), 3.62 (s, 2H), 3.26 (t, *J*=6.0 Hz, 4H), 3.01 (d, *J*=5.2 Hz, 3H), 2.95 (t, *J*=6.0 Hz, 2H), 2.89 (t, *J*=6.0 Hz, 2H), 2.68 (t, *J*=6.0 Hz, 4H), 2.23 (s, 3H), 2.04 (t, *J*=5.8 Hz, 2H).

**Example 8: Preparation of 6-fluoro-N-methyl-5-(4-((3-methyl-2-oxo-2,3-dihydro-1H-pyrrolo[1,2,3-de]quinoxaline-8-yl)methyl)piperazine-1-yl)pyridi ne carboxamide (Compound 26)**

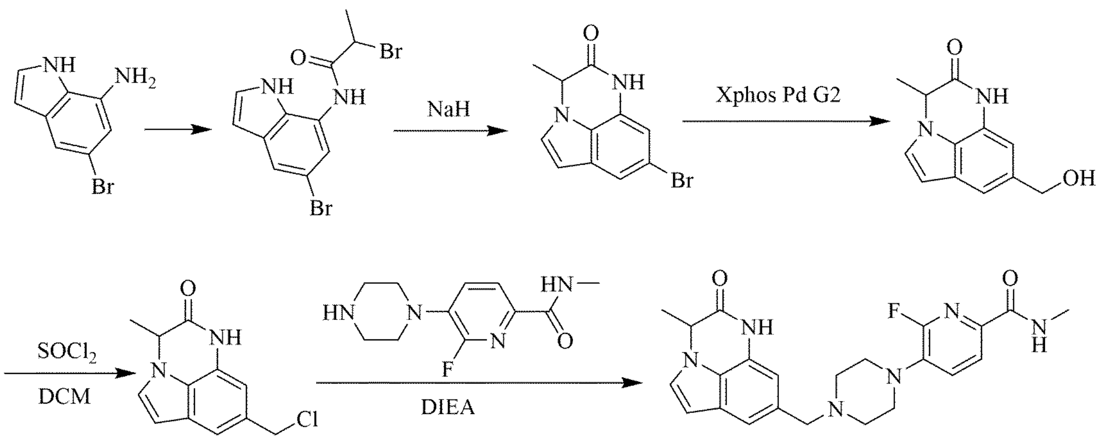


1. **Preparation of 2-bromo-N-(5-bromo-1H-indole-7-yl)propionamide**

5-bromo-1H-indole-7-amine (279 mg, 1.3 mmol) was dissolved in ethyl acetate (10 mL), added with pyridine (411

mg, 5.2 mmol) and 2-bromopropionic acid (367 mg, 2.4 mmol), and added with 1-propylphosphoric anhydride (2.5 g, 50% ethyl acetate solution, 3.9 mmol) at -10°C to react for 0.5 hour, and then the reaction solution was added with water for quenching. The reaction solution was concentrated, and the crude product was purified by silica gel column chromatography (ethyl acetate/petroleum ether=0-40%) to obtain the product (300 mg, yield 65.6%).

**2. Preparation of 8-bromo-3-methyl-1H-pyrrolo[1,2,3-de]quinoxaline-2(3H)-one**

The 2-bromo-N-(5-bromo-1H-indole-7-yl)propionamide (300 mg, 0.9 mmol) was dissolved in DMF(5 mL), and added with NaH (60%)(72 mg, 1.8 mmol) to react at 25°C for 0.5 hour, and the reaction was finished. The reaction solution was extracted with water and ethyl acetate, and an organic phase was collected and concentrated. The crude product was purified by silica gel column chromatography (ethyl acetate/petroleum ether=0-40%) to obtain the product (212 mg, 92.2%).

**3. Preparation of 8-(hydroxymethyl)-3-methyl-1H-pyrrolo[1,2,3-de]quinoxaline-2(3H)-one**

The 8-bromo-3-methyl-1H-pyrrolo[1,2,3-de]quinoxaline-2(3H)-one (186 mg, 0.7 mmol), tri-n-butyltin methanol (257 mg, 0.8 mmol) and Xphos Pd G2 (55 mg, 0.07 mmol) were dissolved in 1,4-dioxane (10 mL) to react at 80°C for 2 hours. An organic phase was dried, concentrated and subjected to column chromatography ($SiO_2$, dichloromethane: methanol=15: 1) to obtain the target compound (150 mg, yield 91.7%).

**4. Preparation of 8-(chloromethyl)-3-methyl-1H-pyrrolo[1,2,3-de]quinoxaline-2(3H)-one**

The 8-(hydroxymethyl)-3-methyl-1H-pyrrolo[1,2,3-de]quinoxaline-2(3H)-one (150 mg, 0.7 mmol) was dissolved in dichloromethane (5 mL), and added with thionyl chloride (333 mg, 2.8 mmol) and DMF (0.1 mL) at 0°C to react at 30 °C for 1 hour, and the reaction was finished. The reaction solution was concentrated, and directly used in the next step.

**5. Preparation of 6-fluoro-N-methyl-5-(4-((3-methyl-2-oxo-2,3-dihydro-1H-pyrrolo[1,2,3-de]quinoxaline-8-yl) methyl)piperazine-1-yl)pyridi ne carboxamide**

6-fluoro-N-methyl-5-(piperazine-1-yl)picolinamide hydrochloride (383 mg, 1.6 mmol) and the 8-(chloromethyl)-3-methyl-1H-pyrrolo[1,2,3-de]quinoxaline-2(3H)-one (the crude product of the previous step) were dissolved in acetonitrile (10 mL), and added with N,N-diisopropylethylamine (724 mg, 5.6 mmol) to react at 80°C for 8 hours, and the reaction was finished. The reaction solution was concentrated, purified by TLC (methanol: dichloromethane=10%), and then subjected to reversed-phase column chromatography (MeOH: $H_2O$=70%) to obtain the product (10 mg, two-step yield 3.3%).

Molecular formula: $C_{23}H_{25}FN_6O_2$, Molecular weight: 436.5, LC-MS (M/e): 437.3 (M +$H^+$)
$^1$H-NMR(400MHz, DMSO-$d_6$) $\delta$: 8.18 (s, 1H), 8.00-7.96 (d,1H), 7.55-7.45 (s, 1H), 7.32-7.26 (m, 1H), 7.23-7.19 (s, 1H), 7.15-7.11 (s,1H), 6.65 (s, 1H), 6.55 (s, 1H), 5.19-5.10 (m, 1H), 3.66-3.63 (m, 2H), 3.30-3.23 (m, 4H), 3.00-2.99 (s, 3H), 2.67 (s, 4H), 1.88 (d, 3H).

**Example 9: Preparation of 6-fluoro-N-methyl-5-(4-((3-methyl-2-oxo-2,3,6,7-tetrahydro-1H,5H-pyrido[1,2,3-de] quinoxaline-9-yl)methyl)piperazine-1 -yl)pyridine carboxamide (Compound 32)**

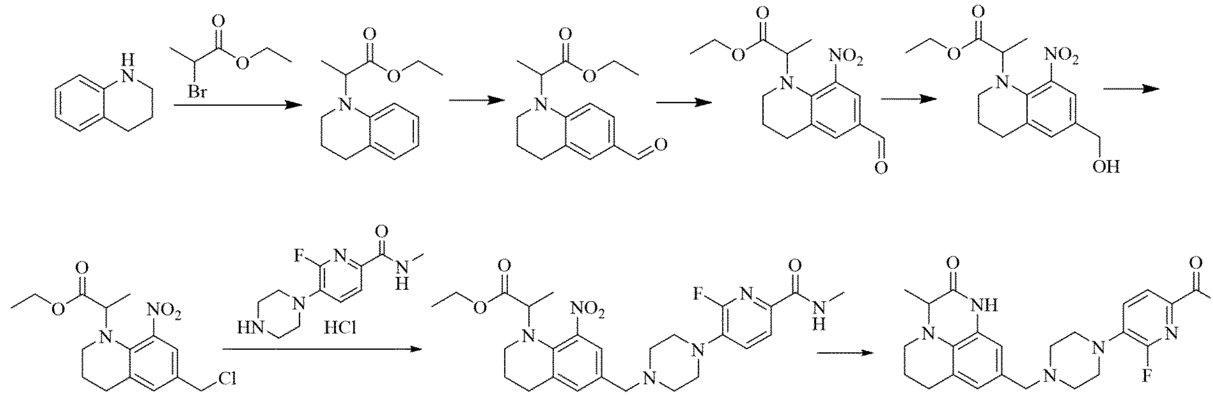

1. **Preparation of ethyl 2-(3,4-dihydroquinoline-1(2H)-yl)propionate**

1,2,3,4-tetrahydroquinoline (2.6 g, 19.5 mmol), ethyl 2-bromopropionate (4.2 g, 23.4 mmol) and DIEA (7.5 g, 58.5 mmol) were dissolved in DMF (50 mL) to react at 100°C for 16 hours after the addition. The reaction solution was poured into water, and extracted with ethyl acetate. An organic phase was concentrated and purified by column chromatography ($SiO_2$, petroleum ether: ethyl acetate=5: 1) to obtain the target compound (2.0 g, yield 44.0%).

**2. Preparation of ethyl 2-(6-formyl-3,4-dihydroquinoline-1(2H)-yl)propionate**

The ethyl 2-(3,4-dihydroquinoline-1(2H)-yl)propionate (2.0 g, 8.6 mmol) was dissolved in dichloromethane (50 mL), and added with DMF (6.3 g, 86.0 mmol) and phosphorus oxychloride (4.0 g, 25.8 mmol) at 0°C to react at 15°C for 2 hours after the addition. The reaction solution was added with water for quenching, added with an aqueous solution of sodium hydroxide to adjust a pH value to be 7, and extracted with ethyl acetate. An organic phase was concentrated and purified by silica gel column ($SiO_2$, petroleum ether: ethyl acetate=3: 1) to obtain the target compound (1.5 g, yield 67.0%).

**3. Preparation of ethyl 2-(6-formyl-8-nitro-3,4-dihydroquinoline-1(2H)-yl)propionate**

The ethyl 2-(6-formyl-3,4-dihydroquinoline-1(2H)-yl)propionate (1.5 g, 5.7 mmol) was dissolved in concentrated sulfuric acid (10 mL), and added with potassium nitrate (688 mg, 6.8 mmol) at 0°C to react at 0°C for 1 hour after the addition. The reaction solution was diluted in water, and extracted with ethyl acetate. An organic phase was concentrated, and directly used in the next step.

**4. Preparation of ethyl 2-(6-(hydroxymethyl)-8-nitro-3,4-dihydroquinoline-1(2H)-yl)propionate**

The ethyl 2-(6-formyl-8-nitro-3,4-dihydroquinoline-1(2H)-yl)propionate (the crude product of the previous step) was dissolved in methanol (30 mL), and added with sodium borohydride (650 mg, 17.1 mmol) to react at 10°C for 2 hours after the addition. The reaction solution was concentrated and purified by column chromatography ($SiO_2$, petroleum ether: ethyl acetate=1: 1) to obtain the target compound (150 mg, yield 8.6%).

**5. Preparation of ethyl 2-(6-(chloromethyl)-8-nitro-3,4-dihydroquinoline-1(2H)-yl)propionate**

The ethyl 2-(6-(hydroxymethyl)-8-nitro-3,4-dihydroquinoline-1(2H)-yl)propionate (150 mg, 0.49 mmol) was dissolved in dichloromethane (12 mL), and added with thionyl chloride (583 mg, 4.9 mmol) at 0°C to react at 0°C for 1 hour after the addition, and it was detected by LCMS until the reaction was finished. The reaction solution was concentrated, and directly used in the next step.

**6. Preparation of ethyl 2-(6-((4-(2-fluoro-6-(methyl carbamoyl)pyridine-3-yl)piperazine-1-yl)methyl)-8-nitro-3,4-dihydroquinoline-1(2H)-yl)propionate**

The ethyl 2-(6-(chloromethyl)-8-nitro-3,4-dihydroquinoline-1(2H)-yl)propionate (the crude product of the previous step) and 6-fluoro-N-methyl-5-(piperazine-1-yl)picolinamide hydrochloride (134 mg, 0.49 mmol) were dissolved in acetonitrile (25 mL), and added with DIEA (194 mg, 1.5 mmol) to react at 80°C for 2 hours after the addition, and it was detected by LCMS until the reaction was finished. An organic phase was concentrated and purified by silica gel plate ($SiO_2$, 100% ethyl acetate) to obtain the target compound (200 mg, yield 77.8%).

**7. Preparation of 6-fluoro-N-methyl-5-(4-((3-methyl-2-oxo-2,3,6,7-tetrahydro-1H,5H-pyrido[1,2,3-de]quinoxaline-9-yl)methyl)piperazine-1 -yl)pyridine carboxamide**

The ethyl 2-(6-((4-(2-fluoro-6-(methyl carbamoyl)pyridine-3-yl)piperazine-1-yl)methyl)-8-nitro-3,4-dihydroquinoline-1(2H)-yl)propionate (200 mg, 0.38 mmol) was dissolved in Methanol (30 mL), and added with palladium on carbon (40 mg) to react at 25°C for 1 hour after the addition, and it was detected by LCMS until the reaction was finished. The reaction solution was filtered with diatomite, the filtrate was concentrated and purified by C18 column (MeCN-0-40%) and then purified by high-pressure liquid phase (MeCN-0-40%) to obtain the target compound (3.8 mg, yield 2.2%).

Molecular formula: $C_{24}H_{29}FN_6O_2$, Molecular weight: 452.5, LC-MS (m/z): 453.1($M+H^+$)
$^1$H-NMR(400 MHz, MeOD) $\delta$: 7.89 (d, J=7.8 Hz, 1H), 7.49-7.53 (m, 1H), 6.72 (s, 1H), 6.66 (s, 1H), 3.81-3.83 (m, 1H), 3.46 (s, 2H), 332-3.43 (m, 1H), 3.23-3.43 (m, 4H), 3.08-3.16 (m, 1H), 2.92 (s, 3H), 2.68-2.82 (m, 2H), 2.59-2.65 (m, 4H), 1.95-2.10 (m, 2H), 1.19-1.26 (m, 3H).

**Example 10: Preparation of 6-fluoro-5-(4-((9-fluoro-3,5-dimethyl-2-oxo-2,3-dihydro-1H-pyrrolo[1,2,3-de]quinoxaline-8-yl)methyl)piperazine-1-yl)-N-methylpyridine formamide (Compound 34)**

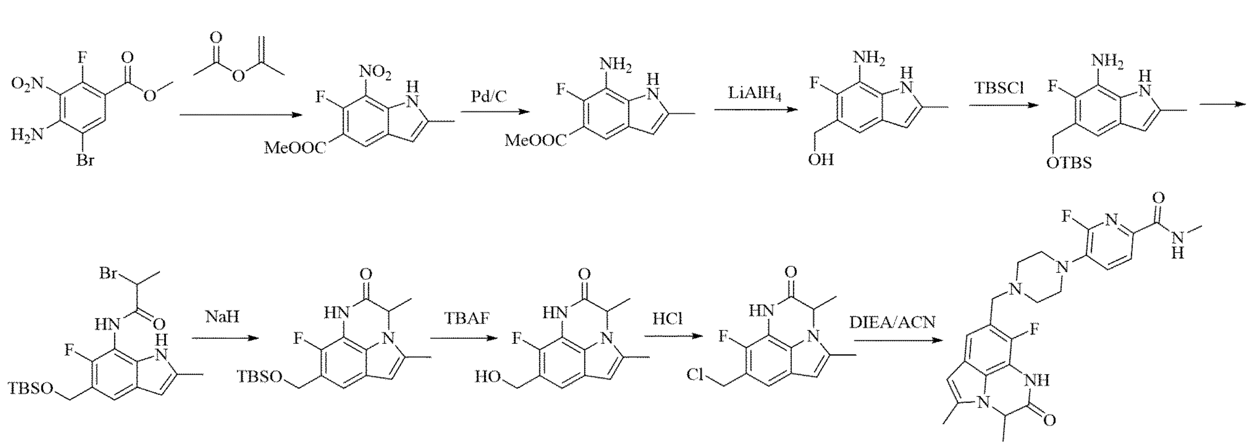


**1. Preparation of methyl 6-fluoro-2-methyl-7-nitro-1H-indole-5-carboxylate**

A toluene (20 mL) solution of methyl 4-amino-5-bromo-2-fluoro-3-nitrobenzoate (3.2 g, 10.9 mmol) was added with tri-n-butyl(methoxy)tin (10.5 g, 32.7 mmol), isopropenyl acetate (5.5 g, 54.9 mmol), palladium acetate (245 mg, 1.1 mmol) and tris(o-tolyl)phosphine (332 mg, 1.1 mmol) to react by microwave at 90°C for 1 hour under the protection of nitrogen. The system was added with a saturated potassium fluoride solution, stirred for 30 minutes, and filtered, and solids were washed with ethyl acetate. The filtrate was extracted with ethyl acetate, and concentrated, and the crude product was purified by silica gel column chromatography (dichloromethane: petroleum ether=1:1) to obtain the product (650 mg, yield 23.6%).

**2. Preparation of methyl 7-amino-6-fluoro-2-methyl-1H-indole-5-carboxylate**

The methyl 6-fluoro-2-methyl-7-nitro-1H-indole-5-carboxylate (800 mg, 3.2 mmol) was dissolved in methanol (50 mL), and added with Pd/C (350 mg, N/A). The mixture was subjected to gas exchange with hydrogen for 3 times to react at 25°C for 3 hours under hydrogen, and the reaction was finished. The reaction solution was filtered to collect solids, so as to obtain a crude product (600 mg).

**3. Preparation of (7-amino-6-fluoro-2-methyl-1H-indole-5-yl)methanol**

The methyl 7-amino-6-fluoro-2-methyl-1H-indole-5-carboxylate (600 mg, 2.7 mmol) was dissolved in tetrahydrofuran (10 mL), and added with lithium aluminum hydride (308 mg, 8.1 mmol) at 0°C to react at 50°C for 1 hour, and the reaction was finished. The reaction solution was quenched with water, added with anhydrous sodium sulfate, stirred and filtered by suction. The filtrate was concentrated, and the crude product was purified by silica gel column chromatography (ethyl acetate: petroleum ether=1: 1) to obtain the product (280 mg, two-step yield 45.5%).

**4. Preparation of 5-((tert-butyldimethylsilyl)oxy)methyl)-6-fluoro-2-methyl-1H-indole-7-amine**

The (7-amino-6-fluoro-2-methyl-1H-indole-5-yl)methanol (260 mg, 1.3 mmol) was dissolved in dichloromethane (20 mL), and added with imidazole (266 mg, 3.9 mmol) and tert-butyldimethylsilyl chloride (294 mg, 2.0 mmol) to react at 25°C for 1 hour, and the reaction was finished. The system was added with water, and extracted with dichloromethane to obtain an organic phase. The organic phase was concentrated, and subjected to column chromatography (ethyl acetate: petroleum ether=1: 3) to obtain the product (370 mg, yield: 89.6%).

**5. Preparation of 2-bromo-N-(5-((tert-butyldimethylsilyl) oxy)methyl)-6-fluoro-2-methyl-1H-indole-7-yl)propionamide**

The 5-((tert-butyldimethylsilyl)oxy)methyl)-6-fluoro-2-methyl-1H-indole-7-amine (330 mg, 1.1 mmol) was dissolved in ethyl acetate (10 mL), added with pyridine (331 mg, 4.2 mmol) and 2-bromopropionic acid (252 mg, 1.6 mmol), and added with 1-propylphosphoric anhydride (w50%, 2.0 g, 3.1 mmol) at -50°C to react for 2 hours. The system was added with for quenching, and extracted with ethyl acetate to obtain an organic phase. The organic phase was concentrated, and the crude product was purified by silica gel column chromatography (ethyl acetate: petroleum ether=1: 6) to obtain the product (465 mg, yield 98.3%).

**6. Preparation of 8-((tert-butyldimethylsilyl)oxy)methyl)-9-fluoro-3,5-dimethyl-1H-pyrrolo[1,2,3-de]quinoxaline-2(3H)-one**

The 2-bromo-N-(5-((tert-butyldimethylsilyl) oxy)methyl)-6-fluoro-2-methyl-1H-indole-7-yl)propionamide (440 mg, 1.0 mmol) was dissolved in N,N-dimethylformamide (10 mL), and added with NaH (60%)(80 mg, 2.0 mmol) to react at 25 °C for 0.5 hour, and the reaction was finished. The reaction solution was extracted with water and ethyl acetate. An organic phase was collected and concentrated, and the crude product was directly used in the next step of reaction.

**7. Preparation of 9-fluoro-8-(hydroxymethyl)-3,5-dimethyl-1H-pyrrolo[1,2,3-de]quinoxaline-2(3H)-one**

The 8-((tert-butyldimethylsilyl)oxy)methyl)-9-fluoro-3,5-dimethyl-1H-pyrrolo[1,2,3-de]quinoxaline-2(3H)-one (the crude product of the previous step) was dissolved in tetrahydrofuran (6 mL), and added with a tetrahydrofuran solution of tetrabutylammonium fluoride (1M, 5.5 mL) to react at 25°C for 3 hours, and the reaction was finished. The system was added with water, and extracted with ethyl acetate to obtain an organic phase. The organic phase was concentrated, and the crude product was purified by column chromatography (EA: PE=100%) to obtain the product (240 mg, two-step yield 97.1%).

**8. Preparation of 8-(chloromethyl)-9-fluoro-3,5-dimethyl-1H-pyrrolo[1,2,3-de]quinoxaline-2(3H)-one hydrochloride**

The 9-fluoro-8-(hydroxymethyl)-3,5-dimethyl-1H-pyrrolo[1,2,3-de]quinoxaline-2(3H)-one (50 mg, 0.20 mmol) was dissolved in acetonitrile (3 mL), and added with an ethyl acetate solution of hydrogen chloride (3.0 mL) to react at 25°C for 1 hour, and the reaction was finished. The reaction solution was concentrated at, and directly used in the next step.

**9. Preparation of 6-fluoro-5-(4-((9-fluoro-3,5-dimethyl-2-oxo-2,3-dihydro-1H-pyrrolo[1,2,3-de]quinoxaline-8-yl) methyl)piperazine-1-yl)-N-methylpyridine formamide**

6-fluoro-N-methyl-5-(piperazine-1-yl)picolinamide hydrochloride (55 mg, 0.20 mmol) and the 8-(chloromethyl)-9-fluoro-3,5-dimethyl-1H-pyrrolo[1,2,3-de]quinoxaline-2(3H)-one hydrochloride (the crude product of the previous step) were dissolved in acetonitrile (10 mL), and added with N,N-diisopropylethylamine (155 mg, 1.2 mmol) to react at 80°C for 2 hours, and the reaction was finished. The system was added with water, and extracted with ethyl acetate to obtain an organic phase. The organic phase was concentrated, and the crude product was purified by column chromatography (dichloromethane: methanol=20: 1) to obtain the product (30 mg, two-step yield 31.8%).

Molecular formula: $C_{24}H_{26}F_2N_6O_2$, Molecular weight: 468.5, LC-MS (M/e): 469.2 (M +$H^+$)
$^1$H-NMR (400MHz, $CDCl_3$) $\delta$: 7.99-7.96 (m, 1H), 7.82 (s, 1H), 7.50-7.48 (m, 1H), 7.31-7.28 (m, 1H), 7.10-7.05 (m, 1H), 6.23 (s, 1H), 5.14-5.09 (m, 1H), 3.76-3.70 (m, 2H), 3.24-3.20 (m, 4H), 3.02-2.99 (m, 3H), 2.71-2.69 (m, 4H), 2.43 (s,3H), 1.69-1.67 (m, 3H).

**Example 11: Preparation of (R)-6-fluoro-5-(4-((9-fluoro-3-methyl-2-oxo-2,3-dihydro-1H-pyrrolo[1,2,3-de]quinoxaline-8-yl)methyl)piperazine-1-yl)-N-methylpyridine formamide (Compound 36)**

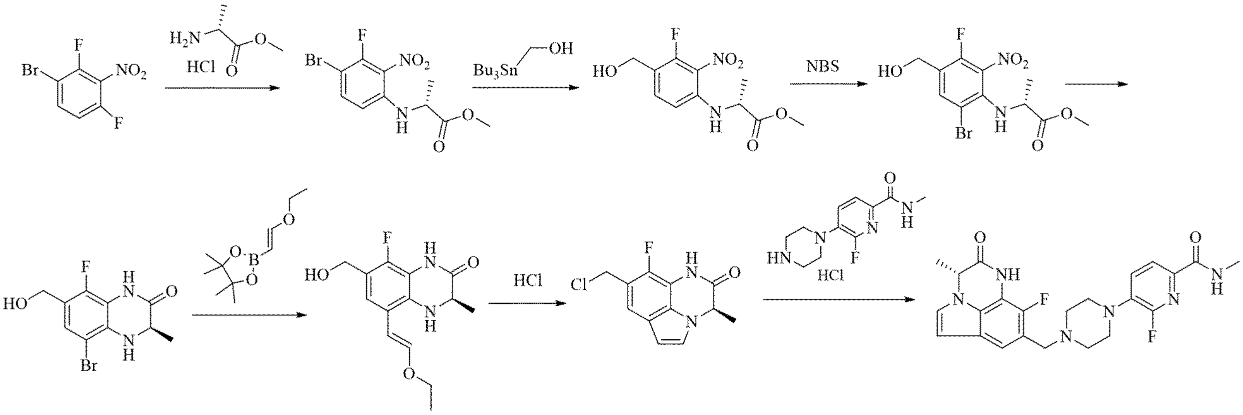


**1. Preparation of methyl (4-bromo-3-fluoro-2-nitrophenyl)-D-alaninate**

Bromo-2,4-difluoro-3-nitrobenzene (2.5 g, 10.5 mmol) was dissolved in N,N-dimethylformamide (50 mL), and added with (D)-alanine methyl ester hydrochloride (1.6 g, 11.5 mmol) and N,N-diisopropylethylamine (3.6 g, 27.9 mmol) to react at 50°C for 5 hours. The reaction solution was concentrated, and the crude product was purified by silica gel column chromatography (n-heptane: ethyl acetate=1: 1) to obtain the product (2.0 g, yield 59.3%).

**2. Preparation of methyl (3-fluoro-4-(hydroxymethyl)-2-nitrophenyl)-D-alaninate**

The methyl (4-bromo-3-fluoro-2-nitrophenyl)-D-alaninate (1.6 g, 5.0 mmol), tri-n-butyltin methanol (1.8 g, 5.6 mmol) and Xphos Pd G2 (390 mg, 0.50 mmol) were dissolved in 1,4-dioxane (70 mL), and subjected to gas exchange with nitrogen to react at 80°C for 5 hours under nitrogen. The reaction solution was concentrated, and the crude product was purified by silica gel column chromatography (n-heptane: ethyl acetate=2: 1) to obtain the product (1.1 g, yield 81.1%).

**3. Preparation of methyl (6-bromo-3-fluoro-4-(hydroxymethyl)-2-nitrophenyl)-D-alaninate**

The methyl (3-fluoro-4-(hydroxymethyl)-2-nitrophenyl)-D-alaninate (1.1 g, 4.0 mmol) was dissolved in N,N-dimethylformamide (30 mL), and added with N-bromosuccinimide (1.1 g, 6.2 mmol) to react at 25 °C for 5 hours. The reaction solution was extracted with water (100 mL) and ethyl acetate (100 mLx2). An organic phase was washed with water (100 mLx2), dried and concentrated, and the crude product was purified by silica gel column chromatography (n-heptane: ethyl acetate=2: 1) to obtain the product (790 mg, yield 55.7%).

**4. Preparation of (R)-5-bromo-8-fluoro-7-(hydroxymethyl)-3-methyl-3,4-dihydroquinoxaline-2(1H)-one**

The methyl (6-bromo-3-fluoro-4-(hydroxymethyl)-2-nitrophenyl)-D-alaninate (400 mg, 1.1 mmol) was dissolved in methanol (10 mL)/tetrahydrofuran (20 mL)/water (4 mL), and added with iron powder (370 mg, 6.6 mmol) and ammonium chloride (590 mg, 11.0 mmol) to react at 70°C for 2 hours. The reaction solution was filtered to remove solids, concentrated, and extracted with water (30 mL) and ethyl acetate (30 mL). The crude product was purified by silica gel column chromatography (n-heptane: ethyl acetate=1: 1) to obtain the product (300 mg, yield 91.1%).

**5. Preparation of (R,E)-5-(2-ethoxyvinyl)-8-fluoro-7-(hydroxymethyl)-3-methyl-3,4-dihydroquinoxaline-2(1H)-one**

The (R)-5-bromo-8-fluoro-7-(hydroxymethyl)-3-methyl-3,4-dihydroquinoxaline-2(1H)-one (300 mg, 1.0 mmol) was dissolved in 1,4-dioxane (10 mL)/water (2 mL), and added with 2-(2-ethoxyvinyl)-4,4,5,5-tetramethyl-1,3,2-dioxaborolane (300 mg, 1.5 mmol), Pd(dppf)$Cl_2$ (75 mg, 0.10 mmol) and sodium carbonate (320 mg, 3.0 mmol). The mixture was subjected to gas exchange with nitrogen to react at 90°C for 5 hours under nitrogen. The reaction solution was concentrated, and extracted with water (30 mL) and ethyl acetate (30 mL). The crude product was purified by silica

gel column chromatography (n-heptane: ethyl acetate=1: 1) to obtain the product (260 mg, yield 89.4%).

**6. Preparation of (R)-8-(chloromethyl)-9-fluoro-3-methyl-1H-pyrrolo[1,2,3-de]quinoxaline-2(3H)-one**

The (R,E)-5-(2-ethoxyvinyl)-8-fluoro-7-(hydroxymethyl)-3-methyl-3,4-dihydroquinoxaline-2(1H)-one (20 mg, 0.071 mmol) was dissolved in acetonitrile (3 mL), and added with an ethyl acetate solution of hydrogen chloride (4 M) (0.3 mL) to react at 25°C for 1 hour. The reaction solution was concentrated, and the crude product was directly used in the next step.

**7. Preparation of (R)-6-fluoro-5-(4-((9-fluoro-3-methyl-2-oxo-2,3-dihydro-1H-pyrrolo[1,2,3-de]quinoxaline-8-yl) methyl)piperazine-1-yl)-N-methylpyridine formamide**

The (R)-8-(chloromethyl)-9-fluoro-3-methyl-1H-pyrrolo[1,2,3-de]quinoxaline-2(3H)-one (the crude product of the previous step) and 6-fluoro-N-methyl-5-(piperazine-1-yl)picolinamide hydrochloride (20 mg, 0.073 mmol) were dissolved in acetonitrile (7 mL), and added with DIEA (46 mg, 0.36 mmol) to react at 70°C for 5 hours. The reaction solution was concentrated, and extracted with water (20 mL) and ethyl acetate (20 mL). The crude product was purified by preparative TLC (dichloromethane: methanol=16: 1) to obtain the product (20 mg, two-step yield 61.7%).

Molecular formula: $C_{23}H_{24}F_2N_6O_2$, Molecular weight: 454.5, LC-MS (m/z):455.0 ($M+H^+$)
$^1$H-NMR (400 MHz, $CDCl_3$) δ: 7.99 (d, *J*=7.0 Hz, 1H), 7.78 (s, 1H), 7.54-7.41 (m, 1H), 7.32-7.25 (m, 1H), 7.18 (d, *J*=5.6 Hz, 1H), 7.14 (d, *J*=2.8 Hz, 1H), 6.54 (d, *J*=2.8 Hz, 1H), 5.15 (t, *J*=7.0 Hz, 1H), 3.73 (s, 2H), 3.26-3.22 (m, 4H), 3.05-3.00 (m, 3H), 2.76-2.65 (m, 4H), 1.85 (d, *J*=7.0 Hz, 3H).

**Example 12: Preparation of (S)-6-fluoro-5-(4-((9-fluoro-3-methyl-2-oxo-2,3-dihydro-1H-pyrrolo[1,2,3-de]quinoxaline-8-yl)methyl)piperazine-1-yl)-N-methylpyridine formamide (Compound 35)**

Referring to the preparation by the method of Example 11, the raw material (D)-alanine methyl ester hydrochloride was replaced by (L)-alanine methyl ester hydrochloride.

Molecular formula: $C_{23}H_{24}F_2N_6O_2$, Molecular weight: 454.5, LC-MS (m/z):454.9 ($M+H^+$)
$^1$H-NMR (400 MHz, $CDCl_3$) δ: 7.99 (d, J=7.8Hz, 1H), 7.78 (s, 1H), 7.55-7.42 (m,1H), 7.32-7.25 (m, 1H), 7.18 (d, J=5.6Hz, 1H), 7.14 (d, J=3.0 Hz, 1H), 6.54 (d, J=3.0 Hz, 1H), 5.15 (q, J=6.9 Hz, 1H), 3.73 (s, 2H), 3.28-3.22 (m, 4H), 3.05-3.00 (m, 3H), 2.76-2.65 (m, 4H), 1.85 (d, J=7.0 Hz, 3H).

**Example 13: Preparation of 6-fluoro-5-(4-((6-fluoro-3-methyl-2-oxo-2,3-dihydro-1H-pyrrolo[1,2,3-de]quinoxaline-8-yl)methyl)piperazine-1-yl)-N-met hylpyridine formamide (Compound 41)**

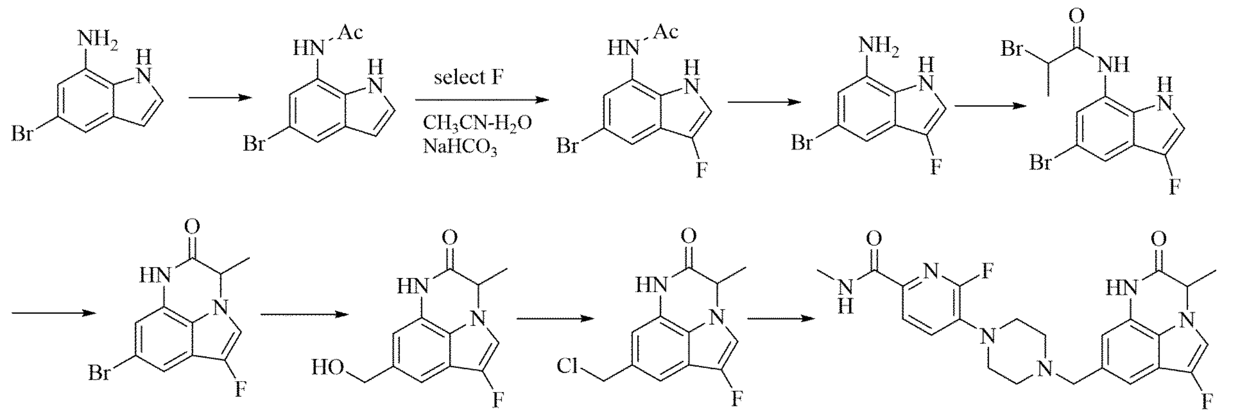


**1. Preparation of N-(5-bromo-1H-indole-7-yl)acetamide**

5-bromo-1H-indole-7-amine (1.4 g, 6.6 mmol) was dissolved in DCM (50 mL), and the reaction mixture was added with triethylamine (2.0 g, 19.8 mmol), and dropwise added with acetyl chloride (0.67 g, 8.5 mmol) to react at 15°C for

1 hour. The reaction solution was concentrated, and the crude product was purified by silica gel column chromatography (ethyl acetate and petroleum ether were mobile phases, and a proportion of ethyl acetate was from 0% to 50%) to obtain the product (1.1 g, yield 65.5%).

**2. Preparation of N-(5-bromo-3-fluoro-1H-indole-7-yl)acetamide**

The N-(5-bromo-1H-indole-7-yl)acetamide (900 mg, 3.6 mmol) was dissolved in acetonitrile (15 mL) and water (3 mL), and added with 1-chloromethyl-4-fluoro-1,4-diazabicyclo[2.2.2] octane bis(tetrafluoroborate) (1.9 g, 5.4 mmol) to react at 10°C for 2 hours. The reaction solution was added with water for quenching. The reaction solution was concentrated, and purified by silica gel column chromatography (ethyl acetate: petroleum ether=1: 2) to obtain a crude product (1.0 g).

**3. Preparation of 5-bromo-3-fluoro-1H-indole-7-amine**

The N-(5-bromo-3-fluoro-1H-indole-7-yl)acetamide (1.0 g, 3.7 mmol) was dissolved in methanol (50 mL), and added with thionyl chloride (2.5 g, 21.0 mmol) to react at 60°C for 1 hour, and then the reaction solution was concentrated. The crude product was purified by silica gel column chromatography (ethyl acetate: petroleum ether=1: 1) to obtain the product (700 mg, yield 82.8%).

**4. Preparation of 2-bromo-N-(5-bromo-3-fluoro-1H-indole-7-yl)propionamide**

The 5-bromo-3-fluoro-1H-indole-7-amine (650 mg, 2.8 mmol) was dissolved in ethyl acetate (20 mL), added with pyridine (460 mg, 5.8 mmol) and 2-bromopropionic acid (800 mg, 5.2 mmol), and dropwise added with 1-cyclopropyl phosphoric anhydride (50%, 3.6 g, 5.7 mmol) at -50°C to react for 2 hours, and then the reaction solution was added with water for quenching. The reaction solution was concentrated, and the crude product was purified by silica gel column chromatography (ethyl acetate: petroleum ether=1: 1) to obtain the product (700 mg, yield 67.8%).

**5. Preparation of 8-bromo-6-fluoro-3-methyl-1H-pyrrolo[1,2,3-de]quinoxaline-2(3H)-one**

The 2-bromo-N-(5-bromo-3-fluoro-1H-indole-7-yl)propionamide (700 mg, 1.9 mmol) was dissolved in DMF(10 mL), and added with NaH (60%)(160 mg, 4.0 mmol) to react at 25°C for 1.5 hours, and the reaction was finished. The reaction solution was extracted with water and ethyl acetate, and an organic phase was collected, concentrated, and purified by thin-layer preparative plate (ethyl acetate: petroleum ether=2: 3) to obtain the product (500 mg, yield is 91.8%).

**6. Preparation of 6-fluoro-8-(hydroxymethyl)-3-methyl-1H-pyrrolo[1,2,3-de]quinoxaline-2(3H)-one**

The 8-bromo-6-fluoro-3-methyl-1H-pyrrolo[1,2,3-*de*]quinoxaline-2(3H)-one (300 mg, 1.1 mmol) was dissolved in 1,4-dioxane (10 mL), and added with (tributyltin)methanol (710 mg, 2.2 mmol) and chlorine(2-dicyclohexylphosphino-2’,4’,6’-triisopropyl-1,1’-biphenyl)[2-(2’-amino-1,1’-biphenyl)]palladium(II) (100 mg, 0.13 mmol) to react at 80 °C for 1 hour, and the reaction was finished. The reaction solution was concentrated, and purified by column chromatography (ethyl acetate: petroleum ether=3:2) to obtain the product (200 mg, yield is 80.6%).

**7. Preparation of 8-(chloromethyl)-6-fluoro-3-methyl-1H-pyrrolo[1,2,3-*de*]quinoxaline-2(3H)-one**

The 6-fluoro-8-(hydroxymethyl)-3-methyl-1H-pyrrolo[1,2,3-*de*]quinoxaline-2(3H)-one (50 mg, 0.21 mmol) was dissolved in dichloromethane (10 mL) and acetonitrile (10 mL), and added with thionyl chloride (150 mg, 1.3 mmol) and DMF (0.1 mL) at 0°C to react at 30°C for 5 hours, and the reaction was finished. The reaction solution was concentrated, and directly used in the next step.

**8. Preparation of 6-fluoro-5-(4-((6-fluoro-3-methyl-2-oxo-2,3-dihydro-1H-pyrrolo[1,2,3-*de*]quinoxaline-8-yl) methyl)piperazine-1-yl)-N-met hylpyridine formamide**

6-fluoro-N-methyl-5-(piperazine-1-yl)picolinamide hydrochloride (60 mg, 0.22 mmol) and the crude product of the previous step were dissolved in acetonitrile (10 mL), and added with N,N-diisopropylethylamine (55 mg, 0.43 mmol) to react at 70°C for 2 hours, and the reaction was finished. The reaction solution was concentrated, and purified by TLC (methanol/dichloromethane=1: 10) to obtain the product (13 mg, two-step yield: 13.4%).

Molecular formula: $C_{23}H_{24}F_2N_6O_2$, Molecular weight: 454.5, LC-MS (M/e):455.1(M $+H^+$)

$^1$H-NMR(400 MHz, $CDCl_3$) 8.11 (s, 1H), 8.00 (d, J=7.84 Hz, 1H), 7.51 (d, J=4.68 Hz, 1H), 7.33-7.30 (m, 1H), 7.28-7.18 (m, 1H), 6.95 (s, 1H), 6.67 (s ,1H), 5.05-5.03 (m, 1H), 3.63-3.50 (m, 2H), 3.23 (m, 4H), 3.01-2.98 (m, 3H), 2.66 (m, 4H), 1.81-1.79 (m, 3H).

9. Chiral column resolution was performed to obtain a stereoisomer compound 41-1 and a compound 41-2, which were characterized as follows:

1) Compound 41-1:

HPLC, retention time: 14.2 min:
$^1$H-NMR(400 MHz, $CDCl_3$) 8.11 (s, 1H), 8.00 (d, J=7.84 Hz, 1H), 7.51 (d, J=4.68 Hz, 1H), 7.33-7.30 (m, 1H), 7.28-7.18 (m, 1H), 6.95 (s, 1H), 6.67 (s ,1H), 5.05-5.03 (m, 1H), 3.63-3.50 (m, 2H), 3.23 (m, 4H), 3.01-2.98 (m, 3H), 2.66 (m, 4H), 1.81-1.79 (m, 3H).

2) Compound 41-2:

HPLC, retention time: 16.2 min:
$^1$H-NMR(400 MHz, $CDCl_3$) 8.11 (s, 1H), 8.00 (d, J=7.84 Hz, 1H), 7.51 (d, J=4.68 Hz, 1H), 7.33-7.30 (m, 1H), 7.28-7.18 (m, 1H), 6.95 (s, 1H), 6.67 (s ,1H), 5.05-5.03 (m, 1H), 3.63-3.50 (m, 2H), 3.23 (m, 4H), 3.01-2.98 (m, 3H), 2.66 (m, 4H), 1.81-1.79 (m, 3H).

**Example 14: Preparation of (R)-6-fluoro-5-(4-((9-fluoro-3,5-dimethyl-2-oxo-2,3-dihydro-1H-pyrrolo[1,2,3-de]quinoxaline-8-yl)methyl)piperazine-1-yl) -N-methylpyridine formamide (Compound 44)**

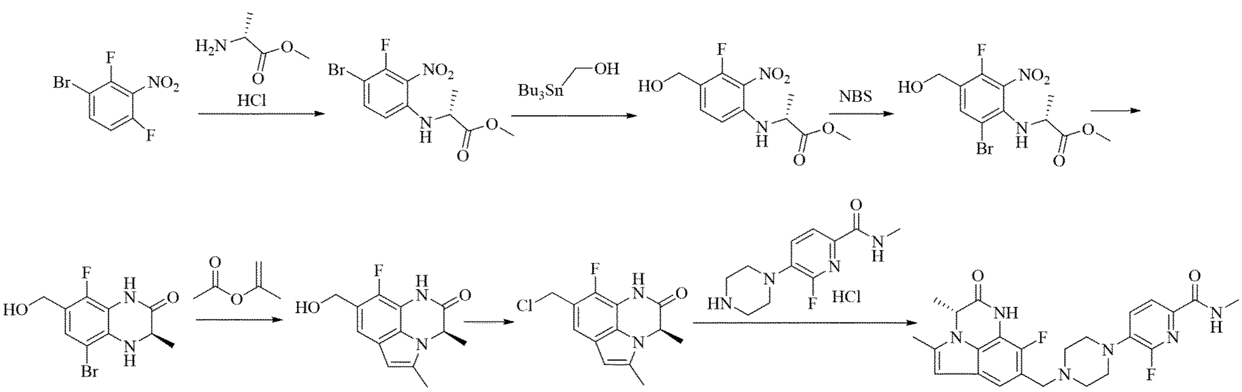


**1. Preparation of methyl (4-bromo-3-fluoro-2-nitrophenyl)-D-alaninate**

Bromo-2,4-difluoro-3-nitrobenzene (10 g, 42.0 mmol) was dissolved in N,N-dimethylformamide (100 mL), and added with D-alanine methyl ester hydrochloride (5.9 g, 42.0 mmol) and N,N-diisopropylethylamine (16.8 g, 0.13 mol) to react at 50°C for 5 hours. The reaction solution was concentrated, and the crude product was purified by silica gel column chromatography (n-heptane: ethyl acetate=1: 1) to obtain the product (7.5 g, yield 56.0%).

**2. Preparation of methyl (3-fluoro-4-(hydroxymethyl)-2-nitrophenyl)-D-alaninate**

The methyl (4-bromo-3-fluoro-2-nitrophenyl)-D-alaninate (7.5 g, 23.4 mmol), (tri-n-butyltin)methanol (8.2 g, 25.7 mmol) and Xphos Pd G2 (1.8 g, 2.3 mmol) were dissolved in 1,4-dioxane (150 mL), and subjected to gas exchange with nitrogen to react at 80°C for 3 hours under nitrogen. The reaction solution was concentrated, and the crude product was purified by silica gel column chromatography (n-heptane: ethyl acetate=1: 1) to obtain the product (4.6 g, yield 72.4%).

**3. Preparation of methyl (6-bromo-3-fluoro-4-(hydroxymethyl)-2-nitrophenyl)-D-alaninate**

The methyl (3-fluoro-4-(hydroxymethyl)-2-nitrophenyl)-D-alaninate (4.6 g, 16.9 mmol) was dissolved in N,N-dimethylformamide (100 mL), and added with N-bromosuccinimide (3.3 g, 18.6 mmol) to react at 25 °C for 16 hours. The

reaction solution was extracted with water (100 mL) and ethyl acetate (100 mLx2). An organic phase was washed with water (100 mLx2), dried and concentrated, and the crude product was purified by silica gel column chromatography (n-heptane: ethyl acetate=3: 1) to obtain the product (3.5 g, yield 59.3%).

**4. Preparation of (R)-5-bromo-8-fluoro-7-(hydroxymethyl)-3-methyl-3,4-dihydroquinoxaline-2(1H)-one**

The methyl (6-bromo-3-fluoro-4-(hydroxymethyl)-2-nitrophenyl)-D-alaninate (390 mg, 1.1 mmol) was dissolved in methanol (15 mL)/tetrahydrofuran (15 mL)/water (5 mL), and added with iron powder (622 mg, 11.1 mmol) and ammonium chloride (1.2 g, 22.2 mmol) to react at 70°C for 1 hour. The reaction solution was filtered to remove solids, concentrated, and extracted with water (30 mL) and ethyl acetate (30 mL), and an organic phase was dried and concentrated to obtain the product (300 mg, yield 93.2%).

**5. Preparation of (R)-9-fluoro-8-(hydroxymethyl)-3,5-dimethyl-1H-pyrrolo[1,2,3-*de*]quinoxaline-2(3H)-one**

The (R)-5-bromo-8-fluoro-7-(hydroxymethyl)-3-methyl-3,4-dihydroquinoxaline-2(1H)-one (300 mg, 1.0 mmol) was dissolved in toluene (11 mL), and added with tributyl(methoxy)tin (995 mg, 3.1 mmol), isopropyl acetate (520 mg, 5.2 mmol), $Pd(OAc)_2$ (23 mg, 0.1 mmol) and tri-o-tolylphosphine (32 mg, 0.1 mmol). The mixture was subjected to gas exchange with nitrogen to react by microwave at 100°C for 1 hour under nitrogen. The reaction solution was concentrated, and purified by silica gel column chromatography (n-heptane: ethyl acetate=1: 1) to obtain the product (100 mg, yield 38.9%).

**6. Preparation of (R)-8-(chloromethyl)-9-fluoro-3,5-dimethyl-1H-pyrrolo[1,2,3-*de*]quinoxaline-2(3H)-one**

The (R)-9-fluoro-8-(hydroxymethyl)-3,5-dimethyl-1H-pyrrolo[1,2,3-*de*]quinoxaline-2(3H)-one (100 mg, 0.4 mmol) was dissolved in dichloromethane (12 mL), and added with an ethyl acetate solution of hydrogen chloride (1.2 mL) to react at 5 °C for 1 hour. The reaction solution was concentrated, and the crude product was directly used in the next step.

**7. Preparation of (R)-6-fluoro-5-(4-((9-fluoro-3,5-dimethyl-2-oxo0-2,3-dihydro-1H-pyrrolo[1,2,3-de]quinoxaline-8-yl)methyl)piperazine-1-yl) -N-methylpyridine formamide**

The (R)-8-(chloromethyl)-9-fluoro-3,5-dimethyl-1H-pyrrolo[1,2,3-de]quinoxaline-2(3H)-one (the crude product of the previous step) and 6-fluoro-N-methyl-5-(piperazine-1-yl)picolinamide hydrochloride (110 mg, 0.4 mmol) were dissolved in acetonitrile (15 mL), and added with DIEA (157 mg, 1.2 mmol) to react at 80°C for 1 hour. The reaction solution was concentrated, and purified by silica gel column chromatography (dichloromethane: methanol=15: 1) to obtain a crude product. The crude product was purified by Prep-TLC (dichloromethane: methanol=15: 1) to obtain the target compound (25 mg, two-step yield 13.1%).

Molecular formula: $C_{24}H_{26}F_2N_6O_2$, Molecular weight: 468.5, LC-MS (m/z): 469.2($M+H^+$)
$^1$H-NMR(400MHz, $CDCl_3$) 7.86-8.3 (m, 1H), 7.90 (s, 1H), 7.45-7.53 (m, 1H), 7.25-7.32 (m, 1H), 7.03-7.12 (m, 1H), 6.23 (s, 1H), 5.12-5.26 (m, 1H), 3.65-3.75 (m, 2H), 3.20-3.30 (m, 4H), 3.00-3.05 (m, 3H), 2.65-2.75 (m, 4H), 2.45 (s, 3H),1.62-1.74 (m, 3H).

**Example 15: Preparation of (R)-6-fluoro-N-methyl-5-(4-((3-methyl-2-oxo-2,3-dihydro-1H-pyrrolo[1,2,3-de]quinoxaline-8-yl)methyl)piperazine-1-yl)p yridine carboxamide (Compound 27)**

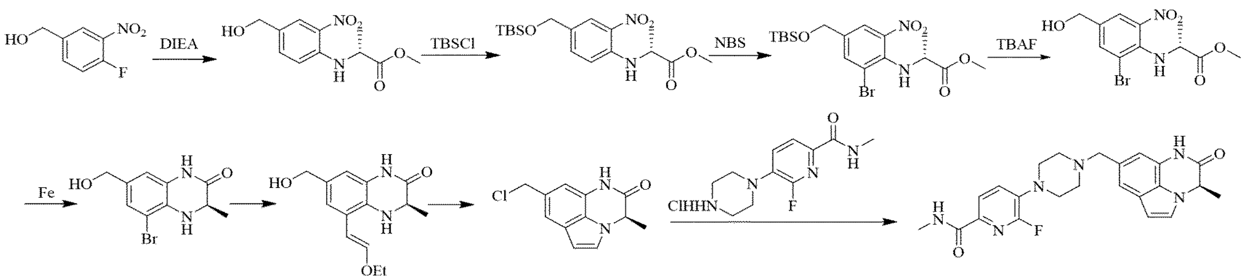

**1. Preparation of methyl (4-(hydroxymethyl)-2-nitrophenyl)-D-alaninate**

(4-fluoro-3-nitrophenyl)methanol (25.0 g, 146.1 mmol) was dissolved in tetrahydrofuran (500 mL), and added with (D)-alanine methyl ester hydrochloride (30.6 g, 219.2 mmol) and N,N-diisopropylethylamine (46.6 g, 438.3 mmol) to react at 80°C for 16 hours, and it was detected by LCMS until the reaction was finished. The reaction solution was concentrated, and the crude product was purified by silica gel column chromatography (n-heptane: ethyl acetate=2: 1) to obtain the product (20.0 g, yield 53.9%).

**2. Preparation of methyl (4-(((tert-butyldimethylsilyl)oxy)methyl)-2-nitrophenyl)-D-alaninate**

The methyl (4-(hydroxymethyl)-2-nitrophenyl)-D-alaninate (12.0 g, 47.2 mmol) and TBSCl (10.7 g, 70.8 mmol) were dissolved in DCM (200 mL), and added with imidazole (6.4 g, 94.4 mmol) to react at 10°C for 0.5 hour. The reaction solution was poured into water, and extracted with ethyl acetate. An organic phase was concentrated, and the crude product was purified by silica gel column chromatography (n-heptane: ethyl acetate=1: 1) to obtain the product (13.6 g, yield 78.2%).

**3. Preparation of methyl (2-bromo-4-((tert-butyldimethylsilyl)oxy)methyl)-6-nitrophenyl)-D-alaninate**

The methyl (4-(((tert-butyldimethylsilyl)oxy)methyl)-2-nitrophenyl)-D-alaninate (13.0 g, 35.3 mmol) and N-bromosuccinimide (7.5 g, 42.4 mmol) were dissolved in N,N-dimethylformamide (200 mL) to react at 40°C for 1 hour after the addition. The reaction solution was directly used in the next step.

**4. Preparation of methyl (2-bromo-4-(hydroxymethyl)-6-nitrophenyl)-D-alaninate**

The methyl (2-bromo-4-((tert-butyldimethylsilyl)oxy)methyl)-6-nitrophenyl)-D-alaninate (the crude product of the previous step) was added with TBAF (1 M, 35.3 mL) to react at 16°C for 1 hour after the addition. The reaction solution was purified by silica gel column chromatography (n-heptane: ethyl acetate=1: 1) to obtain the product (9.0 g, two-step yield 76.6%).

**5. Preparation of (R)-5-bromo-7-(hydroxymethyl)-3-methyl-3,4-dihydroquinoxaline-2(1H)-one**

The methyl (2-bromo-4-(hydroxymethyl)-6-nitrophenyl)-D-alaninate (10.8 g, 32.4 mmol) was dissolved in methanol (90 mL)/tetrahydrofuran (90 mL)/water (30 mL), and added with iron powder (9.0 g, 161.2 mmol) and ammonium chloride (17.3 g, 323.3 mmol) to react at 70°C for 2 hours. The reaction solution was filtered to remove solids, concentrated, and extracted with water (100 mL) and ethyl acetate (100 mL), and an organic phase was dried and concentrated to obtain the target compound (7.2 g, yield 81.9%).

**6. Preparation of (R,E)-5-(2-ethoxyvinyl)-7-(hydroxymethyl)-3-methyl-3,4-dihydroquinoxaline-2(1H)-one**

The (R)-5-bromo-7-(hydroxymethyl)-3-methyl-3,4-dihydroquinoxaline-2(1H)-one (7.1 g, 26.2 mmol) was dissolved in 1,4-dioxane (150 mL) and water (20.0 mL), and added with 2-(2-ethoxyvinyl)-4,4,5,5-tetramethyl-1,3,2-dioxacyclopentaborane (7.8 g, 39.3 mmol), $Pd(dppf)Cl_2$ (1.9 g, 2.6 mmol) and sodium carbonate (5.6 g, 52.4 mmol). The mixture was subjected to gas exchange with nitrogen to react at 100°C for 2 hours under nitrogen, and it was detected by LCMS until the reaction was finished. The reaction solution was diluted with water, and extracted with ethyl acetate. An organic phase was concentrated and purified by silica gel column chromatography (n-heptane: ethyl acetate=1: 3) to obtain the product (4.6 g, yield 67.0%).

**7. Preparation of (R)-8-(chloromethyl)-3-methyl-1H-pyrrolo[1,2,3-de]quinoxaline-2(3H)-one**

The (R,E)-5-(2-ethoxyvinyl)-7-(hydroxymethyl)-3-methyl-3,4-dihydroquinoxaline-2(1H)-one (1.9 g) was dissolved in dichloromethane (100 mL), and added with an ethyl acetate solution of hydrogen chloride (16 mL) to react at 5°C for 10 minutes. The reaction solution was concentrated, and the crude product was directly used in the next step.

**8. Preparation of (R)-6-fluoro-N-methyl-5-(4-((3-methyl-2-oxo-2,3-dihydro-1H-pyrrolo[1,2,3-de]quinoxaline-8-yl) methyl)piperazine-1-yl)p yridine carboxamide**

The (R)-8-(chloromethyl)-3-methyl-1H-pyrrolo[1,2,3-de]quinoxaline-2(3H)-one (the crude product of the previous step) and 6-fluoro-N-methyl-5-(piperazine-1-yl)picolinamide hydrochloride (1.97 g, 7.2 mmol) were dissolved in

acetonitrile (200 mL), and added with DIEA (9.3 g, 71.8 mmol) to react at 80°C for 2 hours. The reaction solution was concentrated, diluted with water, and extracted with ethyl acetate. An organic phase was dried, concentrated and purified by silica gel column chromatography (dichloromethane: methanol=40: 1) to obtain the product (600 mg, yield 19.0%).

Molecular formula: $C_{23}H_{25}FN_6O_2$, Molecular weight: 436.5, LC-MS (m/z): 437.2($M+H^+$)
$^1$H-NMR(400MHz, $CDCl_3$) δ : 7.95-8.05 (m, 1H), 7.70-7.82 (m, 1H), 7.41-7.58 (m, 1H), 7.12-7.28 (m, 2H), 6.49-6.58 (m, 2H), 5.09-5.20 (m, 1H), 3.64 (s, 2H), 3.15-3.30 (m, 4H), 2.90-3.05 (m, 3H), 2.55-2.70 (m, 4H), 1.85-1.92 (m, 3H).

**Example 16: Preparation of (S)-6-fluoro-N-methyl-5-(4-((3-methyl-2-oxo-2,3-dihydro-1H-pyrrolo[1,2,3-de]quinoxaline-8-yl)methyl)piperazine-1-yl)py ridine carboxamide (Compound 28)**

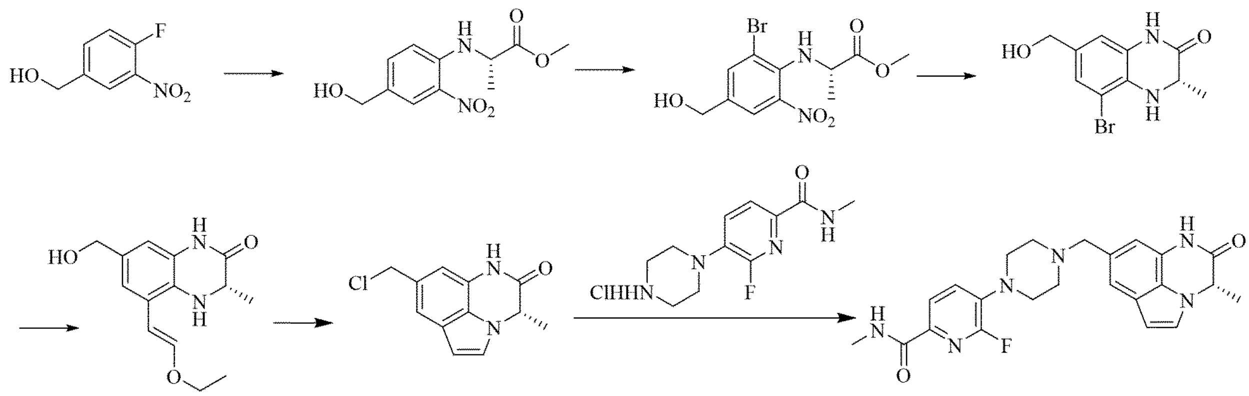

1. **Preparation of methyl (4-(hydroxymethyl)-2-nitrophenyl)-L-alaninate**

(4-fluoro-3-nitrophenyl)methanol (10.3 g, 60.2 mmol) was dissolved in tetrahydrofuran (200 mL), and added with (L)-alanine methyl ester hydrochloride (12.6 g, 90.3 mmol) and N,N-diisopropylethylamine (23.3 g, 0.18 mol) to react at 80°C for 16 hours, and it was detected by LCMS until the reaction was finished. The reaction solution was concentrated, and the crude product was purified by silica gel column chromatography (n-heptane: ethyl acetate=2: 1) to obtain the product (10.0 g, yield 65.4%).

**2. Preparation of methyl (2-bromo-4-(hydroxymethyl)-6-nitrophenyl)-L-alaninate**

The methyl (4-(hydroxymethyl)-2-nitrophenyl)-L-alaninate (10.0 g, 39.4 mmol) and N-bromosuccinimide (7.7 g, 43.3 mmol) were dissolved in N,N-dimethylformamide (100 mL) to react at 10°C for 16 hours after the addition. The reaction solution was poured into water, and extracted with ethyl acetate. An organic phase was concentrated, and the crude product was purified by silica gel column chromatography (n-heptane: ethyl acetate=1: 1) to obtain the product (7.0 g, yield 53.6%).

**3. Preparation of (S)-5-bromo-7-(hydroxymethyl)-3-methyl-3,4-dihydroquinoxaline-2(1H)-one**

The methyl (2-bromo-4-(hydroxymethyl)-6-nitrophenyl)-L-alaninate (7.0 g, 21.1 mmol) was dissolved in methanol (40 mL)/tetrahydrofuran (40 mL)/water (10 mL), and added with iron powder (7.3 g, 0.13 mol) and ammonium chloride (13 g, 0.25 mol) to react at 70°C for 2 hours. The reaction solution was filtered to remove solids, concentrated, and extracted with water (30 mL) and ethyl acetate (30 mL), and an organic phase was dried and concentrated to obtain the target compound (4.0g, yield 70.2%).

**4. Preparation of (S,E)-5-(2-ethoxyvinyl)-7-(hydroxymethyl)-3-methyl-3,4-dihydroquinoxaline-2(1H)-one**

The (S)-5-bromo-7-(hydroxymethyl)-3-methyl-3,4-dihydroquinoxaline-2(1H)-one (70 mg, 0.26 mmol) was dissolved in 1,4-dioxane (5 mL) and water (0.5 mL), and added with 2-(2-ethoxyvinyl)-4,4,5,5-tetramethyl-1,3,2-dioxacyclopentaborane (61 mg, 0.31 mmol), Pd(dppf)$Cl_2$ (19 mg, 26.0 μmol) and sodium carbonate (55 mg, 0.52 mmol). The mixture was subjected to gas exchange with nitrogen to react at 90 °C for 2 hours under nitrogen, and it was detected by LCMS until the reaction was finished. The reaction solution was diluted with water, and extracted with ethyl acetate. An

organic phase was concentrated and purified by silica gel column chromatography (n-heptane: ethyl acetate=1: 3) to obtain the product (40 mg, yield 58.8%).

**5. Preparation of (S)-8-(chloromethyl)-3-methyl-1H-pyrrolo[1,2,3-de]quinoxaline-2(3H)-one**

The (S,E)-5-(2-ethoxyvinyl)-7-(hydroxymethyl)-3-methyl-3,4-dihydroquinoxaline-2(1H)-one (40 mg, 0.15 mmol) was dissolved in dichloromethane (10 mL), and added with an ethyl acetate solution of hydrogen chloride (0.8 mL) to react at 5°C for 10 minutes. The reaction solution was concentrated, and the crude product was directly used in the next step.

**6. Preparation of (S)-6-fluoro-N-methyl-5-(4-((3-methyl-2-oxo-2,3-dihydro-1H-pyrrolo[1,2,3-de]quinoxaline-8-yl) methyl)piperazine-1-yl)py ridine carboxamide**

The (S)-8-(chloromethyl)-3-methyl-1H-pyrrolo[1,2,3-de]quinoxaline-2(3H)-one (the crude product of the previous step) and 6-fluoro-N-methyl-5-(piperazine-1-yl)pyridine carboxamide hydrochloride (41 mg, 0.15 mmol) were dissolved in acetonitrile (10 mL), and added with DIEA (58 mg, 0.45 mmol) to react at 80°C for 2 hours. The reaction solution was concentrated, diluted with water, and extracted with ethyl acetate. An organic phase was dried, concentrated and purified by silica gel plate (dichloromethane: methanol=15: 1) to obtain the product (11 mg, two-step yield 16.7%).

Molecular formula: $C_{23}H_{25}FN_6O_2$, Molecular weight: 436.5, LC-MS (m/z): 437.2($M+H^+$)
$^1$H-NMR (400MHz, $CDCl_3$) δ: 7.95-8.05 (m, 1H), 7.70-7.82 (m, 1H), 7.41-7.58 (m, 1H), 7.12-7.28 (m, 2H), 6.49-6.58 (m, 2H), 5.09-5.20 (m, 1H) ,3.64 (s, 2H), 3.15-3.30 (m, 4H), 2.90-3.05 (m, 3H), 2.55-2.70 (m, 4H), 1.85-1.92 (m, 3H).

**Example 17: Preparation of 6-fluoro-N-methyl-5-(4-((3-methyl-2-oxo-1,2,4,5-tetrahydro cyclopentane[de]quinoline-7-yl)methyl)piperazine-1-yl)pyridine carboxamide (Compound 31)**

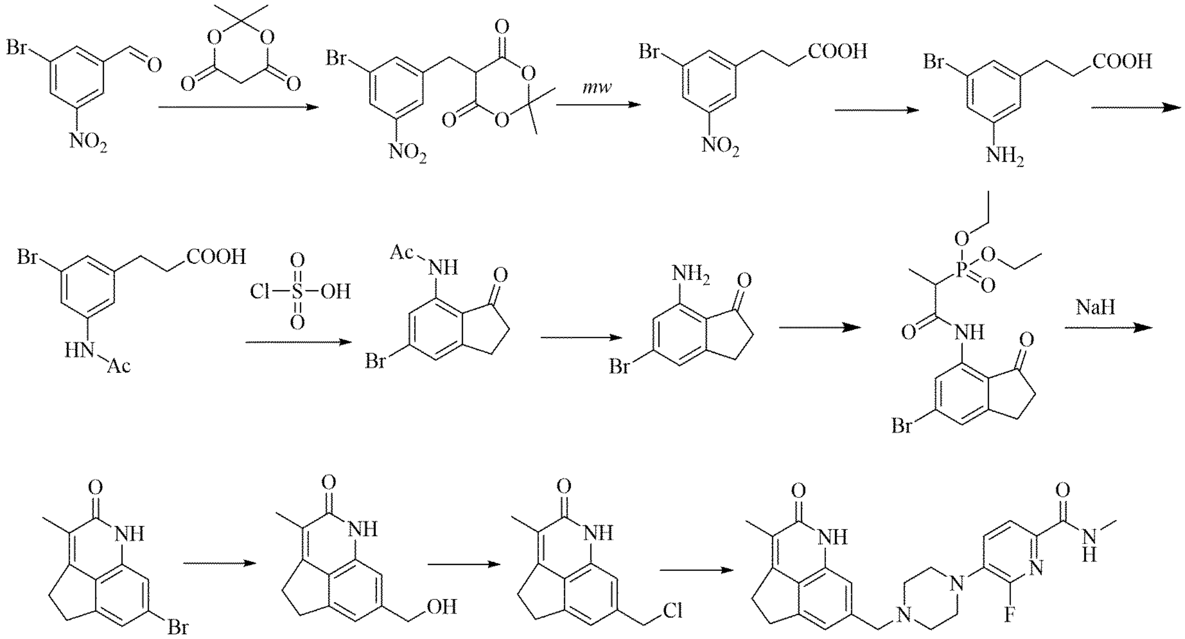


1. **Preparation of 5-(3-bromo-5-nitrobenzyl)-2,2-dimethyl-1,3-dioxane-4,6-dione**

Triethylamine (100 mL) was dropwise added with formic acid (130 mL) at 0°C, and stirred for 30 minutes. The mixture was added with 3-bromo-5-nitrobenzaldehyde (9.0 g, 39.1 mmol), 2,2-dimethyl-1,3-dioxane-4,6-dione (5.6 g, 39.1 mmol) and N,N-dimethylformamide (50 mL) to react at 25°C for 12 hours. After the reaction was finished, the reaction solution was added with water (200 mL) for quenching, and extracted with ethyl acetate (100 mL*3). An organic phase was collected, and spin-dried to obtain a crude product (20.0 g). The crude product was directly used in the next step of reaction.

**2. Preparation of 3-(3-bromo-5-nitrophenyl)propionic acid**

An acetonitrile (100 mL) solution of 5-(3-bromo-5-nitrobenzyl)-2,2-dimethyl-1,3-dioxane-4,6-dione (20.0 g, the crude product) was added with water (10 mL) to react at 120°C for 6 hours. After the reaction was finished, the reaction solution was spin-dried, added with water (100 mL), and added with 2 M hydrochloric acid solution to adjust a pH value to be 1. The reaction solution was extracted with ethyl acetate (100 mL*3). An organic phase was collected, spin-dried, and purified by column chromatography ($SiO_2$, petroleum ether: ethyl acetate=1: 1) to obtain the product (10.0 g, two-step yield 93.2%).

3. **Preparation of 3-(3-amino-5-bromophenyl)propionic acid**

An ethanol/water (100 mL/20 mL) solution of 3-(3-bromo-5-nitrophenyl)propionic acid (10.0 g, 36.5 mmol) was added with iron powder (6.1 g, 109.5 mmol) and ammonium chloride (5.9 g, 109.5 mmol) to react at 80°C for 12 hours. After the reaction was finished, the reaction solution was concentrated and purified by column chromatography ($SiO_2$, 100% ethyl acetate) to obtain the product (6.4 g, yield 71.9%).

**4. Preparation of 3-(3-acetamido-5-bromophenyl)propionic acid**

Acetic anhydride (50 mL) was added with 3-(3-amino-5-bromophenyl)propionic acid (6.4 g, 26.2 mmol) to react at 25°C for 1 hour, and the reaction was finished. The reaction solution was concentrated, and the crude product was purified by silica gel column chromatography (n-heptane: ethyl acetate=1: 1) to obtain the product (2.4 g, yield 32.0%).

**5. Preparation of N-(6-bromo-3-oxo-2,3-dihydro-1H-indene-4-yl)acetamide**

Chlorosulfonic acid (20 mL) was added with 3-(3-acetamido-5-bromophenyl)propionic acid (2.4 g, 8.4 mmol) to react at 0°C for 3 hours, and the reaction was finished. The reaction solution was slowly poured into ice water, and extracted with ethyl acetate (50 mL*2). An organic phase was collected and concentrated, and the crude product was purified by silica gel column chromatography (n-heptane: ethyl acetate=4: 1) to obtain the product (898 mg, yield 39.9%).

**6. Preparation of 7-amino-5-bromo-2,3-dihydro-1H-indene-1-one**

A methanol (20 mL) solution of N-(6-bromo-3-oxo-2,3-dihydro-1H-indene-4-yl)acetamide (898 mg, 3.4 mmol) was added with lithium hydroxide monohydrate (286 mg, 6.8 mmol) to react at 25°C for 1 hour, and the reaction was finished. The reaction solution was spin-dried, and the crude product was purified by silica gel column chromatography (dichloromethane: methanol=20: 1) to obtain the product (600 mg, yield 79.2%).

**7. Preparation of diethyl (1-((6-bromo-3-oxo-2,3-dihydro-1H-indene-4-yl)amino)-1-oxopropyl-2-yl)phosphite**

The 7-amino-5-bromo-2,3-dihydro-1H-indene-1-one (300 mg, 1.3 mmol) and 2-(diethoxyphosphoryl)propionic acid (307 mg, 1.5 mmol) were dissolved in N,N-dimethylformamide (4 mL), and added with EDCI (633 mg, 3.3 mmol) to react at 25°C for 2 hours, and the reaction was finished. The reaction solution was added with water (10 mL) and ethyl acetate (15 mLx2). An organic phase was washed with water (20 mLx2), dried and concentrated to obtain a crude product (500 mg). The crude product was directly used in the next step.

**8. Preparation of 7-bromo-3-methyl-4,5-dihydrocyclopentane[de]quinoline-2(1H)-one**

The diethyl (1-((6-bromo-3-oxo-2,3-dihydro-1H-indene-4-yl)amino)-1-oxopropyl-2-yl)phosphite (500 mg, the crude product) was dissolved in N,N-dimethylformamide (10 mL), and added with NaH (60%)(156 mg, 3.9 mmol) to react by microwave at 100°C for 30 minutes, and the reaction was finished. The reaction solution was concentrated, and the crude product was purified by silica gel column chromatography (dichloromethane: methanol=15: 1) to obtain the product (200 mg, two-step yield 57.1%).

**9. Preparation of 7-(hydroxymethyl)-3-methyl-4,5-dihydrocyclopentane[de]quinoline-2(1H)-one**

The 7-bromo-3-methyl-4,5-dihydrocyclopentane[de]quinoline-2(1H)-one (200 mg, 0.76 mmol) was dissolved in 1,4-dioxane (10 mL), and added with (tributyltin)methanol (365 mg, 1.1 mmol) and XPhos Pd G2 (60 mg, 0.076 mmol) to react at 90°C for 2 hours, and the reaction was finished. The reaction solution was concentrated, and the crude product was

purified by silica gel column chromatography (dichloromethane: methanol=15: 1) to obtain the product (80 mg, yield 49.1%).

**10. Preparation of 7-(chloromethyl)-3-methyl-4,5-dihydrocyclopentane[de]quinoline-2(1H)-one**

The 7-(hydroxymethyl)-3-methyl-4,5-dihydrocyclopentane[de]quinoline-2(1H)-one (80 mg, 0.37 mmol) and N,N-dimethylformamide (9 mg, 0.12 mmol) were dissolved in dichloromethane (7 mL), and added with thionyl chloride (78 mg, 0.66 mmol) to react at 25°C for 1 hour. The reaction solution was concentrated, and the crude product was directly used in the next step.

**11. Preparation of 6-fluoro-N-methyl-5-(4-((3-methyl-2-oxo-1,2,4,5-tetrahydrocyclopentane[de]quinoline-7-yl) methyl)piperazine-1-yl)pyridi ne carboxamide**

The 7-(chloromethyl)-3-methyl-4,5-dihydrocyclopentane[de]quinoline-2(1H)-one (the crude product of the previous step) and 6-fluoro-N-methyl-5-(piperazine-1-yl)picolinamide hydrochloride (90 mg, 0.37 mmol) were dissolved in acetonitrile (7 mL), and added with DIEA (143 mg, 1.1 mmol) and potassium carbonate (152 mg, 1.1 mmol) to react at 85°C for 10 hours, and the reaction was finished. The reaction solution was concentrated, and the crude product was purified by Prep-TLC (dichloromethane: methanol=10: 1) to obtain the product (25 mg, two-step yield 15.5%).

Molecular formula: $C_{24}H_{26}FN_5O_2$, Molecular weight: 435.2, LC-MS (m/z): 436.2(M+H$^+$)
$^1$H-NMR(400MHz, DMSO-d6) $\delta$: 11.33 (s, 1H), 8.38 (s, 1H), 7.83 (d, $J$=8.0 Hz, 1H), 7.55 (t, $J$=9.4 Hz, 1H), 6.99 (s, 1H), 6.94 (s, 1H), 3.57 (s, 2H), 3.32-3.07 (m, 8H), 2.76 (s, 3H), 2.65-2.50 (m, 4H), 2.00 (s, 3H).

**Example 18: Preparation of 6-fluoro-5-(4-((8-fluoro-3-methyl-2-oxo-1,2,4,5-tetrahydrocyclopentyl[de]quinoline-7-yl)methyl)piperazine-1-yl)-N-methy lpicolinamide (Compound 6)**

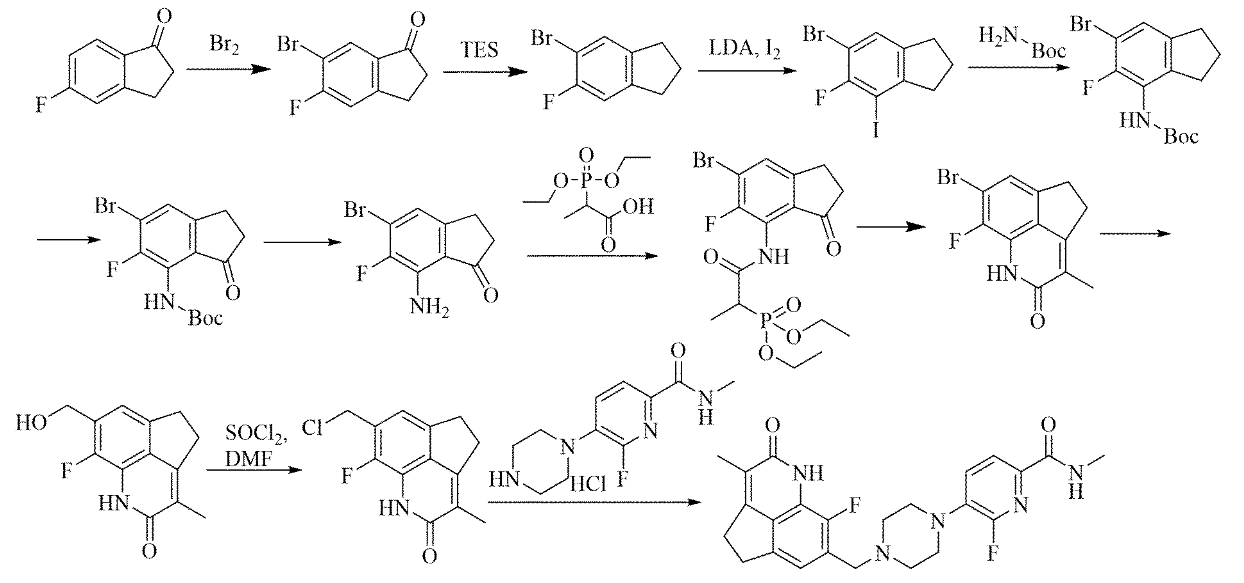


**1. Preparation of 6-bromo-5-fluoro-2,3-dihydro-1H-indene-1-one**

A 1,2-dichloroethane (100 mL) solution of 5-fluoro-2,3-dihydro-1H-indene-1-one (24 g, 159.5 mmol) was added into a 1,2-dichloroethane (400 mL) solution of aluminum trichloride (53.3 g, 399.8 mmol) to react at 25°C for 10 minutes, and then the reaction solution was dropwise added with bromine (38 g, 237.8 mmol), and heated to 70°C to react for 2 hours. The reaction solution was poured into ice water and 1M hydrochloric acid solution (300 mL), and then added with ethyl acetate (300 mL). The mixture was filtered with diatomite, and the filtrate was subjected to liquid separation. An organic phase was concentrated, and purified by column chromatography (n-heptane: ethyl acetate=2: 1) to obtain the product (11.1 g, yield 30.3%).

**2. Preparation of 5-bromo-6-fluoro-2,3-dihydro-1H-indene**

The 6-bromo-5-fluoro-2,3-dihydro-1H-indene-1-one (7.0 g, 30.6 mmol) was added into trifluoroacetic acid (80 mL), and then added with triethylsilane (80 mL) to react at 25°C for 18 hours. The reaction solution was concentrated, and purified by column chromatography (eluted with n-heptane) to obtain the product (5.5 g, yield 83.7%).

**3. Preparation of 6-bromo-5-fluoro-4-iodine-2,3-dihydro-1H-indene**

The 5-bromo-6-fluoro-2,3-dihydro-1H-indene (5.75 g, 26.75 mmol) was dissolved in tetrahydrofuran (125 mL), cooled to -75°C, and stirred for 10 minutes. The mixture was slowly dropwise added with LDA (20.4 mL, 40.8 mmol) to react at -65°C ~ -75°C for 30 minutes, and then the reaction solution was added with saturated sodium sulfite (70 mL) for quenching, and then extracted with ethyl acetate (150 mL). An organic phase was concentrated, and purified by reversed-phase column chromatography (acetonitrile/water=10%-90%) to obtain the product (3.7 g, yield 40.4%).

**4. Preparation of tert-butyl (6-bromo-5-fluoro-2,3-dihydro-1H-indene-4-yl)carbamate**

The 6-bromo-5-fluoro-4-iodine-2,3-dihydro-1H-indene (2.7 g, 7.92 mmol), $NH_2Boc$ (0.93 g, 7.94 mmol), Xant-Phos (0.46 g, 0.80 mmol), $Pd_2(dba)_3$ (0.36 g, 0.39 mmol) and cesium carbonate (5.2 g, 16.0 mmol) were added into toluene (100 mL) in sequence to react at 105°C for 18 hours under the protection of $N_2$, and the reaction was finished. The reaction solution was concentrated, and the crude product was purified by silica gel column chromatography (ethyl acetate/petroleum ether= 0%-30%) to obtain the product (1.1 g, yield 42.1%).

**5. Preparation of tert-butyl (6-bromo-5-fluoro-3-oxo-2,3-dihydro-1H-indene-4-yl)carbamate**

The tert-butyl (6-bromo-5-fluoro-2,3-dihydro-1H-indene-4-yl)carbamate (1.0 g, 3 mmol) was dissolved in acetone (60 mL), and added with an aqueous solution of magnesium sulfate (6.4 g, 15%) and potassium permanganate (1.5 g, 9.5 mmol) to react at 25°C for 18 hours, and the reaction was finished. The reaction solution was concentrated, and the crude product was purified by silica gel column chromatography (ethyl acetate/petroleum ether= 0%-33%) to obtain the product (0.9 g, yield 86.3%).

**6. Preparation of 7-amino-5-bromo-6-fluoro-2,3-dihydro-1H-indene-1-one**

The tert-butyl (6-bromo-5-fluoro-3-oxo-2,3-dihydro-1H-indene-4-yl)carbamate (900 mg, 2.6 mmol) was dissolved in ethyl acetate (5 mL), and added with HCl/EA (9 mL, 36 mmol) to react at 25°C for 16 hours, and the reaction was finished. The reaction solution was concentrated to obtain the product (700 mg, yield 95.4%).

**7. Preparation of diethyl (1-((6-bromo-5-fluoro-3-oxo-2,3-dihydro-1H-indene-4-yl)amino)-1-oxopropyl-2-yl) phosphate**

2-(diethoxyphosphoryl)propionic acid (810 mg, 3.8 mmol) was dissolved in DCM (20 mL), and added with oxalyl chloride (800 mg, 6.3 mmol) and N,N-dimethylformamide (0.5 mL) to react at 25°C for 0.5 hour, so as to obtain a solution for later use.

The 7-amino-5-bromo-6-fluoro-2,3-dihydro-1H-indene-1-one (600 mg, 2.1 mmol) and DIEA (1.1 g, 8.5 mmol) were dissolved in THF (20 mL), and then the above solution was added into the reaction solution to react at 25°C for 2 hours, and the reaction was finished. The reaction solution was concentrated, and the crude product was purified by silica gel column chromatography (ethyl acetate: petroleum ether=1: 1) to obtain the product (1.0 g, yield 93.2%).

**8. Preparation of 7-bromo-8-fluoro-3-methyl-4,5-dihydrocyclopentadiene[de]quinoline-2(1*H*)-one**

The diethyl (1-((6-bromo-5-fluoro-3-oxo-2,3-dihydro-1H-indene-4-yl)amino)-1-oxopropyl-2-yl)phosphate (500 mg, 1.15 mmol) and NaH (124 mg, 3.1 mmol) were dissolved in DMF (15 mL), and stirred by microwave at 100°C for 20 minutes. After the reaction was finished, the reaction solution was poured into water (50 mL), and extracted with ethyl acetate (100 mL×3). Organic phases were combined and concentrated, and the crude product was purified by silica gel column chromatography (ethyl acetate/petroleum ether=10%-50%) to obtain the product (100 mg, yield 30.9%).

**9. Preparation of 8-fluoro-7-(hydroxymethyl)-3-methyl-4,5-dihydrocyclopentadiene[de]quinoline-2(1H)-one**

The 7-bromo-8-fluoro-3-methyl-4,5-dihydrocyclopentadiene[de]quinoline-2(1*H*)-one (150 mg, 0.53 mmol),

(acetoxymethyl)potassium trifluoroborate (150 mg, 1.06 mmol), sodium carbonate (170 mg, 1.6 mmol) and RuPhosPdG3 (46 mg, 0.05 mmol) were added into dioxane (25 mL) and water (1 mL) in sequence to react at 95°C for 16 hours under the protection of $N_2$, and the reaction was finished. The reaction solution was concentrated, and the crude product was purified by silica gel column chromatography (ethyl acetate/petroleum ether= 20%-100%) to obtain the product (50 mg, yield 40.3%).

**10. Preparation of 7-(chloromethyl)-8-fluoro-3-methyl-4,5-dihydrocyclopentadiene[de]quinoline-2(1H)-one**

The 8-fluoro-7-(hydroxymethyl)-3-methyl-4,5-dihydrocyclopentadiene[de]quinoline-2(1H)-one (50 mg, 0.21 mmol) and N,N-dimethylformamide (200 mg) were dissolved in dichloromethane (7 mL), and added with thionyl chloride (250 mg, 2.1 mmol) to react at 20°C for 2 hours. The reaction solution was concentrated, and the crude product was directly used in the next step.

**11. Preparation of 6-fluoro-5-(4-((8-fluoro-3-methyl-2-oxo-1,2,4,5-tetrahydrocyclopentyl[de]quinoline-7-yl) methyl)piperazine-1-yl)-N-methy lpicolinamide**

The 7-(chloromethyl)-8-fluoro-3-methyl-4,5-dihydrocyclopentadiene[de]quinoline-2(1H)-one (the crude product of the previous step) and 6-fluoro-N-methyl-5-(piperazine-1-yl)picolinamide hydrochloride (82 mg, 0.3 mmol) were dissolved in acetonitrile (40 mL), and added with DIEA (257 mg, 1.99 mmol) to react at 85°C for 2 hours. After the reaction was finished, solids were precipitated and filtered out, and the filter cake was washed with acetonitrile (5 mL). The filter cake was collected to obtain the product (35 mg, two-step yield 36.0%).

Molecular formula: $C_{24}H_{25}F_2N_3O_2$, Molecular weight: 453.5, LC-MS (m/z): 454.3(M+$H^+$)
$^1$H-NMR(400MHz, DMSO) $\delta$: 11.59 (s, 1H), 8.37 (d, *J*=4.6 Hz, 1H), 7.81 (d, *J*=7.8 Hz, 1H), 7.53 (t, *J*=8.7 Hz, 1H), 7.01 (d, *J*=4.2 Hz, 1H), 3.63 (s, 2H), 3.30 (s, 2H), 3.12-3.17 (m, 6H), 2.74 (d, *J*=4.5 Hz, 3H), 2.56 (s, 4H), 1.96 (s, 3H).

**Example 19: Preparation of (R)-6-fluoro-N-methyl-5-(4-((4-methyl-5-oxo-5,6-dihydro-4H-imidazo[1,5,4-de]quinoxaline-8-yl)methyl)piperazine-1-yl)p yridine carboxamide (Compound 29-1)**

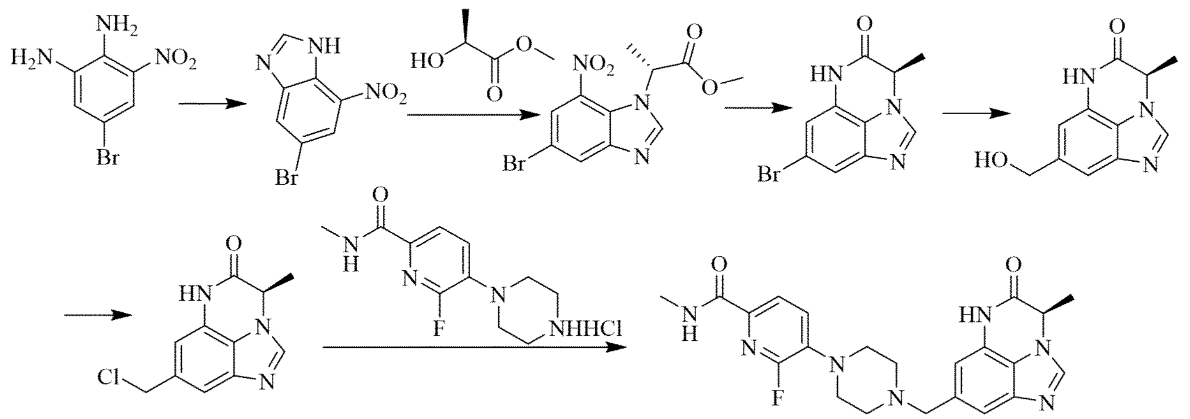

**1. Preparation of 5-bromo-7-nitro-1H-benzo[d]imidazole**

5-bromo-3-nitrobenzene-1,2-diamine (4.0 g, 17.2 mmol) was dissolved in HCl (4 M, 50 mL), and added with formic acid (950 mg, 20.7 mmol). The system reacted at 120°C for 1 hour and then was added with ammonia water to adjust a pH value to be 7, solids were precipitated and filtered out, and the filter cake was spin-dried to obtain the product (3.8 g, yield 91.1%).

**2. Preparation of methyl (R)-2-(5-bromo-7-nitro-1H-benzo[d]imidazole-1-yl)propionate**

The 5-bromo-7-nitro-1H-benzo[d]imidazole (1.2 g, 5.0 mmol) was dissolved in tetrahydrofuran (50 mL), and added with triphenylphosphine (2.0 g, 7.5 mmol), methyl (S)-2-hydroxypropionate (885 mg, 7.5 mmol) and diisopropyl azodicarboxylate (1.5 g, 7.5 mmol). The system reacted at 20°C for 1 hour, and then was concentrated, washed with water,

extracted with ethyl acetate and concentrated, and the crude product was purified by column chromatography (EA: PE=1: 1) to obtain the product (600 mg, yield 35.3%).

**3. Preparation of (R)-8-bromo-4-methyl-4H-imidazo[1,5,4-de]quinoxaline-5(6H)-one**

The methyl (R)-2-(5-bromo-7-nitro-1H-benzo[d]imidazole-1-yl)propionate (300 mg, 0.91 mmol), zinc powder (572 mg, 8.8 mmol) and ammonium chloride (466 mg, 8.8 mmol) were dissolved in methanol (20 mL), water (5 mL) and tetrahydrofuran (20 mL) to react at 70°C for 2 hours. The reaction solution was extracted with ethyl acetate, and then an organic phase was concentrated, and directly used in the next step.

**4. Preparation of (R)-8-(hydroxymethyl)-4-methyl-4H-imidazo[1,5,4-delquinoxaline-5(6H)-one**

The (R)-8-bromo-4-methyl-4H-imidazo[1,5,4-de]quinoxaline-5(6H)-one (the crude product of the previous step), tributyltin methanol (282 mg, 0.88 mmol) and XPhos Pd G2 (69 mg, 88.0 μmol) were dissolved in dioxane (10 mL) to react at 80°C for 2 hours after the addition, and the reaction was finished. The reaction solution was extracted with water and ethyl acetate, an organic phase was collected, concentrated, and purified by column chromatography (MeOH: DCM=1: 10) to obtain the product (60 mg, yield 31.6%).

**5. Preparation of (R)-8-(chloromethyl)-4-methyl-4H-imidazo[1,5,4-de]quinoxaline-5(6H)-one**

The (R)-8-(hydroxymethyl)-4-methyl-4H-imidazo[1,5,4-de]quinoxaline-5(6H)-one (60 mg, 0.28 mmol) was dissolved in DCM (10 mL), and added with sulfoxide chloride (322 mg, 2.8 mmol) and DMF (21 mg, 0.28 mmol) to react at 20°C for 6 hours after the addition. After the reaction was finished, the crude product was concentrated, and directly used in the next step of reaction.

**6. Preparation of (R)-6-fluoro-N-methyl-5-(4-((4-methyl-5-oxo-5,6-dihydro-4H-imidazo[1,5,4-de])quinoxaline-8-yl)methyl)piperazine-1-yl) pyridine carboxamide**

The (R)-8-(chloromethyl)-4-methyl-4H-imidazo[1,5,4-de]quinoxaline-5(6H)-one (the crude product of the previous step) was dissolved in acetonitrile (10 mL), and added with DIEA (98 mg, 0.76 mmol) and 6-fluoro-N-methyl-5-(piperazine-1-yl)pyridine carboxamide hydrochloride (77 mg, 0.28 mmol) to react at 70°C for 2 hours. After the reaction was finished, the reaction solution was concentrated and purified by column chromatography (MeoH: DCM=1: 10) to obtain the product (30 mg, two-step yield: 24.8%).

Molecular formula: $C_{22}H_{24}FN_7O_2$, Molecular weight: 437.5, LC-MS (M/e): 438.1(M +$H^+$)
$^1$H-NMR(400 MHz, $CDCl_3$) $\delta$: 7.98 (m, 2H), 7.81 (s, 1H), 7.49 (s, 1H), 7.37 (s, 1H), 7.30 (s, 1H), 6.75 (s, 1H), 5.27-5.25 (m, 1H), 3.65 (s, 2H), 3.21-3.09 (m, 4H), 2.99-2.98 (m, 3H), 2.65-2.61 (m, 4H), 1.93-1.92 (m, 3H).

**Example 20: Preparation of (S)-6-fluoro-N-methyl-5-(4-((4-methyl-5-oxo-5,6-dihydro-4H-imidazo[1,5,4-de]quinoxaline-8-yl)methyl)piperazine-1-yl)p yridine carboxamide (Compound 29-2)**

Referring to the preparation by the method of Example 19, the chiral intermediate methyl (S)-2-hydroxypropionate was replaced by methyl (R)-2-hydroxypropionate.

Molecular formula: $C_{22}H_{24}FN_7O_2$, Molecular weight: 437.5, LC-MS (M/e): 438.1(M +$H^+$)
$^1$H-NMR(400 MHz, $CDCl_3$) $\delta$: 7.98 (m,2H), 7.81 (s, 1H), 7.49 (s, 1H), 7.37 (s, 1H), 7.30 (s, 1H), 6.75 (s, 1H), 5.27-5.25(m, 1H), 3.65 (s, 2H), 3.21-3.09 (m, 4H), 2.99-2.98 (m, 3H), 2.65-2.61 (m, 4H), 1.93-1.92 (m, 3H).

**Example 21: Preparation of (Compound 38)**

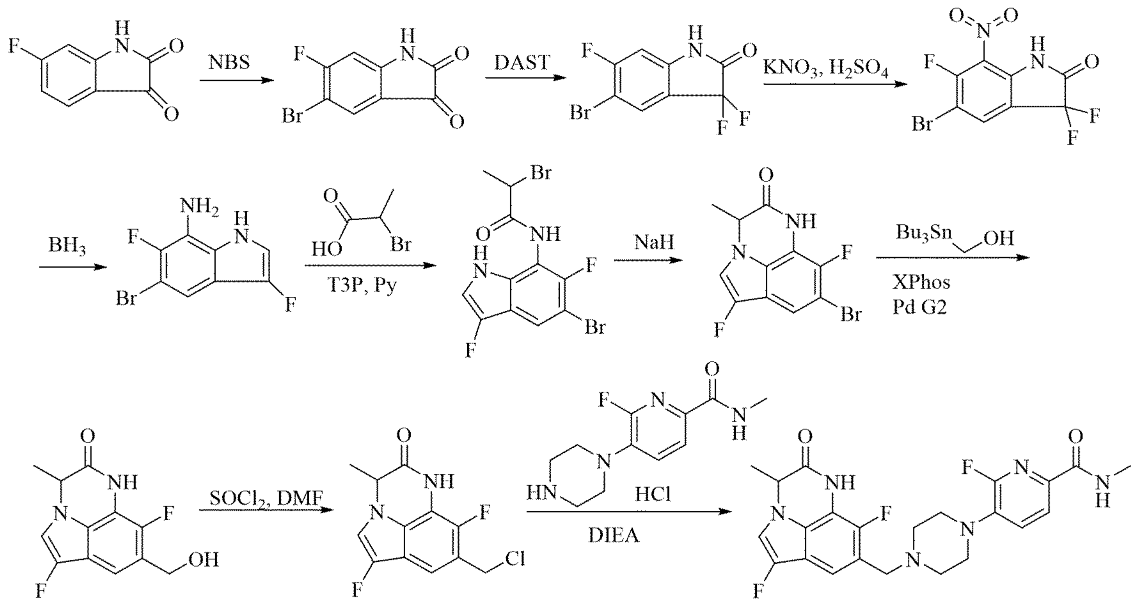


**1. Preparation of 5-bromo-6-fluoroindole-2,3-dione**

6-fluoroindole-2,3-dione (10.0 g, 60.6 mmol) was dissolved in DMF (150 mL), and added with NBS (12.0 g, 67.4 mmol) to react at 10°C for 16 hours. The reaction solution was slowly poured into ice water to precipitate solids, filtered to remove the filtrate, and dried to obtain a crude product (8.0 g). The crude product was purified by silica gel column chromatography (ethyl acetate: tetrahydrofuran=9: 1) to obtain the product (4 g, yield 27.1%).

**2. Preparation of 5-bromo-3,3,6-trifluoroindole-2-one**

The 5-bromo-6-fluoroindole-2,3-dione (4.0 g, 16.4 mmol) was dissolved in dichloromethane (110 mL), and added with DAST (7.9 g, 49.0 mmol) to react at 15°C for 3 hours. The reaction solution was added with water for quenching, extracted, and purified by silica gel column chromatography (n-heptane: ethyl acetate =2: 1) to obtain the product (2.2 g, yield 50.5%).

**3. Preparation of 5-bromo-3,3,6-trifluoro-7-nitroindole-2-one**

The 5-bromo-3,3,6-trifluoroindole-2-one (2.2 g, 8.3 mmol) was dissolved in concentrated sulfuric acid (30 mL), and added with potassium nitrate (1.3 g, 12.9 mmol) to react at 0°C for 1 hour. The reaction solution was slowly poured into ice water to precipitate solids, filtered to remove the filtrate, and dried to obtain the product (2.0 g, yield 77.8%).

**4. Preparation of 5-bromo-3,6-difluoro-1H-indole-7-amine**

The 5-bromo-3,3,6-trifluoro-7-nitroindole-2-one (2.0 g, 6.4 mmol) was dissolved in tetrahydrofuran (10 mL), and added with a borane-tetrahydrofuran solution (1 M, 30 mL) to react at 70°C for 2 hours. The reaction solution was added with methanol for quenching, concentrated, and purified by silica gel column chromatography (n-heptane: ethyl acetate=1: 1) to obtain a crude product (1.2 g), and the crude product was purified by C18 column (methanol: water=0-46%) to obtain the product (700 mg, yield 44.1%).

**5. Preparation of 2-bromo-N-(5-bromo-3,6-difluoro-1H-indole-7-yl)propionamide**

The 5-bromo-3,6-difluoro-1H-indole-7-amine (700 mg, 2.8 mmol), 2-bromopropionic acid (875 mg, 5.7 mmol) and pyridine (890 mg, 11.3 mmol) were dissolved in ethyl acetate (40 mL), cool to -10°C, and added with an ethyl acetate solution of 1-cyclopropyl phosphoric anhydride (50%, 5.4 g, 8.5 mmol) to react at -10°C for 1 hour. The reaction solution was extracted with water and ethyl acetate, and the crude product was purified by silica gel column chromatography (n-heptane: ethyl acetate=2: 1) to obtain the product (1.0 g, yield 92.4%).

**6. Preparation of 8-bromo-6,9-difluoro-3-methyl-1H-pyrrolo[1,2,3-de]quinoxaline-2(3H)-one**

The 2-bromo-N-(5-bromo-3,6-difluoro-1H-indole-7-yl)propionamide (1.0 g, 2.6 mmol) was dissolved in DMF (30 mL), and added with 60% sodium hydride (266 mg, 6.7 mmol) to react at 20°C for 0.5 hour. The reaction solution was added with water for quenching, and extracted with water and ethyl acetate, and the crude product was purified by silica gel column chromatography (n-heptane: ethyl acetate=2: 1) to obtain the product (68 mg, yield 86.3%).

**7. Preparation of 6,9-difluoro-8-(hydroxymethyl)-3-methyl-1H-pyrrolo[1,2,3-de]quinoxaline-2(3H)-one**

The 8-bromo-6,9-difluoro-3-methyl-1H-pyrrolo[1,2,3-de]quinoxaline-2(3H)-one (680 mg, 2.3 mmol), tri-n-butyltin methanol (872 mg, 2.7 mmol) and Xphos Pd G2 (182 mg, 0.23 mmol) were dissolved in 1,4-dioxane (70 mL) to react at 85°C for 2 hours. The reaction solution was concentrated, and the crude product was purified by silica gel column chromatography (n-heptane: ethyl acetate=1: 2) to obtain the product (440 mg, yield 77.3%).

**8. Preparation of 8-(chloromethyl)-6,9-difluoro-3-methyl-1H-pyrrolo[1,2,3-de]quinoxaline-2(3H)-one**

The 6,9-difluoro-8-(hydroxymethyl)-3-methyl-1H-pyrrolo[1,2,3-de]quinoxaline-2(3H)-one (100 mg, 0.40 mmol) was dissolved in dichloromethane (20 mL), and dropwise added with N,N-dimethylformamide (74 mg, 1.0 mmol) and thionyl chloride (240 mg, 2.0 mmol) in sequence to react at 20°C for 2 hours. The reaction solution was concentrated, and the crude product was directly used in the next step.

**9. Preparation of 5-(4-((6,9-difluoro-3-methyl-2-oxo-2,3-dihydro-1H-pyrrolo[1,2,3-de]quinoxaline-8-yl)methyl) piperazine-1-yl)-6-fluoro-N-methylpyridine formamide**

The 8-(chloromethyl)-6,9-difluoro-3-methyl-1H-pyrrolo[1,2,3-de]quinoxaline-2(3H)-one (the crude product of the previous step) and 6-fluoro-N-methyl-5-(piperazine-1-yl)picolinamide hydrochloride (110 mg, 0.40 mmol) were dissolved in acetonitrile (20 mL), and added with DIEA (260 mg, 2.0 mmol) to react at 80°C for 16 hours. The reaction solution was concentrated, and extracted with water and (dichloromethane: methanol=10:1), and the crude product was purified by silica gel column chromatography (dichloromethane: methanol=30: 1) to obtain the product (130 mg, two-step yield 69.4%).

Molecular formula: $C_{23}H_{23}F_3N_6O_2$, Molecular weight: 472.5, LC-MS (m/z): 473.2(M+H$^+$)
$^1$H-NMR(400MHz, $CDCl_3$) 7.99 (d, *J*=8.0 Hz, 1H), 7.84 (s, 1H), 7.49 (d, *J*=4.8 Hz, 1H), 7.32-7.29 (m, 1H), 7.18 (d, *J*=5.6 Hz, 1H), 6.94 (d, *J*=3.2 Hz, 1H), 5.03 (t, *J*=6.8 Hz, 1H), 3.73 (s, 2H), 3.24 (t, *J*=4.8 Hz, 4H), 3.00 (d, *J*=5.0 Hz, 3H), 2.71 (t, *J*=4.8 Hz, 4H), 1.79 (d, *J*=7.0 Hz, 3H)

**Example 22: Preparation of 5-(4-((3,3-dimethyl-2-oxo-2,3-dihydro-1H-pyrrolo[1,2,3-de]quinoxaline-8-yl)methyl) piperazine-1-yl)-6-fluoro-N-methylpicolinamide (Compound 46)**

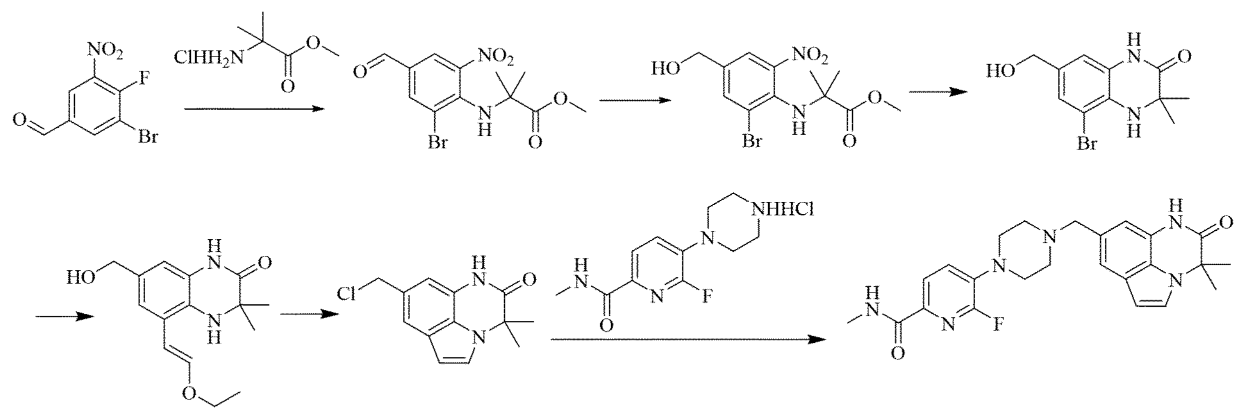

**1. Preparation of methyl 2-((2-bromo-4-formyl-6-nitrophenyl)amino)-2-methylpropionate**

3-bromo-4-fluoro-5-nitrobenzaldehyde (3.0 g, 12.1 mmol) was dissolved in DMF (50mL), and added with methyl

2-amino-2-methylpropionate hydrochloride (1.9 g, 12.1 mmol) and DIEA (4.7 g, 36.3 mmol). The system reacted at 15°C for 16 hours after the addition. The reaction solution was poured into water, and extracted with ethyl acetate. An organic phase was concentrated and purified by silica gel column (petroleum ether: ethyl acetate=3: 1) to obtain the compound in title (1.4 g, yield: 33.7%).

**2. Preparation of methyl 2-((2-bromo-4-(hydroxymethyl)-6-nitrophenyl)amino)-2-methylpropionate**

The methyl 2-((2-bromo-4-formyl-6-nitrophenyl)amino)-2-methylpropionate (1.4 g, 4.1 mmol) was dissolved in ethanol (60 mL), and added with sodium borohydride (234 mg, 6.1 mmol) to react at 20°C for 1 hour after the addition, and then the reaction solution was directly concentrated, washed with water, extracted with ethyl acetate and concentrated. The crude product was purified by column chromatography (EA: PE=3: 1) to obtain the product (810 mg, yield 57.4%).

**3. Preparation of 5-bromo-7-(hydroxymethyl)-3,3-dimethyl-3,4-dihydroquinoxaline-2(1H)-one**

The methyl 2-((2-bromo-4-(hydroxymethyl)-6-nitrophenyl)amino)-2-methylpropionate (200 mg, 0.57 mmol), iron powder (319 mg, 5.7 mmol) and ammonium chloride (302 mg, 5.7 mmol) were dissolved in methanol (15 mL), water (3 mL) and tetrahydrofuran (15 mL) to react at 70°C for 3 hours. The reaction solution was filtered with diatomite, the filtrate was concentrated, and diluted with water and ethyl acetate, and an organic phase was concentrated to obtain the compound in title (130 mg, yield: 79.2%).

**4. Preparation of (E)-5-(2-ethoxyvinyl)-7-(hydroxymethyl)-3,3-dimethyl-3,4-dihydroquinoxaline-2(1H)-one**

The 5-bromo-7-(hydroxymethyl)-3,3-dimethyl-3,4-dihydroquinoxaline-2(1H)-one (130 mg, 0.46 mmol), 2-(2-ethoxyvinyl)-4,4,5,5-tetramethyl-1,3,2-dioxaborane (137 mg, 0.69 mmol), Pd(dppf)$Cl_2$ (34 mg, 46.0 μmol) and sodium carbonate (98 mg, 0.92 mmol) were dissolved in dioxane (10 mL) and water (1 mL) to react at 90°C for 1 hour after the addition, and the reaction was finished. The reaction solution was extracted with water and ethyl acetate, and an organic phase was collected, concentrated, and purified by column chromatography (petroleum ether: ethyl acetate=1: 1) to obtain the product (80 mg, yield 63.5%).

**5. Preparation of 8-(chloromethyl)-3,3-dimethyl-1H-pyrrolo[1,2,3-de]quinoxaline-2(3H)-one**

The (E)-5-(2-ethoxyvinyl)-7-(hydroxymethyl)-3,3-dimethyl-3,4-dihydroquinoxaline-2(1H)-one (80 mg, 0.29 mmol) was dissolved in DCM (10 mL), and added with hydrochloric acid/ethyl acetate (0.5 mL) to react at 20°C for 0.5 hour after the addition. After the reaction was finished, the crude product was concentrated, and directly used in the next step of reaction.

**6. Preparation of 5-(4-((3,3-dimethyl-2-oxo-2,3-dihydro-1H-pyrrolo[1,2,3-de]quinoxaline-8-yl)methyl)piperazine-1-yl)-6-fluoro-N-methylpi colinamide**

The 8-(chloromethyl)-3,3-dimethyl-1H-pyrrolo[1,2,3-de]quinoxaline-2(3H)-one (the crude product of the previous step) was dissolved in acetonitrile (10 mL), and added with DIEA (112 mg, 0.87 mmol) and 6-fluoro-N-methyl-5-(piperazine-1-yl)pyridine carboxamide hydrochloride (80 mg, 0.29 mmol) to react at 70°C for 2 hours. After the reaction was finished, the reaction solution was concentrated and purified by silica gel plate (MeoH: DCM=1: 15) to obtain the product (2.5 mg, two-step yield: 1.9%).

Molecular formula: $C_{24}H_{27}FN_6O_2$, Molecular weight: 450.5, LC-MS (M/e): 451.2(M +$H^+$)
$^1$H-NMR(400 MHz, $CDCL_3$) $\delta$: 7.98-8.08 (m, 2H), 7.45-7.52 (m, 1H), 7.28-7.31 (m, 1H), 7.15-7.20 (m, 2H), 6.63 (s, 1H), 6.53 (s, 1H), 3.65 (s, 2H), 3.21-3.27 (m, 4H), 2.98-2.99 (m, 3H), 2.61-2.67 (m, 4H), 1.81 (s, 6H).

**Example 23: Preparation of (R)-6-fluoro-5-(4-((6-fluoro-3-methyl-2-oxo-2,3-dihydro-1H-pyrazolo[1,5,4-de]quinoxaline-8-yl)methyl)piperazine-1-yl)-N-methylpicolinamide (Compound 49-2)**

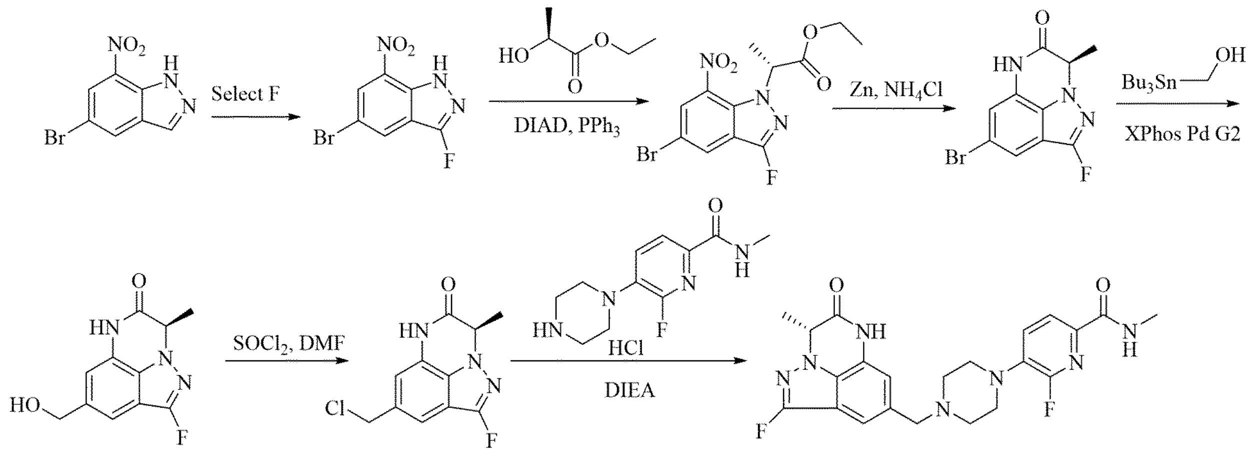


**1. Preparation of 5-bromo-3-fluoro-7-nitro-1H-indazole**

5-bromo-7-nitro-1H-indazole (350 mg, 1.4 mmol) was dissolved in acetonitrile (7 mL), and added with Select F (1.0 g, 2.8 mmol) to react by microwave at 125°C for 1 hour. The reaction solution was concentrated, and the crude product was purified by silica gel column chromatography (n-heptane: ethyl acetate=9: 1) to obtain the product (120 mg, yield 31.9%).

The remaining synthesis steps referred to Example 19.

Characterization data of Compound 49-2:

Molecular formula: $C_{22}H_{23}F_2N_7O_2$, Molecular weight: 455.5, LC-MS (m/z): 456.2($M+H^+$)
$^1$H-NMR(400MHz, $CDCl_3$) δ: 8.21 (s, 1H), 7.99 (d, *J*=7.2 Hz, 1H), 7.50 (d, *J*=4.8 Hz, 1H), 7.32-7.29 (m, 1H), 7.17 (s, 1H), 6.83 (s, 1H), 5.18 (t, *J*=7.0 Hz, 1H), 3.60 (s, 2H), 3.25-3.15 (m, 4H), 3.00 (d, *J*=5.0 Hz, 3H), 2.76-2.65 (m, 4H), 1.86 (d, *J*=7.0 Hz, 3H).

**Example 24: Preparation of (S)-6-fluoro-5-(4-((6-fluoro-3-methyl-2-oxo-2,3-dihydro-1H-pyrazolo[1,5,4-de]quinoxaline-8-yl)methyl)piperazine-1-yl)-N -methylpicolinamide (Compound 49-1)**

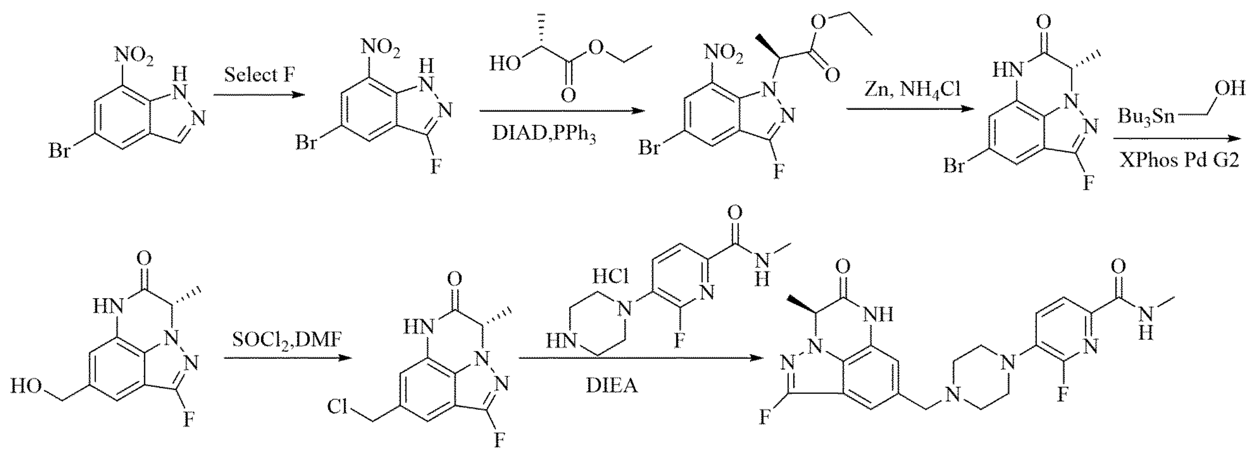


Referring to Example 23, characterization data were as follows:

Molecular formula: $C_{22}H_{23}F_2N_7O_2$, Molecular weight: 455.5, LC-MS (m/z): 456.2($M+H^+$)
$^1$H-NMR(400MHz, $CDCl_3$) δ: 8.01 (s, 1H), 7.99 (d, *J*=7.2 Hz, 1H), 7.50 (d, *J*=4.8 Hz, 1H), 7.32-7.29 (m, 1H), 7.17 (s, 1H), 6.83 (s, 1H),5.18 (t, *J*=7.0 Hz, 1H), 3.60 (s, 2H), 3.25-3.15 (m, 4H), 3.00 (d, *J*=5.0 Hz, 3H), 2.76-2.65 (m, 4H), 1.86 (d, *J*=7.0 Hz, 3H).

**Example 25: Preparation of (R)-6-fluoro-N-methyl-5-(4-((3-methyl-2-oxo-2,3-dihydro-1H-pyrazolo[1,5,4-de]quinoxaline-8-yl)methyl)piperazine-1-yl)pyridine carboxamide (Compound 63-1)**

Referring to Example 23, the 5-bromo-7-nitro-1H-indazole directly reacted with ethyl (S)-2- hydroxypropionate.

Characterization data of Compound 63-1:

Molecular formula: $C_{22}H_{24}FN_7O_2$, Molecular weight: 437.5, LC-MS (m/z): 438.2(M+H$^+$)

$^1$H-NMR(400MHz, $CDCl_3$) δ: 8.22 (s, 1H), 8.00-7.94 (m, 2H), 7.50 (d, *J*=4.8 Hz, 1H), 7.32-7.24 (m, 2H), 6.79 (s, 1H), 5.37 (t, *J*=7.0 Hz, 1H), 3.62 (s, 2H), 3.27-3.15 (m, 4H), 2.99 (d, *J*=5.0 Hz, 3H), 2.70-2.60 (m, 4H), 1.94 (d, *J*=7.0 Hz, 3H).

**Example 26: Preparation of (S)-6-fluoro-N-methyl-5-(4-((3-methyl-2-oxo-2,3-dihydro-1H-pyrazolo[1,5,4-de]quinoxaline-8-yl)methyl)piperazine-1-yl)pyridine carboxamide (Compound 63-2)**

Referring to Examples 23 and 25, the 5-bromo-7-nitro-1H-indazole directly reacted with ethyl (R)-2-hydroxypropionate.

Molecular formula: $C_{22}H_{24}FN_7O_2$, Molecular weight: 437.5, LC-MS (m/z): 438.2(M+H$^+$)

$^1$H-NMR(400MHz, $CDCl_3$) δ: 8.08 (s, 1H), 8.00-7.94 (m, 2H), 7.50 (d, *J*=4.8 Hz, 1H), 7.32-7.24 (m, 2H), 6.79 (s, 1H), 5.37 (t, *J*=7.0 Hz, 1H), 3.62 (s, 2H), 3.27-3.15 (m, 4H), 2.99 (d, *J*=5.0 Hz, 3H), 2.70-2.60 (m, 4H), 1.94 (d, *J*=7.0 Hz, 3H).

**Example 27: Preparation of (R)-6-fluoro-5-(4-((9-fluoro-3-methyl-2-oxo-2,3-dihydro-1H-pyrazolo[1,5,4-de]quinoxaline-8-yl)methyl)piperazine-1-yl)-N-methylpicolinamide (Compound 64-1)**

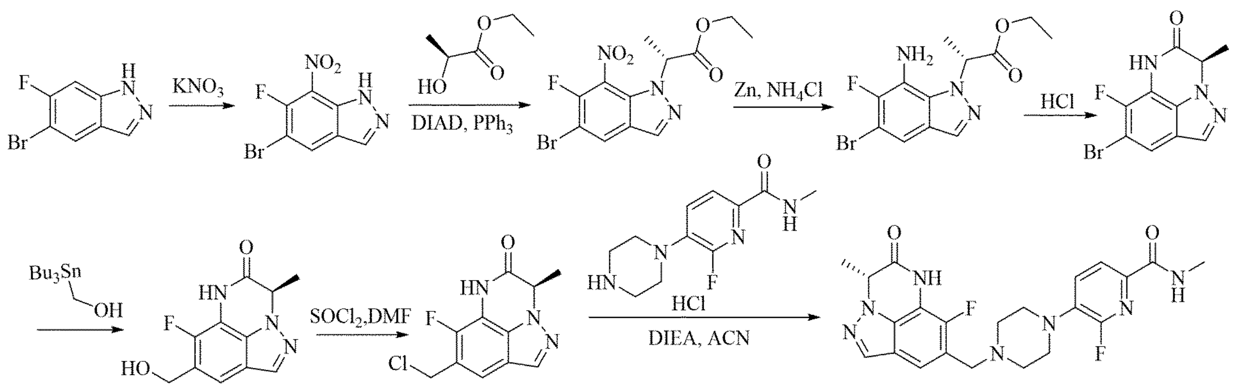


**1. Preparation of 5-bromo-6-fluoro-7-nitro-1H-indazole**

5-bromo-6-fluoro-1H-indazole (2.0 g, 9.3 mmol) was dissolved in concentrated sulfuric acid (50 mL), cooled to 0°C, and added with potassium nitrate (1.1 g, 10.9 mmol) to react at 15°C for 16 hours. The reaction solution was poured into ice water, extracted with ethyl acetate, and purified by silica gel column chromatography (n-heptane: ethyl acetate=2: 1), and the crude product was purified by C18 column (methanol: water=3: 2) to obtain the product (1.2 g, yield 49.6%).

**2. Preparation of ethyl (R)-2-(5-bromo-6-fluoro-7-nitro-1H-indazole-1-yl)propionate**

The 5-bromo-6-fluoro-7-nitro-1H-indazole (500 mg, 1.9 mmol), ethyl (S)-2-hydroxypropionate (340 mg, 2.9 mmol) and triphenylphosphine (760 mg, 2.9 mmol) were dissolved in tetrahydrofuran (20 mL), and added with DIAD (580 mg, 2.9 mmol) to react at 15°C for 1 hour. The reaction solution was concentrated, and the crude product was purified by silica gel column chromatography (n-heptane: ethyl acetate=10: 1) to obtain the product (100 mg, yield 14.4%).

**3. Preparation of ethyl (R)-2-(7-amino-5-bromo-6-fluoro-1H-indazole-1-yl)propionate**

The ethyl (R)-2-(5-bromo-6-fluoro-7-nitro-1H-indazole-1-yl)propionate (100 mg, 0.28 mmol) was dissolved in tetrahydrofuran/methanol/water (8/8/2 mL), and added with zinc powder (272 mg, 4.2 mmol) and ammonium chloride (297 mg, 5.6 mmol) to react at 70°C for 3 hours. The reaction solution was filtered to remove solids, and the filtrate was directly

used in the next step.

**4. Preparation of (R)-8-bromo-9-fluoro-3-methyl-1H-pyrazolo[1,5,4-de]quinoxaline-2(3H)-one**

The filtrate of the previous step was added with an ethyl acetate solution of hydrogen chloride (4 M, 1 mL) to react at 15°C for 1 hour. The reaction solution was concentrated, added with a saturated sodium bicarbonate solution to adjust a pH value to be 8-9, and extracted with water and ethyl acetate. An organic phase was dried and concentrated to obtain the product (90 mg).

**5. Preparation of (R)-9-fluoro-8-(hydroxymethyl)-3-methyl-1H-pyrazolo[1,5,4-de]quinoxaline-2(3H)-one**

The (R)-8-bromo-9-fluoro-3-methyl-1H-pyrazolo[1,5,4-de]quinoxaline-2(3H)-one (90 mg, 0.32 mmol) and (tributyltin)methanol (122 mg, 0.38 mmol) were dissolved in 1,4-dioxane (10 mL), and added with Xphos Pd G2 (49 mg, 0.062 mmol) to react at 80°C for 2 hours. The reaction solution was concentrated, and the crude product was purified by silica gel column chromatography (dichloromethane: methanol=20: 1) to obtain the product (45 mg, yield 60.4%).

**6. Preparation of (R)-8-(chloromethyl)-9-fluoro-3-methyl-1H-pyrazolo[1,5,4-de]quinoxaline-2(3H)-one**

The (R)-9-fluoro-8-(hydroxymethyl)-3-methyl-1H-pyrazolo[1,5,4-de]quinoxaline-2(3H)-one (45 mg, 0.19 mmol) and N,N-dimethylformamide (45 mg, 0.62 mmol) were dissolved in dichloromethane (13 mL), and added with thionyl chloride (182 mg, 1.5 mmol) to react at 20°C for 1 hour. The reaction solution was concentrated, and the crude product was directly used in the next step.

**7. Preparation of (R)-6-fluoro-5-(4-((9-fluoro-3-methyl-2-oxo-2,3-dihydro-1H-pyrazolo[1,5,4-de]quinoxaline-8-yl) methyl)piperazine-1-yl)-N-methylpicolinamide**

The (R)-8-(chloromethyl)-9-fluoro-3-methyl-1H-pyrazolo[1,5,4-de]quinoxaline-2(3H)-one (the crude product of the previous step) and 6-fluoro-N-methyl-5-(piperazine-1-yl)picolinamide hydrochloride (68 mg, 0.25 mmol) were dissolved in acetonitrile (15 mL), and added with DIEA (160 mg, 1.2 mmol) to react at 80°C for 3 hours. The reaction solution was concentrated, and the crude product was purified by silica gel column chromatography (dichloromethane: methanol=20: 1) to obtain the product (35 mg, two-step yield 40.2%).

Molecular formula: $C_{22}H_{23}F_2N_7O_2$, Molecular weight: 455.5, LC-MS (m/z): 456.2(M+H$^+$)
$^1$H-NMR(400MHz, $CDCl_3$) δ: 8.00-7.95 (m, 2H), 7.91 (s, 1H), 7.48 (d, *J*=4.8 Hz, 1H), 7.30-7.25 (m, 2H), 5.34 (t, *J*=7.0 Hz, 1H), 3.70 (s, 2H), 3.23-3.21 (m, 4H), 2.99 (d, *J*=5.0 Hz, 3H), 2.72-2.68 (m, 4H), 1.93 (d, *J*=7.0 Hz, 3H)

**Example 28: Preparation of (S)-6-fluoro-5-(4-((9-fluoro-3-methyl-2-oxo-2,3-dihydro-1H-pyrazolo[1,5,4-de]quinoxaline-8-yl)methyl)piperazine-1-yl)-N -methylpicolinamide (Compound 64-2)**

Referring to Example 27, the chiral intermediate ethyl (S)-2-hydroxypropionate was replaced by ethyl (R)-2-hydroxypropionate.
Characterization data:

Molecular formula: $C_{22}H_{23}F_2N_7O_2$, Molecular weight: 455.5, LC-MS (m/z): 456.2(M+H$^+$)
$^1$H-NMR(400MHz, $CDCl_3$) δ: 8.00-7.95 (m, 2H), 7.93 (s, 1H), 7.49 (d, *J*=4.8 Hz, 1H), 7.31-7.25 (m, 2H), 5.34 (t, *J*=7.0 Hz, 1H), 3.70 (s, 2H), 3.23-3.21 (m, 4H), 2.99 (d, *J*=5.0 Hz, 3H), 2.72-2.68 (m, 4H), 1.93 (d, *J*=7.0 Hz, 3H).

**Example 29: Preparation of (R)-6-fluoro-N-methyl-5-(4-((4-methyl-5-oxo-5,6-dihydro-4H-[1,2,3]triazolo[1,5,4-de] quinoxaline-8-yl)methyl)piperazine-1-yl)pyridine carboxamide (Compound 68-1)**

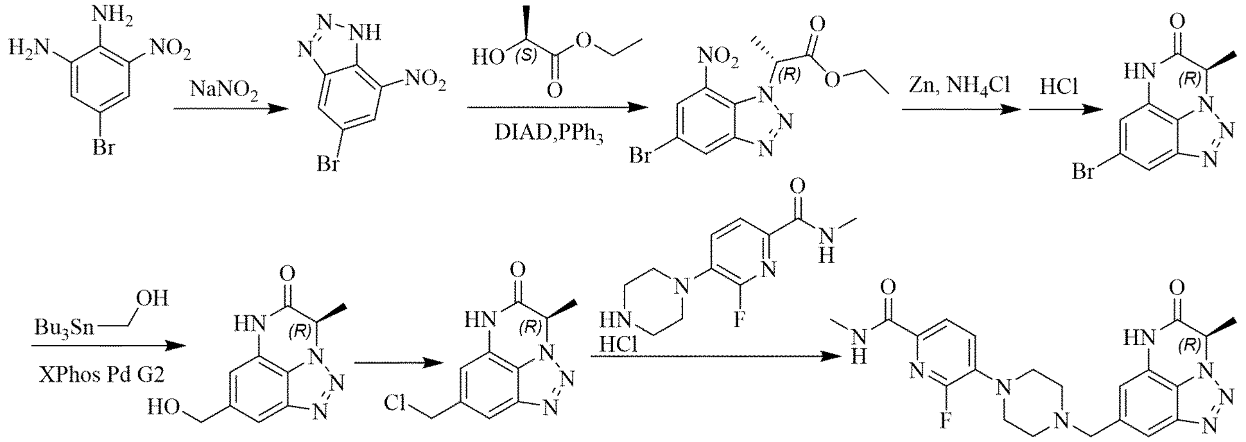


**1. Preparation of 5-bromo-7-nitro-1H-benzo[d] [1,2,3]triazole**

5-bromo-3-nitrobenzene-1,2-diamine (3.6 g, 15.5 mmol) was dissolved in acetic acid (80 mL), added with sodium nitrite (1.18 g, 17.1 mmol), and heated to 60°C to react for 2 hours. The reaction solution was poured into water (200 mL) and filtered, and the filter cake was washed with water (200 mL) and dried in vacuum at 50°C for 5 hours to obtain the product (3.6 g, yield 95.5%).

The remaining steps referred to Example 27, and characterization data of Compound 68-1 were as follows:

Molecular formula: $C_{21}H_{23}FN_8O_2$, Molecular weight: 438.5, LC-MS (m/z): 439.2($M+H^+$)
$^1$H-NMR(400MHz, $CDCl_3$) δ : 8.85 (s, 1H), 7.99 (d, *J*=7.8 Hz, 1H), 7.55 (s, 1H), 7.52 (d, *J*=8.6 Hz, 1H), 7.27-7.33 (m, 1H), 6.95 (s, 1H), 5.59-5.64 (m, 1H), 3.67 (s, 2H), 3.21 (s, 4H), 3.00 (d, *J*=4.5 Hz, 3H), 2.60-2.65 (m, 4H), 2.08 (d, *J*=6.9 Hz, 3H).

**Example 30: Preparation of (S)-6-fluoro-N-methyl-5-(4-((4-methyl-5-oxo-5,6-dihydro-4H-[1,2,3]triazolo[1,5,4-de] quinoxaline-8-yl)methyl)piperazine-1-yl)pyridine carboxamide (Compound 68-2)**

Referring to Examples 29 and 27, the chiral intermediate was replaced by ethyl (R)-2-hydroxypropionate.

Characterization data:

Molecular formula: $C_{21}H_{23}FN_8O_2$, Molecular weight: 438.5, LC-MS (m/z): 439.2($M+H^+$)
$^1$H-NMR(400MHz, $CDCl_3$) δ : 8.53 (s, 1H), 7.99 (d, *J*=8.2 Hz, 1H), 7.55 (s, 1H), 7.51 (d, *J*=3.7 Hz, 1H), 7.28-7.31 (m, 1H), 6.94 (s, 1H), 5.59-5.64 (m, 1H), 3.67 (s, 2H), 3.21 (s, 4H), 3.00 (d, *J*=5.0 Hz, 3H), 2.66 (s, 4H), 2.08 (d, *J*=7.0 Hz, 3H).

The following compounds were prepared by the same or similar methods of the above preparation examples:

| Compound | LC-MS (M/e, $M+H^+$) | Compound | LC-MS (M/e, $M+H^+$) | Compound | LC-MS (M/e, $M+H^+$) | Compound | LC-MS (M/e, $M+H^+$) |
|---|---|---|---|---|---|---|---|
| 2 | 467.2 | 4 | 469.2 | 5 | 455.2 | 7 | 468.2 |
| 9 | 424.2 | 10 | 424.2 | 12 | 439.2 | 13 | 434.2 |
| 14 | 457.2 | 16 | 435.2 | 17 | 441.2 | 18 | 441.2 |
| 19 | 442.2 | 20 | 442.2 | 21 | 442.2 | 22 | 423.2 |
| 23 | 467.2 | 29 | 438.2 | 30 | 438.2 | 33 | 469.2 |
| 37 | 437.2 | 39 | 456.2 | 40 | 469.2 | 45 | 449.2 |
| 47 | 452.2 | 48 | 470.2 | 50 | 474.2 | 51 | 438.2 |
| 52 | 456.2 | 53 | 456.2 | 54 | 474.2 | 55 | 452.2 |
| 56 | 470.2 | 57 | 438.2 | 58 | 456.2 | 59 | 456.2 |

(continued)

| Compound | LC-MS (M/e, M+H$^+$) | Compound | LC-MS (M/e, M+H$^+$) | Compound | LC-MS (M/e, M+H$^+$) | Compound | LC-MS (M/e, M+H$^+$) |
|---|---|---|---|---|---|---|---|
| 60 | 474.2 | 61 | 452.2 | 62 | 470.2 | 65-1 | 456.2 |
| 65-2 | 456.2 | 66-1 | 452.2 | 66-2 | 452.2 | 67-1 | 488.2 |
| 67-2 | 488.2 | 69 | 437.2 | | | | |

**Experimental scheme**

Exemplary experimental schemes for some compounds of the present application are provided below to demonstrate the beneficial activities and technical effects of the compounds of the present application. However, it should be understood that the following experimental schemes are only examples of the contents of the present application, and are not intended to limit the scope of the present application.

**Experimental Example 1 In-vitro cytological inhibitory activities of compounds of the present application**

Test sample: the compounds of the present application, structural formulae and preparation methods of the compounds were shown in the examples.

Reagents and cell lines used in the experiment:

DMEM: dulbecco’s modified eagle medium modified basal medium;
ITS-G: insulin-transferrin-selenium additive;
CTG: CellTiter-Glo cell viability assay kit;
Glutathione: GSH
FBS: fetal bovine serum;
MDA-MB-436: BRCA1 mutated human breast cancer cells.

Experimental method (CelltiterGlo assay)

1. Cell preparation

1.1 Cell culture:

The MDA-MB-436 cells were adherent cells, and the culture solution was DMEM+10%FBS+1% ITS-G+16 μg/ml glutathione.

1.2 Preparation of cell suspension:

Cells in the logarithmic growth phase were collected and counted by a platelet counter. The cell viability was detected by a trypan blue exclusion method, and the cell viability was kept over 90%. The cell concentration was adjusted to be in a proper range, and 90 μL of cell suspension was added into a 96-well plate respectively.

Table 1. Number of inoculated cells

| Cell line | Number of inoculated cells |
|---|---|
| MDA-MB-436 | 3000 cells/well |

2. Preparation of test compounds

2.1 DMSO stock solutions of the test compounds were prepared, and the concentration of the stock solutions of the test compounds was 10 mM.

2.2 Preparation of working stock solutions of test compounds

The stock solutions of the test compounds were diluted by 10 times from 10 mM to 1 mM with DMSO, and then continuously gradiently diluted by 3 times with DMSO into a total of 9 concentrations. Then, 2 μL of DMSO gradiently diluted compounds were respectively added into 198 μL of culture solution (DMEM+10%FBS+1% ITS-G+16 μg/ml glutathione) to give the working stock solutions of the test compounds (the concentration of the working stock solutions of the compounds was 10 times of the final concentration, and the maximum concentration of the working stock solutions was 10 μM).

2.3 Compound treatment

10 μL of compound working stock solution was added into each well in a 96-well plate inoculated with cells (diluted by 10 times, the final concentration of DMSO was 0.1%).

The final concentrations of the test compounds were: 1000.00 nM, 333.33 nM, 111.11 nM, 37.04 nM, 12.35 nM, 4.11 nM, 1.37 nM, 0.46 nM and 0.15 nM.

2.4 Setting of control well

Solvent control: 0.1% DMSO (2 μL of DMSO was diluted with 198 μL of culture solution, and then 10 μL of the diluted solution was added into the well plate).

Blank control: the 96-well plate was detected and read 0 hour after the addition.

2.5 The 96-well plate was placed in a 5% $CO_2$ cell incubator at 37°C to culture for 7 days.

3. Detection

A CTG reagent was thawed and the 96-well plate was equilibrated to room temperature for 30 minutes, 60 μL of reagent (Celititer Glo assay kit) was added into each well and shaken by a shaker for 2 minutes to mix uniformly (in the dark), and the mixture was incubated at room temperature for 20 minutes (in the dark). Light signal values were read by a multi-functional microplate reader.

4. Data processing

1) Inhibition (%)=(read number of DMSO solvent control well-read number of test compound)/(read number of DMSO solvent control well-read number of blank control well)×100%,
2) A graph was made to obtain a curve and $IC_{50}$.

Experimental result and conclusion

Table 2. In-vitro cytological activities of compounds of the present application

| Test compound | MDA-MB-436 ($IC_{50}$, nM) | Test compound | MDA-MB-436 ($IC_{50}$, nM) |
|---|---|---|---|
| Compound 3 | 13.3 | Compound 8 | 129 |
| Compound 11 | 21.5 | Compound 25 | 15.8 |
| Compound 26 | 12.5 | Compound 28 | 6.1 |
| Compound 31 | 2.2 | Compound 34 | 13.6 |
| Compound 35 | 46.5 | Compound 36 | 11.1 |
| Compound 41 | 7.7 | Compound 44 | 8.6 |
| Compound 27 | 4.9 | Compound 6 | 2.0 |
| Compound 38 | 15.2 | Compound 41-1 | 5.9 |
| Compound 41-2 | 5.5 | Compound 46 | 9.2 |
| Compound 49-1 | 3.6 | Compound 49-2 | 2.9 |
| Compound 63-1 | 4.5 | Compound 63-2 | 7.3 |
| Compound 64-1 | 3.2 | Compound 64-2 | 14.1 |

(continued)

| Test compound | MDA-MB-436 ($IC_{50}$, nM) | Test compound | MDA-MB-436 ($IC_{50}$, nM) |
|---|---|---|---|
| Compound 29-1 | 2.6 | Compound 29-2 | 3.3 |
| Compound 38-1 | 25.9 | Compound 38-2 | 14.9 |
| Compound 68-1 | 16.9 | Compound 68-2 | 14.7 |

It can be seen from Table 2 that the compounds of the present application can effectively inhibit the proliferation of MDA-MB-436 cells, indicating that the compounds of the present application can significantly inhibit the growth of cells with DNA repair ability deficiency, and have the potential of clinical application in treating cancerous diseases with DNA repair deficiency.

**Experimental Example 2 In-vitro enzymatic activities of compounds of the present application**

Test sample: the compounds of the present application, structural formulae and preparation methods of the compounds were shown in the examples.

Experimental reagents:

| Reagent | Supplier |
|---|---|
| PARP1 | BPS |
| PARP2 | BPS |
| Histone | Active Motif |
| Activited DNA | Genscript |
| anti-rabbit IgG, HRP-linked Antibody | CST |
| anti-Poly/Mono-ADP Ribose (E6F6A) Rabbit mAb | CST |
| SuperSignal ELISA Femto Substrate | THERMO PIERCE |
| Biotin-NAD+ | R&D |
| Strep-HRP | Thermo Pierce |
| QuantaRed enhanced chemifluorescent HRP Substrate | Thermo Pierce |

Experimental materials:

| Material | Supplier |
|---|---|
| 384-Well plate | Perkin Elmer |

Experimental method:

1. Preparation of histone-coated 384-well plate
5 μL of Histone solution was added into each well of 384-well plate, and incubated at 4°C overnight.
2. The histone-coated 384-well plate was rinsed with PBST buffer for 3 times, and incubated with 50 μL of blocking buffer at room temperature for 1 hour. The well plate was washed with PBST buffer for 3 times.
3. Compound dilution

1) The compounds of the present application were prepared to 20 mM with DMSO to serve as the test stock

solutions.

2) The stock solutions of the compounds of the present application were gradiently diluted by 4 times into 10 concentrations, with the maximum concentration of 20 mM.

3) 50 nL of diluted compound solution was added into each well of 96-well plate, then 19.95 μL of working solution was added into each well, and the mixture was centrifugally shaken at 1000 rpm for 1 minute. 5 μL of compound was transferred to the treated 384-well plate.

4. Enzyme reaction experiment

1) 10 μL of LDNA solution was added into a negative control well, 10 μL of PARP1&DNA (or PARP2&DNA) mixed solution was added into wells excluding the control well, and then 10 μL of NAD+ reagent was added into each well, and incubated at 25°C for 60 minutes.

2) The 384-well plate was washed with PBST buffer for 3 times.

3) Finial concentrations of the test compounds were 1000 nM, 250 nM, 62.5 nM, 15.6 nM, 3.9 nM, 0.98 nM, 0.24 nM, 0.061 nM, 0.015 nM and 0.0038 nM.

5. Detection

1) 20 μL of anti-Poly/Mono-ADP Ribose Rabbit mAb was added into each well, and incubated at room temperature for 1.5 hours, and then the 384-well plate was washed with PBST buffer for 3 times.

2) An anti-rabbit IgG and an HRP-linked Antibody were diluted by 2000 times with blocking buffer, 200 μL of diluted antibody was added into each well and incubated at room temperature for 1 hour, and the 384-well plate was washed with PBST buffer for 3 times.

3) 25 μL of Supersignal ELISA femto substrate was added into each well for chemiluminescence detection.

6. Data analysis

The inhibition (% inh) was calculated by the following formula:

$$\text{inhibition}(\%) = 100\% \times \frac{Max - Signal}{Max - Min}$$

where, Max represented: a luminous signal intensity of a positive control well without adding the compound;
Min represented: a luminous signal intensity of a negative control well without adding the enzyme; and
Signal represented: a luminous signal intensity of the compound of the test sample.

The $IC_{50}$ was calculated by the following formula:

$$\text{Y} = \text{Bottom} + \frac{\text{Top} - \text{Bottom}}{1 + (\text{IC50} \div X) \times HillSlope}$$

where, Y represented: %inhibition;
X represented: the concentration of the compound;
Top represented: the maximum inhibition; and Bottom represented: the minimum inhibition; and
HillSlope represented: an absolute value of the maximum slope of curve (i.e. a midpoint of the curve).

Experimental results:

Table 3 Enzymatic inhibitory activities of compounds of the present application

| Compound | PARP1 $IC_{50}$ (nM) | PARP2 $IC_{50}$ (nM) | Compound | PARP1 $IC_{50}$ (nM) | PARP2 $IC_{50}$ (nM) |
|---|---|---|---|---|---|
| Compound 3 | ++++ | >1000 | Compound 25 | +++++ | ++ |
| Compound 26 | ++++ | + | Compound 27 | ++++ | + |
| Compound 28 | ++++ | + | Compound 31 | +++++ | ++ |

(continued)

| Compound | PARP1 $IC_{50}$ (nM) | PARP2 $IC_{50}$ (nM) | Compound | PARP1 $IC_{50}$ (nM) | PARP2 $IC_{50}$ (nM) |
|---|---|---|---|---|---|
| Compound 34 | ++++ | + | Compound 35 | +++++ | >1000 |
| Compound 36 | ++++ | >1000 | Compound 38 | ++++ | + |
| Compound 41-1 | +++++ | + | Compound 41-2 | +++++ | + |
| Compound 46 | +++ | + | Compound 49-1 | ++++ | + |
| Compound 49-2 | +++++ | + | Compound 63-1 | +++++ | + |
| Compound 63-2 | +++++ | + | Compound 64-1 | +++++ | + |
| Compound 29-1 | ++++ | >1000 | Compound 29-2 | ++++ | >1000 |
| Compound 38-1 | ++++ | + | Compound 38-2 | ++++ | + |
| Note: +: 100-1000 nM; ++: 10-100 nM; +++: 3-10 nM; ++++: 1-3 nM; +++++: smaller than 1 nM. | | | | | |

Experimental conclusion:

It can be seen from Table 3 that the compounds of the present application have good inhibitory activity and selectivity for PARP1, and are effective PARP1 inhibitors with high selectivity.

**Claims**

**1.** A compound as shown in formula (I), a pharmaceutically acceptable salt thereof or a stereoisomer thereof,

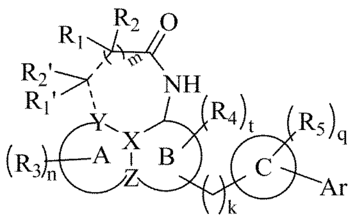

(I)

wherein,

X, Y and Z are each independently selected from N, C and CH;
ring A and ring B are each independently selected from 5-7 membered cycloalkyl, 5-7 membered heterocyclyl, phenyl and 5-7 membered heteroaryl;
ring C is selected from 3-11 membered cycloalkyl, 3-11 membered heterocyclyl, 6-11 membered aryl and 5-11 membered heteroaryl;
Ar is selected from 3-11 membered cycloalkyl, 3-11 membered heterocyclyl, 6-11 membered aryl and 5-11 membered heteroaryl, each of which is optionally substituted by 1-3 Q substituents; each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p$-3-10 membered cycloalkyl, $-(CH_2)_p$-3-10 membered heterocycloalkyl, $-(CH_2)_p-N(R^a)(R^b)$, $-(CH_2)_p-O-R^a$, $-(CH_2)_p-P(O)(R^a)(R^b)$, $-(CH_2)_p-S(O)(R^a)$, $-(CH_2)_p-S(O)_2(R^a)$, $-(CH_2)_p-C(O)(R^a)$, $-(CH_2)_p-C(O)O(R^a)$, $-(CH_2)_p-O-C(O)(R^a)$, $-(CH_2)_p-C(O)N(R^a)(R^b)$ and $-(CH_2)_p-N(R^b)-C(O)(R^a)$;
each $R_1$ and each $R_2$ are independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl; or $R_1$, $R_2$ and a carbon atom linked thereto form 3-7 membered cycloalkyl or 3-7 membered heterocyclyl;
$R_1$' and $R_2$' are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$

alkoxy-$C_{1-6}$ alkyl;
each $R_3$, each $R_4$ and each $R_5$ are independently selected from H, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;
$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy-$C_{1-6}$ alkyl, 3-10 membered cycloalkyl and 3-10 membered heterocyclyl;
m is 0, 1 or 2, and when m is 2, two adjacent ring carbon atoms are linked by a single bond or a double bond;
n and t are each independently selected from 0, 1, 2 and 3;
p and k are each independently selected from 0, 1 and 2;
q is 0, 1, 2, 3 or 4; and
--- represents a single bond or a double bond.

**2.** The compound, the pharmaceutically acceptable salt thereof or the stereoisomer thereof according to claim 1, wherein, ring A and ring B are each independently selected from 5-6 membered cycloalkyl, 5-6 membered heterocyclyl, phenyl and 5-6 membered heteroaryl;

optionally, ring A and ring B are each independently selected from cyclopentyl, cyclohexyl, cyclopentenyl, cyclohexenyl, phenyl, pyrrolyl, pyrazolyl, imidazolyl, pyridyl, pyrimidinyl, pyrazinyl, pyridazinyl, dihydropyrrolyl, pyrrolidinyl, dihydropyrazolyl, pyrazolidinyl, dihydroimidazolyl, imidazolidinyl, dihydropyridyl, tetrahydropyridyl, piperidinyl, dihydropyrimidinyl, tetrahydropyrimidinyl, hexahydropyrimidinyl, dihydropyrazinyl, tetrahydropyrazinyl, piperazinyl, dihydropyridazinyl, tetrahydropyridazinyl, hexahydropyridazinyl, furyl, dihydrofuryl, tetrahydrofuryl, pyranyl, dihydropyranyl, tetrahydropyranyl, thiazolyl, oxazolyl, triazolyl, dihydrothiazolyl, tetrahydrothiazolyl, dihydrooxazolyl and tetrahydrooxazolyl; and
more preferably, ring A, ring B, and X, Y and Z together form the following group:

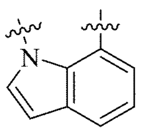, 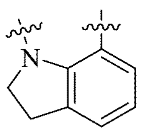, 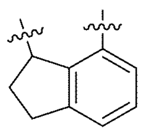, 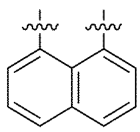, 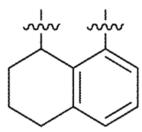, 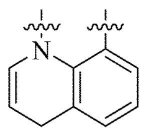, 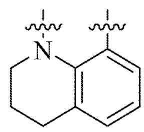,

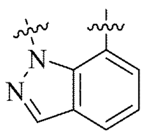, 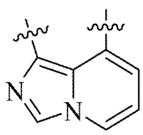, 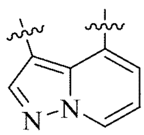, 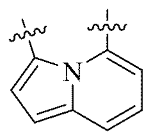, 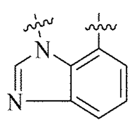 or 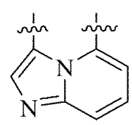.

**3.** The compound, the pharmaceutically acceptable salt thereof or the stereoisomer thereof according to claim 1, wherein, ring C is selected from 5-6 membered cycloalkyl, 5-6 membered heterocyclyl, phenyl, 5-6 membered heteroaryl, 8-11 membered fused cycloalkyl, 8-11 membered spiro cycloalkyl, 7-9 membered bridged cycloalkyl, 8-11 membered fused heterocyclyl, 8-11 membered spiro heterocyclyl and 7-9 membered bridged heterocyclyl;

preferably, ring C is selected from 5-6 membered cycloalkyl, 5-6 membered heterocyclyl, 7-9 membered bridged cycloalkyl and 7-9 membered bridged heterocyclyl; and
more preferably, ring C is selected from the following group:

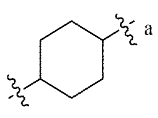, 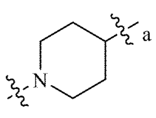, 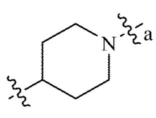, 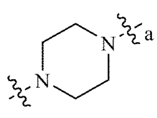, 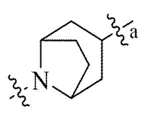, 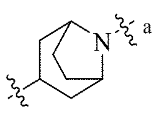,

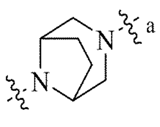, 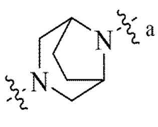, 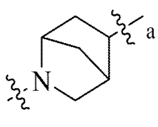, 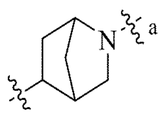, 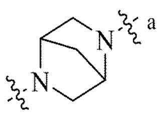 or 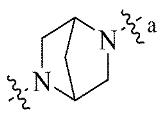,

and a-terminal is linked to Ar.

**4.** The compound, the pharmaceutically acceptable salt thereof or the stereoisomer thereof according to any one of claims 1 to 3, wherein,

Ar is selected from 5-6 membered cycloalkyl, 5-6 membered heterocyclyl, phenyl and 5-6 membered heteroaryl optionally substituted by 1-2 Q groups;
preferably, Ar is selected from phenyl and 5-6 membered heteroaryl optionally substituted by 1-2 Q groups;
more preferably,Ar is selected from phenyl, pyridyl, pyrimidinyl, pyrazinyl and pyridazinyl optionally substituted by 1-2 Q groups;
each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p-N(R^a)(R^b)$, $-(CH_2)_p-O-R^a$, $-(CH_2)_p-P(O)(R^a)(R^b)$, $-(CH_2)_p-S(O)(R^a)$, $-(CH_2)_p-S(O)_2(R^a)$, $-(CH_2)_p-C(O)(R^a)$, $-(CH_2)_p-C(O)O(R^a)$, $-(CH_2)_p-O-C(O)(R^a)$, $-(CH_2)_p-C(O)N(R^a)(R^b)$ and $-(CH_2)_p-N(R^b)-C(O)(R^a)$; and
$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy-$C_{1-6}$ alkyl, cyclopropyl and cyclobutyl.

**5.** The compound, the pharmaceutically acceptable salt thereof or the stereoisomer thereof according to claim 1, wherein, X, Y and Z are each independently selected from N and C;
ring A, ring B, and X, Y and Z together form the following group:

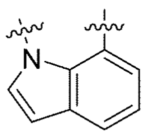,
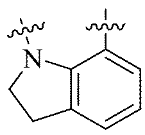,
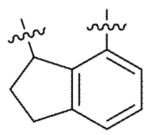,
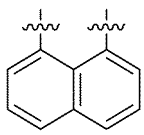,
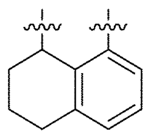,
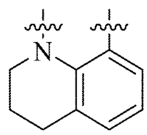,
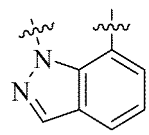,
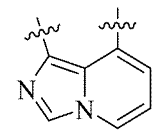,
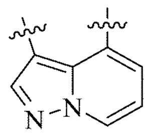,
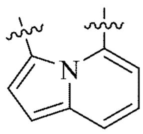,
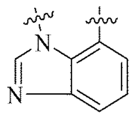 or
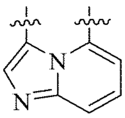;

ring C is selected from the following group:

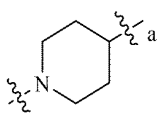,
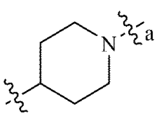 or
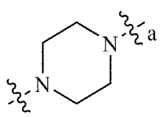,

and a-terminal is linked to Ar;

Ar is pyridyl optionally substituted by 1-2 Q groups; each Q is independently selected from H, halogen, hydroxyl, amino, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl, $C_{1-6}$ alkoxy, halo $C_{1-6}$ alkoxy, hydroxyl $C_{1-6}$ alkoxy, amino $C_{1-6}$ alkoxy, $-(CH_2)_p-C(O)N(R^a)(R^b)$ and $-(CH_2)_p-N(R^b)-C(O)(R^a)$;
$R_1$ and $R_2$ are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl; or $R_1$, $R_2$ and a carbon atom linked thereto form 3-4 membered cycloalkyl or 3-4 membered heterocyclyl;
$R_1$' and $R_2$' are each independently selected from hydrogen, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;
each $R_3$, each $R_4$ and each $R_5$ are independently selected from H, halogen, hydroxyl, amino, cyano, $C_{1-6}$ alkyl, halo $C_{1-6}$ alkyl, hydroxyl $C_{1-6}$ alkyl, amino $C_{1-6}$ alkyl, cyano $C_{1-6}$ alkyl, $C_{1-6}$ alkoxy, $C_{1-6}$ alkylthio, halo $C_{1-6}$ alkoxy, halo $C_{1-6}$ alkylthio, hydroxyl $C_{1-6}$ alkoxy, amino $C_{1-6}$ alkoxy, hydroxyl $C_{1-6}$ alkylthio, amino $C_{1-6}$ alkylthio and $C_{1-6}$ alkoxy-$C_{1-6}$ alkyl;
$R^a$ and $R^b$ are each independently selected from hydrogen, $C_{1-6}$ alkyl, halo $C_{1-6}$ alkyl, hydroxyl $C_{1-6}$ alkyl, amino $C_{1-6}$ alkyl, $C_{1-6}$ alkoxy-$C_{1-6}$ alkyl and cyclopropyl;

m is 0 or 1;
n and t are each independently selected from 0, 1, 2 and 3;
p and k are each independently selected from 0, 1 and 2;
q is 0, 1, 2, 3 or 4; and
--- represents a single bond or a double bond.

**6.** The compound, the pharmaceutically acceptable salt thereof or the stereoisomer thereof according to claim 5, wherein,

ring C is

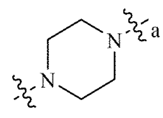

,

a-terminal is linked to Ar and the other terminal is linked to a para-position of X in ring B through alkylene;
Ar is pyridyl optionally substituted by 1-2 Q groups; each Q is independently selected from H, fluorine, chlorine, hydroxyl, amino, $C_{1-4}$ alkyl, fluoro $C_{1-4}$ alkyl, hydroxyl $C_{1-4}$ alkyl, amino $C_{1-4}$ alkyl, $C_{1-4}$ alkoxy-$C_{1-4}$ alkyl, $C_{1-4}$ alkoxy, fluoro $C_{1-4}$ alkoxy, hydroxyl $C_{1-4}$ alkoxy, amino $C_{1-4}$ alkoxy and -C(O)N($R^a$)($R^b$);
$R_1$ and $R_2$ are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl; or $R_1$, $R_2$ and a carbon atom linked thereto form cyclopropyl or cyclobutyl;
$R_1$' and $R_2$' are each independently selected from hydrogen, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl;
each $R_3$, each $R_4$ and each $R_5$ are independently selected from H, fluorine, chlorine, bromine, hydroxyl, amino, cyano, $C_{1-4}$ alkyl, halo $C_{1-4}$ alkyl, hydroxyl $C_{1-4}$ alkyl, amino $C_{1-4}$ alkyl, cyano $C_{1-4}$ alkyl, $C_{1-4}$ alkoxy, $C_{1-4}$ alkylthio, halo $C_{1-4}$ alkoxy, halo $C_{1-4}$ alkylthio, hydroxyl $C_{1-4}$ alkoxy, amino $C_{1-4}$ alkoxy, hydroxyl $C_{1-4}$ alkylthio, amino $C_{1-4}$ alkylthio and $C_{1-4}$ alkoxy-$C_{1-4}$ alkyl;
$R^a$ and $R^b$ are each independently selected from hydrogen, methyl, ethyl, isopropyl and cyclopropyl;
n and t are each independently selected from 0, 1 and 2;
k is 1; and
q is 0 or 1.

**7.** The compound, the pharmaceutically acceptable salt thereof or the stereoisomer thereof according to claim 1 selected from the following compounds:

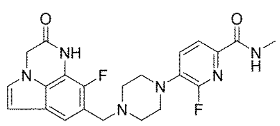

1

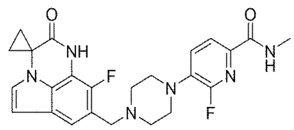

2

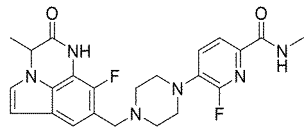

3

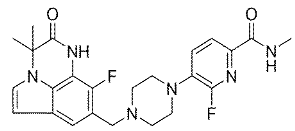

4

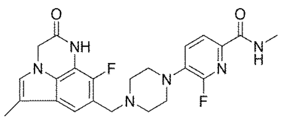

5

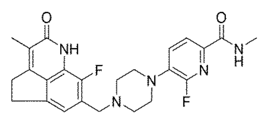

6

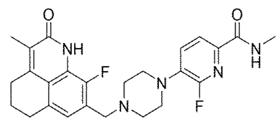

7

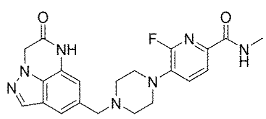

8

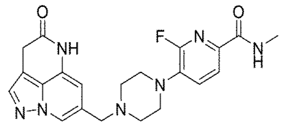
9

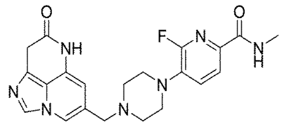
10

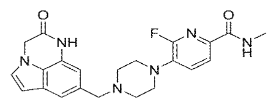
11

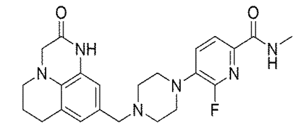
12

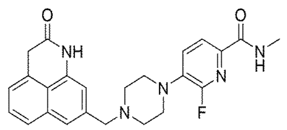
13

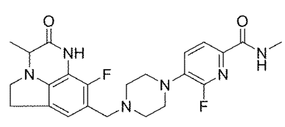
14

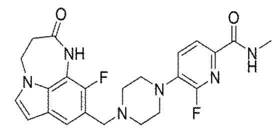
15

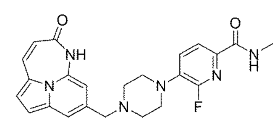
16

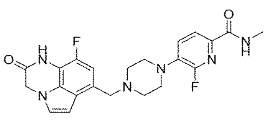
17

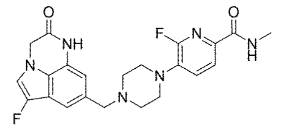
18

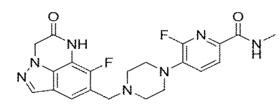
19

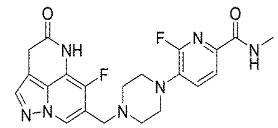
20

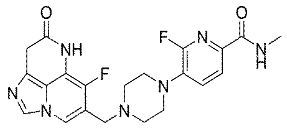
21

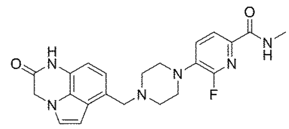
22

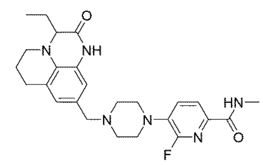
23

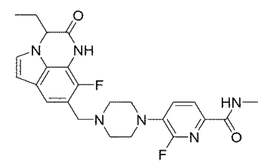
24

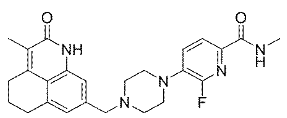
25

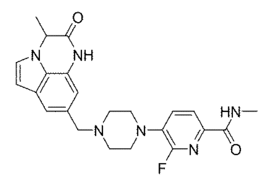
26

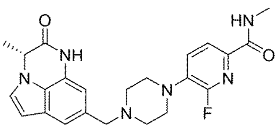
27

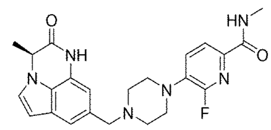
28

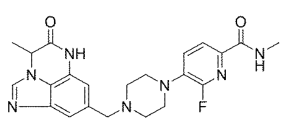
29

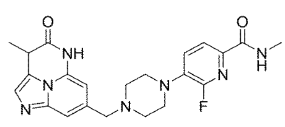
30

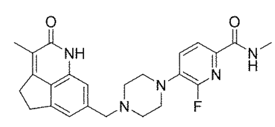
31

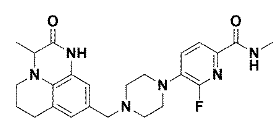
32

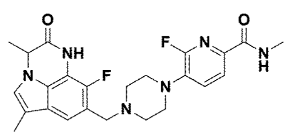
33

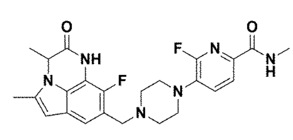
34

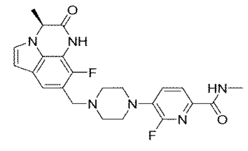
35

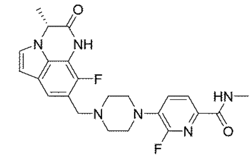
36

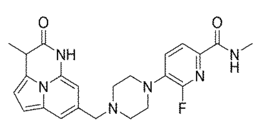
37

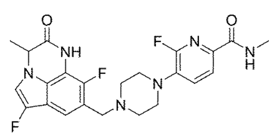
38

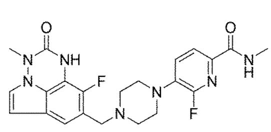
39

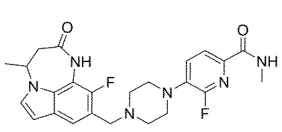
40

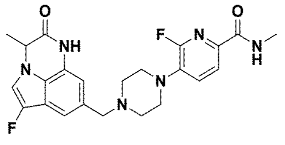
41

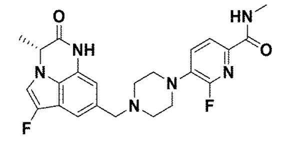
42

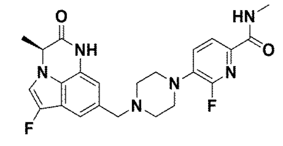
43

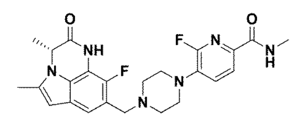
44

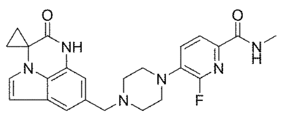
45

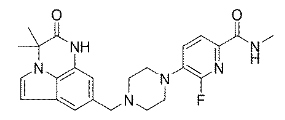
46

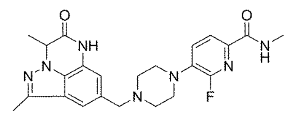
47

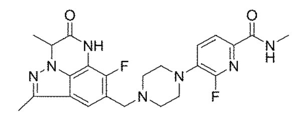
48

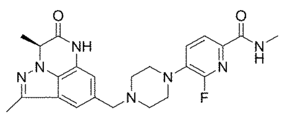
47-1

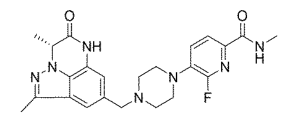
47-2

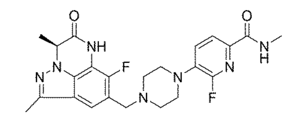
48-1

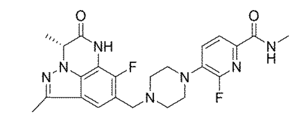
48-2

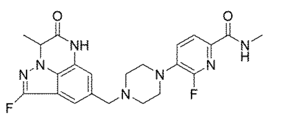
49

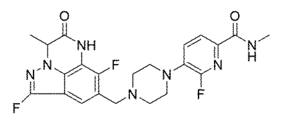
50

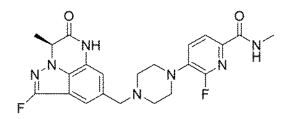
49-1

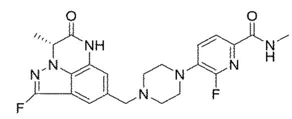
49-2

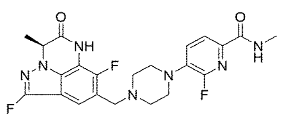
50-1

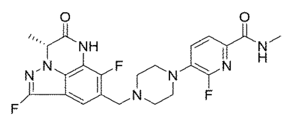
50-2

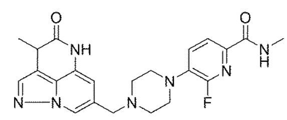
51

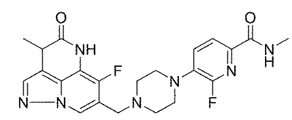
52

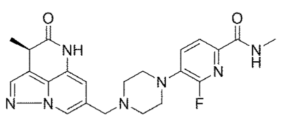
51-1

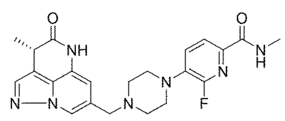
51-2

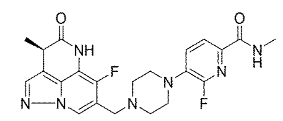
52-1

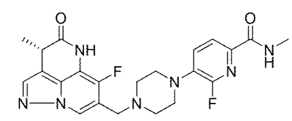
52-2

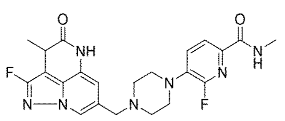
53

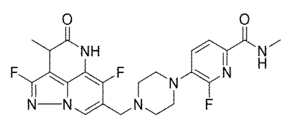
54

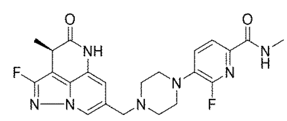
53-1

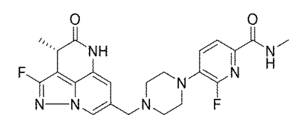
53-2

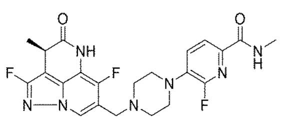
54-1

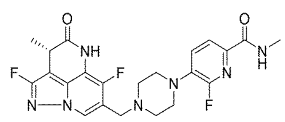
54-2

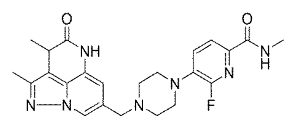
55

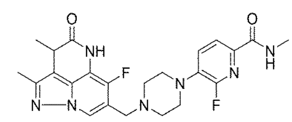
56

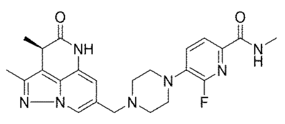
55-1

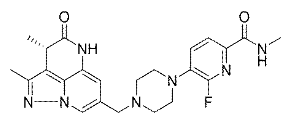
55-2

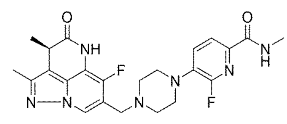
56-1

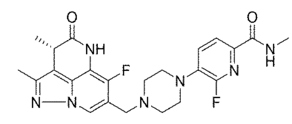
56-2

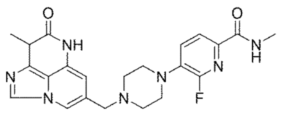
57
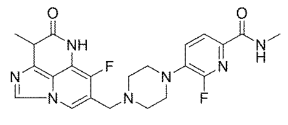
58
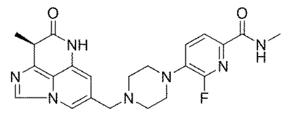
57-1
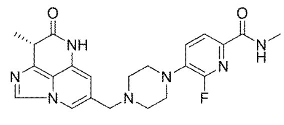
57-2

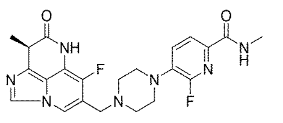
58-1
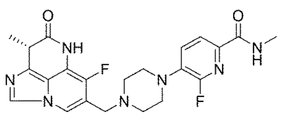
58-2
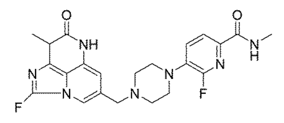
59
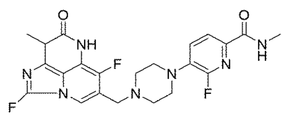
60

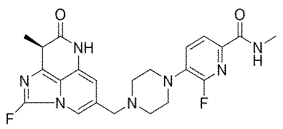
59-1
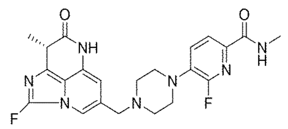
59-2
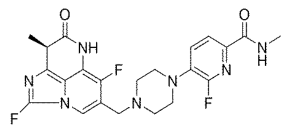
60-1
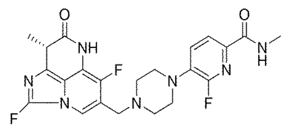
60-2

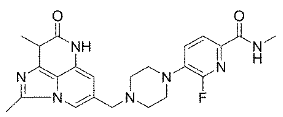
61
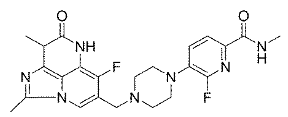
62
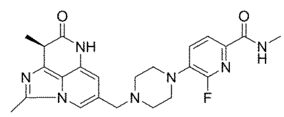
61-1
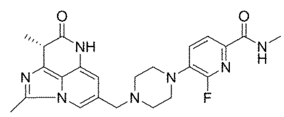
61-2

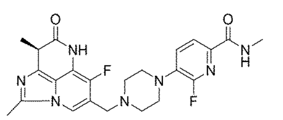
62-1
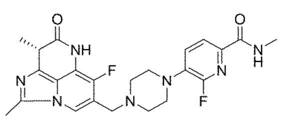
62-2
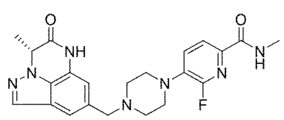
63-1
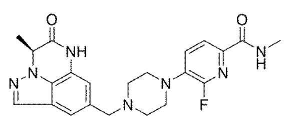
63-2

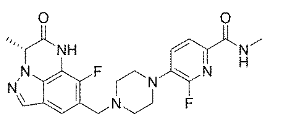
64-1
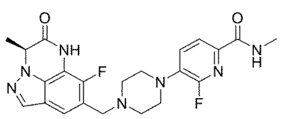
64-2
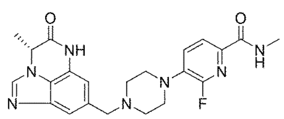
29-1
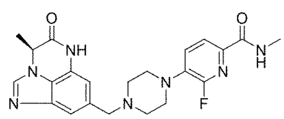
29-2

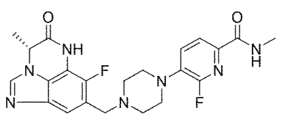
65-1
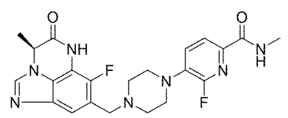
65-2
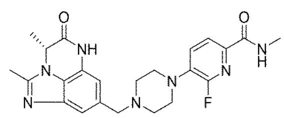
66-1
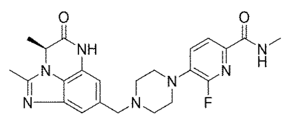
66-2

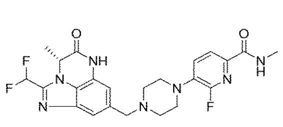
67-1
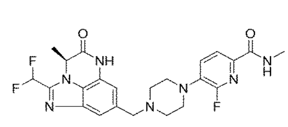
67-2
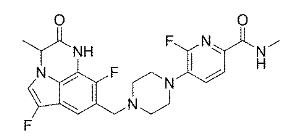
38-1
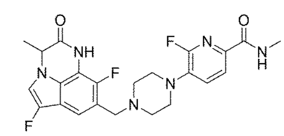
38-2

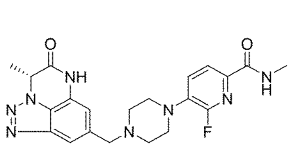
68-1
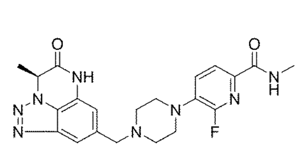
68-2
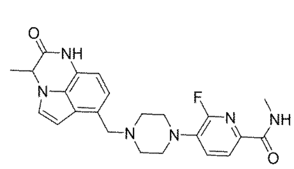
69

**8.** A pharmaceutical composition, wherein the pharmaceutical composition comprises the compound, the pharmaceutically acceptable salt thereof or the stereoisomer thereof according to any one of claims 1 to 7, and one or more pharmaceutically acceptable excipients.

**9.** A use of the compound, the pharmaceutically acceptable salt thereof or the stereoisomer thereof according to any one of claims 1 to 7 and the pharmaceutical composition according to claim 8 in preparing a drug for preventing and/or treating a disease related to PARP overexpression, wherein the disease is selected from neuropathic pain, epilepsy, stroke, Alzheimer’s disease, Parkinson’s disease, amyotrophic lateral sclerosis, Huntington’s disease, schizophrenia, chronic and acute pain, anemia, neuron damage after hypoxia, neurodegenerative diseases, atherosclerosis, hyperlipidemia, heart tissue damage, coronary artery disease, myocardial infarction, cardiogenic shock, diabetic neuropathy, osteoarthritis and osteoporosis.

**10.** A use of the compound, the pharmaceutically acceptable salt thereof or the stereoisomer thereof according to any one of claims 1 to 7 and the pharmaceutical composition according to claim 8 in preparing a drug for preventing and/or treating a cancer related to PARP overexpression,

preferably, the cancer lacks an HR-dependent DNA DSB repair pathway; and
more preferably, the cancer comprises one or more cancer cells, and the cancer cells lack BRCA1 and/or BRCA2, or the cancer cells have a BRCA1 and/or BRCA2 deficient phenotype.

## INTERNATIONAL SEARCH REPORT

International application No. **PCT/CN2023/128071**

**A. CLASSIFICATION OF SUBJECT MATTER**

C07D519/00(2006.01)i; C07D471/04(2006.01)i; C07D401/14(2006.01)i; A61K31/444(2006.01)i; A61K31/4545(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, CNKI, DWPI, WPABS, REGISTRY(STN), CAPLUS(STN): 多环, 聚（ADP 核糖）聚合酶, 抑制剂, 癌, 肿瘤, polycyclic, poly(ADP-ribose) polymerase, PARP, inhibitor, cancer, tumor, tumour

**C. DOCUMENTS CONSIDERED TO BE RELEVANT**

| Category* | Citation of document, with indication, where appropriate, of the relevant passages | Relevant to claim No. |
|---|---|---|
| PX | WO 2023122140 A1 (SYNNOVATION THERAPEUTICS, INC.) 29 June 2023 (2023-06-29) claims 1 and 52-58 | 1-10 |
| A | CN 115232154 A (SHANGHAI HANSOH BIOMEDICAL CO., LTD.) 25 October 2022 (2022-10-25) entire document | 1-10 |
| A | WO 2022222921 A1 (WIGEN BIOMEDICINE TECHNOLOGY (SHANGHAI) CO., LTD.) 27 October 2022 (2022-10-27) entire document | 1-10 |
| A | WO 2011014681 A1 (TAKEDA PHARMACEUTICAL CO., LTD. et al.) 03 February 2011 (2011-02-03) entire document | 1-10 |

☐ Further documents are listed in the continuation of Box C.    ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 January 2024** | **30 January 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/128071**

| Patent document cited in search report | Publication date (day/month/year) | Patent family member(s) | Publication date (day/month/year) |
|---|---|---|---|
| WO 2023122140 A1 | 29 June 2023 | TW 202332438 A | 16 August 2023 |
| CN 115232154 A | 25 October 2022 | TW 202309026 A | 01 March 2023 |
| | | CA 3215823 A1 | 27 October 2022 |
| | | WO 2022223025 A1 | 27 October 2022 |
| | | BR 112023019797 A2 | 07 November 2023 |
| | | AU 2022261029 A1 | 19 October 2023 |
| | | IL 307824 A | 01 December 2023 |
| WO 2022222921 A1 | 27 October 2022 | None | |
| WO 2011014681 A1 | 03 February 2011 | None | |

Form PCT/ISA/210 (patent family annex) (July 2022)